# EUROPÄISCHE PATENTSCHRIFT

(11) **EP 0 582 198 B1**
(45) Veröffentlichungstag und Bekanntmachung des Hinweises auf die Patenterteilung: **08.02.2006**
(21) Anmeldenummer: 93112074.5
(22) Anmeldetag: 28.07.1993
(51) Int. Cl.: C07C 69/712, C07C 69/716, A01N 39/02, C07D 215/26, A01N 43/42, C07C 323/62, C07D 263/58, C07D 239/34, A01N 43/54, C07C 67/31, A01N 25/32

(54) **Substituierte (Hetero-)Arylverbindungen, Verfahren zu deren Herstellung, diese enthaltende Mittel und deren Verwendung als Safener**
Substituted (hetero-)aryle compounds, process for their preparation, those containing compositions and their use as safeners
Composés (hétéro-)aryliques substitués, procédé pour leur préparation, compositions les contenant et leur utilisation comme agents de protection

(30) Priorität: 01.08.1992 DE 4225493
(43) Veröffentlichungstag der Anmeldung: 09.02.1994
(73) Patentinhaber: Bayer CropScience GmbH, 65929 Frankfurt/Main (DE)
(72) Erfinder: Holdgrün, Xenia, Dr., D-65830 Kriftel/Taunus (DE); Willms, Lothar, Dr., D-56204 Hillscheid (DE); Bauer, Klaus, Dr., D-63456 Hanau (DE); Trinks, Klaus, Dr., D-65439 Flörsheim am Main (DE); Bieringer, Hermann, Dr., D-65817 Eppstein/Ts. (DE)

(56) Entgegenhaltungen:
- EP-A- 0 154 153
- FR-A- 1 465 584
- US-A- 3 928 602

## Beschreibung

Die Erfindung betrifft das technische Gebiet der Pflanzenschutzmittel, insbesondere Wirkstoff-Antidot-Kombinationen, die hervorragend für den Einsatz gegen konkurrierende Schadpflanzen in Nutzpflanzenkulturen geeignet sind.

Bei der Anwendung von Pflanzenbehandlungsmitteln, insbesondere bei der Anwendung von Herbiziden, können unerwünschte Schäden an den behandelten Kulturpflanzen auftreten. Viele Herbizide sind nicht voll verträglich (selektiv) mit einigen wichtigen Kulturpflanzen, wie Mais, Reis oder Getreide, so daß ihrem Einsatz enge Grenzen gesetzt sind. Sie können deshalb manchmal überhaupt nicht oder nur in solch geringen Aufwandmengen eingesetzt werden, daß die erwünschte breite herbizide Wirksamkeit gegen die Schadpflanzen nicht gewährleistet ist. So können beispielsweise viele Herbizide der weiter unten genannten Stoffklassen (A) nicht ausreichend selektiv in Mais, Reis oder in Getreide eingesetzt werden. Besonders bei der Nachauflaufapplikation von Herbiziden treten phytotoxische Nebenwirkungen an den Kulturpflanzen auf, und es ist wünschenswert, eine derartige Phytotoxizität zu vermeiden oder zu verringern.

Es ist bereits bekannt, Herbizide in Kombination mit Verbindungen einzusetzen, welche die Phytotoxizität der Herbizide bei Kulturpflanzen reduzieren, ohne die herbizide Wirkung gegen die Schadpflanzen entsprechend zu reduzieren. Solche Kombinationspartner werden "Safener" oder "Antidots" genannt.

Aus EP-A-31 938 ist die Verwendung von Aryloxycarbonsäurenitrilen und Aryloxycarbonsäureamidoximen als Safener für Herbizide aus der Reihe der Phenoxyphenoxycarbonsäureester, Chloracetanilide und Dimedon-derivate bekannt, In EP-A-170 906 werden unter anderem Phenoxycarbonsäureoximester und in EP-A-154 153 Aryloxy-Verbindungen als Safener für Phenoxyphenoxy- sowie Heteroaryloxyphenoxy-herbizide beschrieben.
In EP-A-112 799 werden 4-Chlorphenoxy- sowie 4-Chlor-2-methyl-phenoxyessigsäure als Safener für 2-[4-(3,5-Dichlorpyridyl-2-oxy)- phenoxy]-propionsäure-propargylester genannt.
EP-A-293 062 beschreibt die Verwendung von Aryloxy-Verbindungen als Safener für Cyclohexandion-herbizide und EP-A-88 066 die Verwendung von 3,5-Bis-(trifluormethyl)-phenoxycarbonsäurederivaten als Safener, insbesondere für Acetamide, speziell für Triallate.
In EP-A-86750 werden Chinolin-8-oxy-alkancarbonsäurenitrile und -amidoxime als Safener für Phenoxyphenoxyalkancarbonsäureester und Sulfonylharnstoffe beschrieben. Aus EP-A 94349 ist die Verwendung entsprechender Carbonsäureester als Safener für Herbizide aus verschiedenen Strukturklassen bekannt.
Aus DE-2637886 ist bereits die Verwendung von 3-Pyridyloxy-alkanamiden als Safener für Herbizide aus der Triazin-, Carbamat- und Haloacetanilid-Reihe bekannt.

Es wurde nun gefunden, daß sich überraschenderweise eine Gruppe von Aryl- und Heteroarylderivaten der nachstehenden Formel I hervorragend dazu eignet, Kulturpflanzen gegen schädigende Wirkungen von aggressiven Agrarchemikalien, insbesondere Herbiziden, zu schützen.

Aryl- und Heteroarylderivate, welche zum Schützen von Kulturpflanzen gegen schädigende Wirkungen von aggressiven Agrarchemikalien geeignet sind, entsprechen der Formel I, 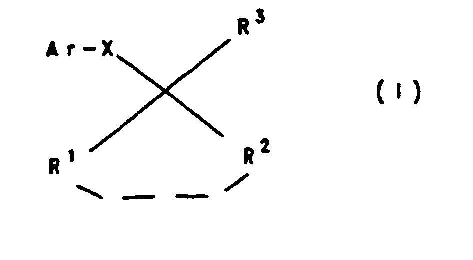 worin
- R¹ und R²: unabhängig voneinander Reste der Formel

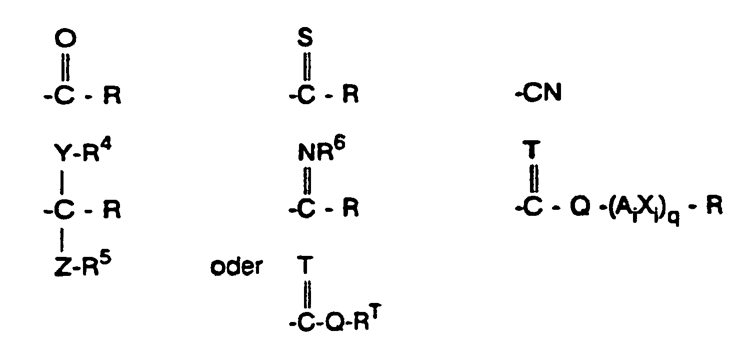

worin R, R^{T}, R⁴, R⁵, R⁶, Y, T, Z, Q, Aᵢ, Xᵢ, q wie weiter unten definiert sind, oder
- R¹ und R²: miteinander verbunden sind und gemeinsam eine Gruppe der Formel

-CO-Q¹-D-Q²-CO-

worin
- Q¹, Q²: unabhängig voneinander wie Q definiert sind und
- D: eine divalente Gruppe der Formel CR'R" oder C=O, wobei R' und R" unabhängig voneinander Wasserstoff oder C₁-C₄-Alkyl bedeuten,
- R³: Wasserstoff, Halogen, C₁-C₁₈-Alkyl, C₂-C₈-Alkenyl, C₂-C₈-Alkinyl, C₁-C₁₈-Alkoxy, C₂-C₈-Alkenyloxy, C₂-C₈-Alkinyloxy, C₁-C₁₈-Alkylthio, C₂-C₈-Alkenylthio, C₂-C₈-Alkinylthio, wobei jeder der letztgenannten 9 Reste jeweils unsubstituiert oder durch einen oder mehrere Reste aus der Gruppe Halogen, Nitro und Cyano substituiert ist, oder C₃-C₁₂-Cycloalkyl, das unsubstituiert oder durch einen oder mehrere Reste aus der Gruppe C₁-C₄-Alkyl, Halogen, Nitro und Cyano substituiert ist, oder SiR^{a}R^{b}R^{c}, worin R^{a}, R^{b} und R^{c} unabhängig voneinander C₁-C₄-Alkyl, C₂-C₄-Alkenyl, C₂-C₄-Alkinyl oder unsubstituiertes oder substituiertes Phenyl bedeuten, oder einen Rest der Formel Ar'X'-, worin Ar' und X' analog Ar bzw. X definiert sind,
- X: O, S, NH-NH oder NR^{d}, wobei R^{d} analog R⁴ definiert ist, oder -CH₂O-, -CH₂S-, -CH(Ar)O- oder -CH(Ar)S-
- Ar: einen aromatischen Rest,
z. B. einen unsubstituierten oder substituierten Phenyl-, Naphthyl- oder Heteroarylrest,
vorzugsweise einen carbocyclischen oder carbobicyclischen Rest der Formel 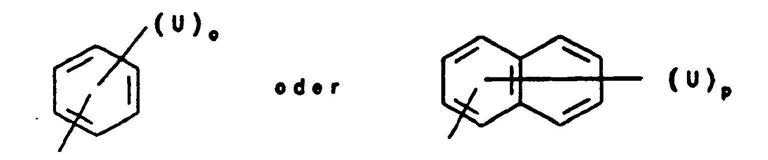 worin
- (U): für gleiche oder verschiedene Reste stehen, welche unabhängig voneinander Wasserstoff, Halogen, Cyano, Nitro, Amino oder C₁-C₈-Haloalkyl, C₁-C₈-Haloalkoxy, C₁-C₈-Alkyl, C₁-C₈-Alkoxy, Mono-(C₁-C₄-alkyl)-amino, Di-(C₁-C₄-alkyl)-amino, C₁-C₈-Alkylthio oder C₁-C₈-Alkylsulfonyl, wobei jeder der letztgenannten 8 Reste unsubstituiert oder durch einen oder mehrere, vorzugsweise bis zu drei gleiche oder verschiedene Substituenten aus der Gruppe enthaltend Halogen, C₁-C₈-Haloalkoxy, Nitro, Cyano, Hydroxy, C₁-C₈-Alkoxy, worin eine oder mehrere, vorzugsweise bis zu drei CH₂-Gruppen durch Sauerstoff ersetzt sein können, C₁-C₈-Alkylthio, C₁-C₆-Alkylsulfinyl, C₁-C₆-Alkylsulfonyl, C₂-C₈-Alkenylthio, C₂-C₆-Alkinylthio, C₂-C₈-Alkenyloxy, C₂-C₈-Alkinyloxy, C₃-C₇-Cycloalkyl. C₃-C₇-Cycloalkoxy, Mono- und Di-(C₁-C₄-alkyl)-amino und C₁-C₈-Alkoxycarbonyl substituiert ist, und vorzugsweise Wasserstoff, Halogen, C₁-C₆-Haloalkyl, wie Trifluormethyl, C₁-C₆-Haloalkoxy, wie Difluormethoxy, C₁-C₆-Alkyl, C₁-C₆-Alkoxy, C₁-C₆-Alkylthio, C₁-C₆-Alkylsulfonyl, Nitro, Amino, (C₁-C₂-Alkyl)-amino, Di-(C₁-C₂-alkyl)-amino oder Cyano bedeuten, und
- o: eine ganze Zahl von 1 bis 5, vorzugsweise 1 bis 3, ist und
- p: eine ganze Zahl von 1 bis 7, vorzugsweise 1 bis 3, ist,

oder Ar einen monocyclischen oder bicyclischen Heteroarylrest aus der Gruppe Furyl, Thienyl, Pyrrolyl, Pyrazolyl, Thiazolyl, Oxazolyl, Pyridinyl, Pyrimidinyl, Pyrazinyl, Pyridazinyl und Chinolinyl, der jeweils unsubstituiert oder durch einen oder mehrere, vorzugsweise ein bis drei der genannten Reste U substituiert ist,
- R: Wasserstoff oder einen aliphatischen, aromatischen, heteroaromatischen, araliphatischen oder heteroaraliphatischen Rest mit 1 bis 30 C-Atomen und mit gegebenenfalls einer oder mehreren funktionellen Gruppen, beispielsweise R einen Rest
Wasserstoff, C₁-C₁₈-Alkyl, C₃-C₁₂-Cycloalkyl, C₂-C₆-Alkenyl oder C₂-C₈-Alkinyl, Heterocyclyl, Phenyl oder Heteroaryl,
wobei jeder der vorstehenden C-haltigen Reste unabhängig voneinander unsubstituiert oder durch einen oder mehrere Reste aus der Gruppe enthaltend Halogen, Cyano, Thio, Nitro, Hydroxy, C₁-C₈-Alkyl, letzteres nur für den Fall cyclischer Reste, C₁-C₈-Haloalkyl, C₁-C₈-Alkoxy, C₂-C₈-Alkenyloxy, C₂-C₈-Alkinyloxy, C₁-C₈-Haloalkoxy, C₁-C₈-Alkylthio, C₂-C₈-Alkenylthio, C₂-C₈-Alkinylthio, C₃-C₇-Cycloalkyl, C₃-C₇-Cycloalkoxy, Reste der Formeln -NR*R** und -CO-NR*R** und -O-CO-NR*R**, wobei R* und R** in den letztgenannten 3 Resten unabhängig voneinander Wasserstoff, C₁-C₈-Alkyl, C₂-C₈-Alkenyl, C₂-C₈-Alkinyl, Benzyl, Phenyl oder substituiertes Phenyl sind oder gemeinsam mit dem N-Atom einen 3- bis 8-gliedrigen Heterocyclus, der noch bis zu 2 weitere Heteroatome aus der Gruppe N, O und S enthalten und durch C₁-C₄-Alkyl substituiert sein kann, bedeuten, sowie (C₁-C₈-Alkoxy)-carbonyl, (C₁-C₈-Alkoxy)-thiocarbonyl, (C₂-C₈-Alkenyloxy)-carbonyl, (C₁-C₈-Alkylthio)-embonyl, (C₂-C₈-Alkenylthio)-carbonyl, (C₂-C₈-Alkinylthio)-carbonyl, (C₂-C₈-Alkinyloxy)-carbonyl, Formyl, (C₁-C₈-Alkyl)-carbonyl, (C₂-C₈-Alkenyl)-carbonyl, (C₂-C₈-Alkinyl)-carbonyl, C₁-C₄-Alkylimino, C₁-C₄-Alkoxyimino, (C₁-C₈-Alkyl)-carbonylamino, (C₂-C₈-Alkenyl)-carbonylamino, (C₂-C₈-Alkinyl)-carbonylamino, (C₁-C₈-Alkoxy)-carbonylamino, (C₂-C₈-Alkenyloxy)-carbonylamino, (C₂-C₈-Alkinyloxy)-carbonylamino, (C₁-C₈-Alkyl)-amino-carbonylamino, (C₁-C₆-Alkyl)-carbonyloxy, das unsubstituiert
oder durch Halogen, NO₂, C₁-C₄-Alkoxy oder gegebenenfalls substituiertes Phenyl substituiert ist, (C₂-C₆-Alkenyl)-carbonyloxy, (C₂-C₆-Alkinyl)-carbonyloxy, (C₁-C₈-Alkoxy)-carbonyloxy, (C₂-C₈-Alkenyloxy)-carbonyloxy, (C₂-C₈-Alkinyloxy)-carbonyloxy, C₁-C₈-Alkylsulfonyl, Phenyl, Phenyl-C₁-C₆-alkoxy, Phenyl-(C₁-C₆-alkoxy)-carbonyl, Phenoxy, Phenoxy-C₁-C₆-alkoxy, Phenoxy-(C₁-C₆-alkoxy)-carbonyl, Phenoxycarbonyl, Phenylcarbonyloxy, Phenylcarbonylamino, Phenyl-(C₁-C₆-alkyl)-carbonylamino und Phenyl-(C₁-C₆-alkyl)-carbonyloxy, wobei die letztgenannten 11 Reste im Phenylring unsubstituiert oder durch einen oder mehrere Reste aus der Gruppe Halogen, C₁-C₄-Alkyl, C₁-C₄-Alkoxy, C₁-C₄-Haloalkyl, C₁-C₄₋Haloalkoxy und Nitro substituiert sind, und Reste der Formeln -SiR'_{3'-}O-SiR'_{3'} (R')₃Si-C₁-C₆-alkoxy, -CO-O-NR'₂, -O-N=CR'₂, -N=CR'_{2'} -O-NR'_{2'} -CH(OR')₂ und -O-(CH₂)ₘ-CH(OR')₂, worin die R' in den genannten Formeln unabhängig voneinander Wasserstoff, C₁-C₄-Alkyl oder Phenyl, das unsubstituiert oder ein- oder mehrfach durch Reste aus der Gruppe Halogen, C₁-C₄-Alkyl, C₁-C₄-Alkoxy, C₁-C₄-Haloalkyl, C₁-C₄-Haloalkoxy und Nitro substituiert ist, oder paarweise eine C₂₋C₆-Alkylenkette und m = 0 bis 6 bedeuten, und einen substituierten Alkoxyrest der Formel R"O-CHR'''CH(OR")-C₁-C₆-alkoxy, worin die R" unabhängig voneinander C₁-C₄-Alkyl oder zusammen eine C₁-C₆₋Alkylengruppe und R"' Wasserstoff oder C₁-C₄-Alkyl bedeuten, substituiert ist,
- R^{T}: einen Rest der Formel -CO-R, -CS-R, -NR^{f}R^{g}, -N=CR^{h}Rⁱ oder SiR^{a}R^{b}R^{c}, wobei R die genannte Bedeutung hat und R^{f}, R^{g}, R^{h} und Rⁱ unabhängig voneinander Wasserstoff, C₁-C₄-Alkyl, C₂-C₄-Alkenyl, C₂-C₄-Alkinyl, Benzyl, Phenyl oder substituiertes Phenyl sind oder R^{f} und R^{g} gemeinsam mit dem N-Atom einen 5- oder 6-gliedrigen Heterocyclus, der noch bis zu 2 weitere Heteroatome aus der Gruppe N, O und S enthalten und durch C₁-C₄-Alkyl substituiert sein kann, bedeuten und
R^{a}, R^{b} und R^{c} unabhängig voneinander C₁-C₄-Alkyl, C₂-C₄-Alkenyl, C₂-C₄-Alkinyl, Phenyl oder substituiertes Phenyl sind,
- Y und Z: unabhängig voneinander Sauerstoff, Schwefel in seinen verschiedenen Oxidationstufen, vorzugsweise S, SO oder SO₂, oder -NR^{e}, wobei R^{e} analog R⁴ definiert ist,
- R⁴ und R⁵: gleich oder verschieden sind und unabhängig voneinander Wasserstoff, C₁-C₆-Alkyl, C₂-C₆-Alkenyl, C₂-C₆-Alkinyl, (C₁-C₄-Alkyl)-carbonyl, wobei jeder der 4 letztgenannten Reste unsubstituiert oder durch einen oder mehrere Substituenten aus der Gruppe enthaltend Halogen, C₁-C₈-Haloalkoxy, Nitro, Cyano, Hydroxy, C₁-C₈-Alkoxy und C₁-C₈-Alkoxy, worin eine oder mehrere, vorzugsweise bis zu drei nicht direkt aneinander gebundene CH₂-Gruppen durch Sauerstoff ersetzt sind, und C₁-C₈-Alkylthio, C₁-C₆-Alkylsulfonyl, C₂-C₈-Alkenylthio, C₂-C₈-Alkinylthio, C₂-C₈-Alkenyloxy, C₂-C₈-Alkinyloxy, C₃-C₇-Cycloalkyl, C₃-C₇-Cycloalkoxy sowie Amino, Mono- und Di-(C₁-C₄-alkyl)-amino substituiert ist, oder Formyl, SiR^{a}R^{b}R^{c}, worin R^{a}, R^{b} und R^{c} unabhängig voneinander C₁-C₄-Alkyl, C₂-C₄-Alkenyl, C₂-C₄-Alkinyl oder unsubstituiertes oder substituiertes Phenyl bedeuten, oder C₃-C₈-Cycloalkyl, C₃-C₈-Cycloalkenyl, Heterocyclyl mit 3 bis 7 Ringatomen, Aryl, Heteroaryl oder Arylcarbonyl, wobei jeder der letztgenannten 6 Reste unsubstituiert oder durch einen oder mehrere Reste aus der Gruppe C₁-C₈-Alkyl, Halogen, C₁-C₈-Haloalkoxy, Nitro, Cyano, Hydroxy, C₁-C₈-Alkoxy und C₁-C₈-Alkoxy, worin eine oder mehrere, vorzugsweise bis zu drei nicht direkt aneinander gebundene CH₂-Gruppen durch Sauerstoff ersetzt sind, und C₁-C₈-Alkylthio, C₁-C₆-Alkylsulfonyl, C₂-C₈-Alkenylthio, C₂-C₈-Alkinylthio, C₂-C₈-Alkenyloxy, C₂-C₈-Alkinyloxy, C₃-C₇-Cycloalkyl, C₃-C₇-Cycloalkoxy sowie Amino, Mono- und Di-(C₁-C₄-alkyl)-amino substituiert ist, oder
- R⁴ und R⁵: gemeinsam eine C₂-C₄-Alkylen-kette oder C₂-C₄-Alkenylen-kette, welche unsubstituiert oder durch 1 oder 2 Reste aus der Gruppe Methyl, Ethyl, Methoxy, Ethoxy und Halogen substituiert ist,
- R⁶: Wasserstoff, C₁-C₄-Alkyl, C₂-C₄-Alkenyl, C₂-C₄-Alkinyl, C₆-C₁₂-Aryl, Heteroaryl, Benzyl, C₁-C₄-Alkoxy, Acyloxy, wie (C₁-C₄-Alkyl)-carbonyloxy oder unsubstituiertes und substituiertes Phenylcarbonyloxy, oder Hydroxy, -NH-CO-NH₂, -NH-CS-NH₂, Mono- und Di-(C₁-C₄-alkyl)-amino, -NH-Acyl, -NHSO₂-(C₁-C₄-alkyl), C₆-C₁₂-Aryloxy, Heteroaryloxy, NH-SO₂-Aryl oder NH-Aryl, worin Aryl bzw. Heteroaryl in den letztgenannten 4 Resten unsubstituiert oder durch einen oder mehrere Reste aus der Gruppe Halogen, Nitro, (C₁-C₄)-Alkyl, (C₁-C₄)-Alkoxy, (C₁-C₄)-Haloalkyl und (C₁-C₄)-Haloalkoxy substituiert ist,
- T: O, S, NR⁷, NOR⁷ oder NO-Acyl,
- Q: O oder S,
- q: eine ganze Zahl von 0 bis 4,
- i: eine Laufiziffer, welche bei q ungleich 0 alle ganzen Zahlen von 1 bis q annimmt, wobei q die oben angegebene Bedeutung hat,
- Xᵢ: unabhängig voneinander O, S, NR⁷, N-(Aᵢ-Xᵢ-)_{q}-R
- Aᵢ: unabhängig voneinander unsubstituiertes oder substituiertes C₁-C₆-Alkylen, C₂-C₆-Alkenylen, C₂-C₆-Alkinylen, C₃-C₆-Cycloalkylen, C₃-C₆-Cycloalkenylen, Heterocyclylen, Arylen oder Heteroarylen und
- R⁷: unabhängig voneinander H, C₁-C₄-Alkyl, C₂-C₄-Alkenyl, C₂-C₄-Alkinyl, C₃-C₆-Cycloalkyl, C₃-C₆-Cycloalkenyl, Heterocyclyl, Aryl oder Heteroaryl

bedeuten.

In der Formel (I) und im folgenden können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die Kohlenstoffgerüste mit 1 bis 4 C-Atomen, bzw. bei ungesättigten Gruppen mit 2 bis 4 C-Atomen bevorzugt. Alkylreste, auch in den zusammengesetzten Bedeutungen wie Alkoxy, Haloalkyl usw., bedeuten z. B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, Butt-3-in-1-yl, 1-Methyl-but-3-in-1-yl.
Halogen bedeutet Fluor, Chlor, Brom oder lod, vorzugsweise Fluor, Chlor oder Brom, insbesondere Fluor oder Chlor. Haloalkyl, -alkenyl und -alkinyl bedeuten durch Halogen teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃. Entsprechendes gilt für Haloalkenyl und andere durch Halogen substituierte Reste.

Aryl bedeutet beispielsweise Phenyl, Naphthyl, Tetrahydronaphthyl, Indenyl, Indanyl, Pentalenyl, Fluorenyl und ähnliches, vorzugsweise Phenyl; Aryloxy bedeutet vorzugsweise die den genannten Arylresten entsprechenden Oxy-Reste, insbesondere Phenoxy.

Heteroaryl bzw. Heteroaryl in Heteroaryloxy bedeutet beispielsweise Pyridyl, Pyrimidinyl, Pyridazinyl, Pyrazinyl, Thienyl, Thiazolyl, Oxazolyl, Furyl, Pyrrolyl, Pyrazolyl und imidazolyl, aber auch bicyclische oder polycyclische aromatische oder araliphatische Verbindungen, z. B. Chinotinyl, Benzoxazolyl etc.

Substituiertes Aryl bzw. Aryloxy, Heteroaryl, Heteroaryloxy, Phenyl, Phenoxy, Benzyl, Benzyloxy bzw. substituierte bicyclische Reste mit aromatischen Anteilen bedeuten beispielsweise einen vom unsubstituierten Grundkörper abgeleiteten substituierten Rest, wobei die Substituenten beispielsweise ein oder mehrere, vorzugsweise 1, 2 oder 3 Reste aus der Gruppe Halogen, Alkyl, Haloalkyl, Alkoxy, Haloalkoxy, Hydroxy, Amino, Nitro, Cyano, Alkoxycarbonyl, Alkylcarbonyl, Formyl, Carbamoyl, Mono- und Dialkylaminocarbonyl, Mono- und Dialkylamino, Alkylsutfinyl und Alkylsulfonyl bedeuten und bei Resten mit C-Atomen solche mit 1 bis 4 C-Atomen, insbesondere 1 oder 2, bevorzugt sind. Bevorzugt sind dabei in der Regel Substituenten aus der Gruppe Halogen, z. B. Fluor und Chlor, C₁-C₄-Alkyl, vorzugsweise Methyl oder Ethyl, C₁-C₄-Haloalkyl, vorzugsweise Trifluormethyl, C₁₋C₄-Alkoxy, vorzugsweise Methoxy oder Ethoxy, C₁-C₄-Haloalkoxy, Nitro und Cyano. Besonders bevorzugt sind dabei die Substituenten Methyl, Methoxy und Chlor.

Gegebenenfalls substituiertes Phenyl ist z.B. Phenyl, das unsubstituiert oder ein- oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)-Halogenalkyl, (C₁-C₄)-Halogenalkoxy und Nitro substituiert ist, z.B. o-, m- und p-Tolyl, Dimethylphenyle, 2-, 3- und 4-Chlorphenyl, 2-, 3- und 4-Trifluor- und - Trichlorphenyl, 2,4-, 3,5-, 2,5- und 2,3-Dichlorphenyl, o-, m- und p-Methoxyphenyl.

Ein drei- bis siebengliedriger wie oben beschriebener heterocyclischer Rest ist vorzugsweise von Benzol abgeleitet, wovon mindestens ein CH durch N und/oder mindestens zwei benachbarte CH-Paare durch NH, S und/oder O ersetzt sind. Der Rest kann benzokondensiert sein. Er ist gegebenenfalls teilweise oder vollständig hydriert und wird dann auch als Heterocyclyl bezeichnet. Es kommen insbesondere Reste wie Oxiranyl, Pyrrolidyl, Piperidyl, Dioxolanyl, Pyrazolyl, Morpholyl, Furyl, Tetrahydrofuryl, Indolyl, Chinolinyl, Pyrimidyl, Azepinyl, Triazolyl, Thienyl und Oxazolyl in Frage.

Acyl bedeutet beispielsweise Formyl, Alkylcarbonyl wie (C₁-C₄-Alkyl)-carbonyl, Phenylcarbonyl, wobei der Phenylring substituiert sein kann, z. B. wie oben für Phenyl gezeigt, oder Alkyloxycarbonyl, Phenyloxycarbonyl, Benzyloxycarbonyl, Alkylsulfonyl und andere Reste von organischen Säuren.

Manche Verbindungen der Formel I enthalten ein oder mehrere asymmetrische C-Atome oder Doppelbindungen, die in der allgemeinen Formel I nicht gesondert angegeben sind. Die durch ihre spezifische Raumform definierten möglichen Stereoisomeren, wie Enantiomere, Diastereomere, E- und Z-Isomere sowie deren Gemische sind jedoch alle von der Formel I umfaßt.

Die Verbindungen der Formel I, welche von Carbonsäuren abgeleitet sind, können Salze bilden, bei denen der Rest R durch ein Äquivalent eines für die Landwirtschaft geeigneten Kations ersetzt wird. Diese Salze sind beispielsweise Metall-, insbesondere Alkali- oder Erdalkalisalze, aber auch Ammoniumsalze oder Salze mit organischen Aminen sowie Salze, die als Kationen Sulfonium- oder Phosphoniumionen enthalten.

Als Salzbildner eignen sich besonders Metalle und organische Stickstoffbasen, vor allem quartäre Ammoniumbasen. Hierbei kommen als zur Salzbildung geeignete Metalle Erdalkalimetalle, wie Magnesium oder Calcium, vor allem aber Alkalimetalle in Betracht, wie Lithium und insbesondere Kalium und Natrium.

Beispiele für zur Salzbildung geeignete Stickstoffbasen sind primäre, sekundäre oder tertiäre, aliphatische und aromatische, gegebenenfalls am Kohlenwasserstoffrest hydroxylierte Amine, wie Methylamin, Ethylamin, Propylamin, Isopropylamin, die vier isomeren Butylamine, Dimethylamin, Diethylamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin, Isochinolin sowie Methanolamin, Ethanolamin, Propanolamin, Dimethanolamin, Diethanolamin oder Triethanolamin.

Beispiele für quartäre Ammoniumbasen sind Tetraalkylammoniumkationen, in denen die Alkylreste unabhängig voneinander geradkettige oder verzweigte C₁-C₆₋Alkylgruppen sind, wie das Tetramethylammoniumkation, das Tetraethylammoniumkation oder das Trimethylethylammoniumkation, sowie weiterhin das Trimethylbenzylammoniumkation, das Triethylbenzylammoniumkation und das Trimethyl-2-hydroxyethylammoniumkation.

Besonders bevorzugt als Salzbildner sind das Ammoniumkation und Di- sowie Trialkylammoniumkationen, in denen die Alkylreste unabhängig voneinander geradkettige oder verzweigte, gegebenenfalls durch eine Hydroxylgruppe substituierte (C₁-C₆)-Alkylgruppen darstellen, wie beispielsweise das Dimethylammoniumkation, das Trimethylammoniumkation, das Triethylammoniumkation, das Di-(2-hydroxyethyl)-ammoniumkation und das Tri-(2-hydroxyethyl)-ammoniumkation.

Von besonderem Interesse sind Verbindungen der Formel (I) oder deren Salze, worin
- R³: Wasserstoff, C₁-C₄-Alkyl, C₁-C₄-Alkoxy, C₅-C₆-Cycloalkyl, Trimethylsilyl, Triethylsilyl oder ein Rest der Formel Ar'X'-, worin Ar' und X' analog Ar bzw. X definiert sind,
- X: O, S, NH, NCH₃ oder NC₂H₅,
- Ar: einen Rest der Formel 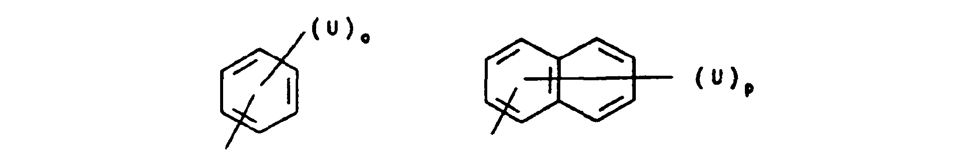 worin
- (U): ür gleiche oder verschiedene Reste stehen, welche unabhängig voneinander Wasserstoff, Halogen, wie Fluor, Chlor, Brom und Iod, Cyano, Nitro, Amino oder C₁-C₄-Haloalkyl, C₁-C₄-Haloalkoxy, C₁-C₄-Alkyl, C₁-C₄-Alkoxy, Mono-(C₁-C₄-alkyl)-amino, Di-(C₁-C₄-alkyl)-amino, C₁-C₄-Alkylthio oder C₁-C₄-Alkylsulfonyl, und
- o: eine ganze Zahl von 1 bis 3 ist und
- p: eine ganze Zahl von 1 bis 3 ist, oder
- Ar: einen monocyclischen oder bicyclischen Heteroarylrest aus der Gruppe Furyl, Thienyl, Pyrrolyl, Pyrazolyl, Thiazolyl, Oxazolyl, Pyridinyl, Pyrimidinyl, Pyrazinyl, Pyridazinyl und Chinolinyl, der unsubstituiert oder durch ein bis drei der vorstehend genannten Reste U substituiert ist, bedeuten.

Von besonderem Interesse sind auch Verbindungen der genannten Formel (I) und deren Salze, worin
- R: Wasserstoff, C₁-C₈-Alkyl, C₄-C₇-Cycloalkyl, C₂-C₈-Alkenyl oder C₂-C₈-Alkinyl, Heterocyclyl, Phenyl oder Heteroaryl ist,
wobei jeder der letztgenannten 7 Reste unabhängig voneinander unsubstituiert oder durch einen oder mehrere Reste aus der Gruppe enthaltend Halogen, Cyano, Thio, Nitro, Hydroxy, C₁-C₄-Alkyl, letzteres nur für den Fall cyclischer Reste, C₁-C₄-Haloalkyl, C₁-C₄-Alkoxy, C₂-C₄-
Alkenyloxy, C₂-C₄-Alkinyloxy, C₁-C₄-Haloalkoxy, C₁-C₄-Alkylthio, C₂-C₄₋Alkenylthio, C₂-C₄-Alkinylthio, C₅-C₆-Cycloalkyl, C₅-C₆-Cycloalkoxy, Amino, Mono- und Di-(C₁-C₄-alkyl)-amino, (C₁-C₆-Alkoxy)-carbonyl, Reste der Formeln -SiR'_{3'} -O-NR'₂, -O-N=CR'₂, -N=CR'_{2'} worin die R' in den genannten Formeln unabhängig voneinander Wasserstoff, C₁₋C₂-Alkyl oder Phenyl oder paarweise eine C₂-C₅-Alkylenkette bedeuten, substituiert ist, bedeutet oder
Verbindungen, worin
- R^{T}: einen Rest der Formel -CO-R, -NR^{f}R^{g} oder -N=CR^{h}Rⁱ, wobei R, R^{f}, R^{g}, R^{h} und Rⁱ die genannten Bedeutungen haben, bedeutet.

Vorzugsweise bedeutet R Wasserstoff, C₁-C₈-Alkyl, C₅-C₆-Cycloalkyl, C₂-C₈₋Alkenyl oder C₂-C₈-Alkinyl, wobei jeder der letztgenannten 4 Reste unabhängig voneinander unsubstituiert oder durch einen oder mehrere Reste aus der Gruppe enthaltend Halogen, Cyano, Nitro, C₁-C₄-Alkoxy, C₂-C₄-Alkenyloxy, C₂-C₄₋Alkinyloxy, C₅-C₆-Cycloalkyl, C₅ C₆-Cyctoalkoxy. Mono- und Di-(C₁-C₄-alkyl)-amino, Reste der Formeln -SiR'₃, -O-N=CR'₂, -N=CR'₂, worin die R' in den genannten Formeln unabhängig voneinander Wasserstoff, C₁-C₂-Alkyl oder Phenyl oder paarweise eine C₂-C₅-Alkylenkette bedeuten, substituiert ist.
- R^{T}: bedeutet vorzugsweise -CO-R, wobei R die genannte Bedeutung hat, oder -NR^{f}R^{g} oder -N=CR^{h}Rⁱ, worin
- R^{f}, R^{g}: unabhängig voneinander H, C₁-C₂-Alkyl, Benzyl oder Phenyl oder gemeinsam mit dem N-Atom Pyrrolidin-1-yl, Piperidin-1-yl, Morpholin-4-yl, Piperazin-1-yl oder Imidazol-1-yl, bzw.
- R^{h}, Rⁱ: unabhängig voneinander H, C₁-C₂-Alkyl, Benzyl oder Phenyl bedeuten.

Von besonderem Interesse sind auch Verbindungen der genannten Formel (I) und deren Salze, worin
- R⁴ und R⁵: gleich oder verschieden sind und unabhängig voneinander Wasserstoff, C₁-C₄-Alkyl, C₂-C₄-Alkenyl, C₂-C₄-Alkinyl, C₅-C₆-Cycloalkyl oder C₅-C₆-Cycloalkenyl bedeuten,

sowie solche Verbindungen, worin
- R⁶: Wasserstoff, C₁-C₄-Alkyl, Phenyl, Benzyl, Hydroxy, NH-CO-NH₂, -NH-Aryl oder C₁-C₄-Alkoxy bedeutet.

Von besonderem Interesse sind auch Verbindungen der genannten Formel (I) und deren Salze, worin
- T: O, S oder NR⁷, vorzugsweise O oder NR⁷,
- Q: O oder S, vorzugsweise O,
- q: eine ganze Zahl von 0 bis 4,
- i: eine Laufziffer, welche bei q ungleich 0 alle ganzen Zahlen von 1 bis q annimmt, wobei q die oben angegebene Bedeutung hat,
- Xᵢ: unabhängig voneinander O, S, NR⁷, N-(Aᵢ-Xᵢ-)_{q}-R
- Aᵢ: unabhängig voneinander unsubstituiertes oder substituiertes C₁-C₄-Alkylen, C₂-C₄-Alkenylen, C₅-C₆-Cycloalkylen, vorzugsweise C₁-C₄-Alkylen,
- R⁷: unabhängig voneinander H, C₁-C₄-Alkyl, C₂-C₄-Alkenyl, C₂-C₄-Alkinyl, C₅-C₆-Cycloalkyl, bedeuten.

Bevorzugt sind Verbindungen der Formel (I) und deren Salze, worin R¹ und R² unabhängig voneinander Reste der Formel 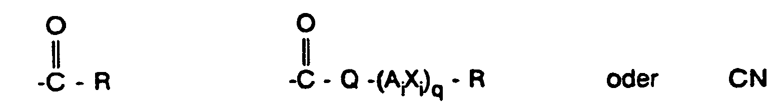
worin R, T, Q, Aᵢ, Xᵢ, und q die genannten Bedeutungen haben, bedeuten.

Die Erfindung betrifft auch ein Verfahren zum Schutz von Kulturpflanzen, vorzugsweise Getreide-, Reis- oder Maispflanzen, vor phytotoxischen Nebenwirkungen von Herbiziden, das dadurch gekennzeichnet ist, daß eine wirksame Menge mindestens einer Verbindung der in Formel I bzw. deren Salz vor, nach oder gleichzeitig mit dem obengenannten herbiziden Wirkstoff auf die Pflanzen, Pflanzensamen oder die Anbaufläche appliziert wird.

Die Erfindung betrifft weiterhin die Verwendung von Verbindungen der Formel I oder deren Salzen zum Schutz von Kulturpflanzen vor phytotoxischen Nebenwirkungen von Herbiziden.

Einige der Verbindungen der allgemeinen Formel I sind bekannt, wie z. B. 2-(Chinolin-8-yl-mercapto)-malonsäurediethylester und 2-(Chinolyl-8-mercapto)-acetessigsäureethylester (G. Buchmann, J. prakt. Chem. 1965, 141); 4-Chlor-phenoxymalonsäurediethylester (J. Izv. Sibirsk. Ord. Akad. Nauk. SSSR 1962 (11), 145-8, s. Chem. Abstracts 59: 5051 g (1963)). Aus US-A-3928602 Verbindungen der Formel I bekannt, worin Ar = Phenyl, das durch die Reste U¹, U² und U³ substituiert ist, wobei U¹ ein Rest aus der Gruppe Halogen, C₁-C₄₋Alkyl, C₁-C₄-Alkoxy, CF₃ und C₁-C₄-Alkylsulfonyl ist und U² und U³ gleich oder verschieden sind und jeweils aus der Gruppe bestehend aus Wasserstoff, Halogen, C₁-C₄-Alkyl, C₁-C₄-Alkoxy, CF₃ und C₁-C₄-Alkylsulfonyl ausgewählt sind, X=O, R¹ und R² jeweils eine Gruppe der Formel -COOR, R³ = C₁-C₄-Alkyl und R = gleiche oder verschiedene Reste aus der Gruppe Wasserstoff und C₁ -C₄ -Alkyl bedeuten. Aus FR-A-1465584 sind Verbindungen der Formel I bekannt, worin Ar = Phenyl, 1,3-Dichlorphenyl, 1,3,5-Trichlorphenyl, 3-Methoxyphenyl, Naphthyl, Cumarinyl, 4-Methyl-cumarinyl oder 7-Flavonyl, X = O, R¹ und R² jeweils eine Gruppe der Formel -COOR, R³ = Wasserstoff und R = gleiche oder verschiedene Reste aus der Gruppe Wasserstoff, Aryl, Alkyl und Aralkyl bedeuten. Ihre Safenerwirkung war aber bisher nicht bekannt. Gegenstand der Erfindung sind auch alle bisher nicht bekannten Verbindungen der Formel I.

Die Verbindungen der allgemeinen Formel I lassen sich nach allgemein bekannten Verfahren herstellen; siehe beispielsweise EP-A-4433; J. Am. Chem. Soc. 62 (1990) 1154; J. Org. Chem. 36 (1971) 3646; Chem. Abstr. 111 (1988) 133625 q; EP-A-326328; J. Am. Chem. Soc. 94 (1972) 712; Ukr. Khim. Zh. (Russ.Ed.) 56 (1990) 638; Chem. Abstr. 114 (1991) 42155 g; Chem. Pharm. Bull. 17 (1969) 419; Chem. Lett. 1973, 287; J. Chem. Soc. Chem. Comm. 1979, 50; Bull. Chem. Soc. Jpn. 45 (1972) 866; J. Org. Chem. 39 (1974) 1233 und dort zitierte Literatur. So kann die Herstellung der erfindungsgemäßen Verbindungen der Formel I in der Weise erfolgen, daß man
a) eine Verbindung der Formel Ar-X-H, in der Ar und X die bei Formel I genannte Bedeutung haben, mit einer Verbindung der Formel II, 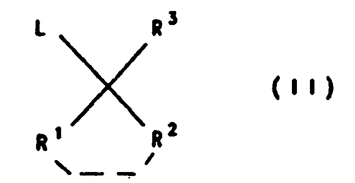
   worin
   - L: eine Abgangsgruppe, wie z. B. Chlor, Brom, Methansulfonyl oder Toluolsulfonyl, bedeutet und
   - R¹, R² und R³: wie bei der genannten Formel I definiert sind, umsetzt oder
b) eine Verbindung der Formel Ar-W mit einer Verbindung der Formel III, 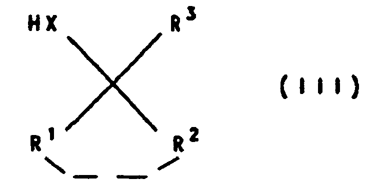
   wobei
   - W: eine Abgangsgruppe, wie z. B. Chlor, Brom, Methansulfonyl oder Toluolsulfonyl bedeutet und
   - Ar, X, R¹, R² und R³: wie bei der genannten Formel I definiert sind, umsetzt oder
c) eine Verbindung der Formel Ar-X-W mit einer Verbindung der Formel IV, 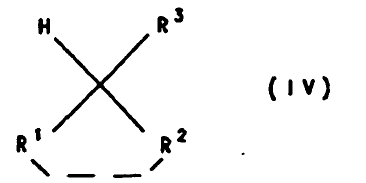
   wobei
   - W: eine Abgangsgruppe, wie z.B. Chlor, Methansulfonyl, Toluolsulfonyl, Dialkylamino, Diacylamino, Arylthio bedeutet, und
   - Ar, X, R¹, R² und R³: wie bei Formel I definiert sind,

   umsetzt oder
d) ein Aryl- oder Heteroaryloxycarbonsäurederivat der Formel V 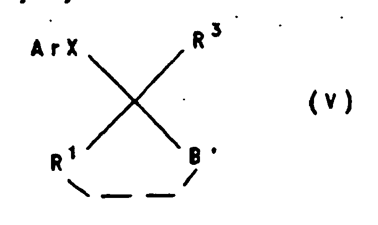
   worin
   Ar, X, R¹ und R³ wie bei Formel I definiert sind und B' eine Gruppe der Formeln 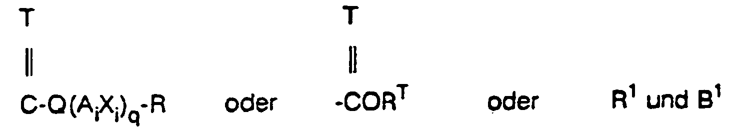 miteinander verbunden sind und gemeinsam eine Gruppe der Formel -CO-Q¹-D-Q²-CO- bedeuten, wobei T, Q, Aᵢ, Xᵢ, q, R, R^{T}, Q¹, Q² und D analog den gleichnamigen Resten in Formel I definiert sind, mit Alkoholen oder Mercaptanen umestert.

Die Umsetzungen nach Variante a) erfolgen vorzugsweise in dipolar aprotischen Lösungsmitteln wie Dimethylsulfoxid, N,N-Dimethylformamid, Methylisobutylketon, Dioxan oder Aceton bei erhöhter Temperatur, insbesondere zwischen 40 und 180°C in Gegenwart einer Base, insbesondere Alkalicarbonaten, wie z. B. Kaliumcarbonat.

Die Umsetzungen nach Variante b) werden vorzugsweise in aprotischen Lösungsmitteln wie Toluol, N,N-Dimethylformamid, Acetonitril, Methylisobutylketon, Dioxan oder Aceton bei erhöhter Temperatur, insbesondere zwischen 40 und 180°C, in Gegenwart einer Base, insbesondere Alkalicarbonaten, wie z. B. Kaliumcarbonat, durchgeführt.

Die Umsetzungen nach Variante c) erfolgen vorzugsweise in aprotischen Lösungsmitteln wie Dimethylsulfoxid, N,N-Dimethylformamid, Tetrahydrofuran, Dioxan, Methylenchlorid oder in Alkoholen wie Methanol, Ethanol bei Normaltemperatur bis erhöhter Temperatur, insbesondere zwischen 20 und 100°C in Gegenwart einer Base, insbesondere Alkalialkohotaten, wie z. B.
Natriummethanolat oder Natriumethanolat.

Die Umesterungen bzw. Amidierungen nach Variante d) erfolgen vornehmlich in der Weise, daß eine Verbindung der Formel V in Gegenwart von Titanalkoholaten als Katalysator mit den Alkoholen bzw. den Aminen bei erhöhten Temperaturen, insbesondere bei Rückflußtemperatur des Reaktionsgemisches, umgesetzt wird.

Verbindungen der Formel I reduzieren oder unterbinden phytotoxische Nebenwirkungen von Herbiziden, die beim Einsatz der Herbizide in Nutzpflanzenkulturen auftreten können, und können deshalb in üblicher Weise als Antidote oder Safener bezeichnet werden.

Die erfindungsgemäßen Verbindungen der Formel I können zusammen mit herbiziden Wirkstoffen oder in beliebiger Reihenfolge ausgebracht werden und sind dann in der Lage, schädliche Nebenwirkungen dieser Herbizide bei Kulturpflanzen zu reduzieren oder völlig aufzuheben, ohne die Wirksamkeit dieser Herbizide gegen Schadpflanzen zu beeinträchtigen.

Hierdurch kann das Einsatzgebiet herkömmlicher Pflanzenschutzmittel ganz erheblich erweitert werden. Herbizide, deren phytotoxische Nebenwirkungen auf Kulturpflanzen mittels Verbindungen der Formel I herabgesetzt werden können, sind z.B. Carbamate, Thiocarbamate, Halogenacetanilide, substituierte Phenoxy-, Naphthoxy- und Phenoxy-phenoxycarbonsäurederivate sowie Heteroaryloxyphenoxyalkancarbonsäurederivate, wie Chinolyloxy-, Chinoxalyloxy-, Pyridyloxy-, Benzoxalyloxy- und Benzthiazolyloxy-phenoxyalkancarbonsäureester, Cyclohexandionabkömmlinge, Imidazolinone, Pyrimidyloxy-pyridincarbonsäurederivate, Pyrimidyloxy-benzoesäure-derivate, Sulfonylhamstoffe, Triazolopyrimidin-sulfonamid-derivate und S-(N-Aryl-N-alkylcarbamoylmethyl)-dithiophosphorsäureester. Bevorzugt sind dabei Phenoxyphenoxy- und Heteroaryloxy-phenoxycarbonsäureester und -salze, Sulfonylharnstoffe und Imidazolinone.

Geeignete Herbizide, die mit den erfindungsgemäßen Safenern kombiniert werden können, sind beispielsweise:
A) Herbizide vom Typ der Phenoxyphenoxy- und Heteroaryloxyphenoxycarbonsäure-(C₁-C₄)alkyl-, (C₂-C₄)alkenyl- und (C₃₋C₄)alkinylester wie
A1) Phenoxy-phenoxy- und Benzyloxy-phenoxy-carbonsäure-derivate, z.B. 2-(4-(2,4-Dichlorphenoxy)-phenoxy)-propionsäuremethylester (Diclofop-methyl), 2-(4-(4-Brom-2-chlorphenoxy)-phenoxy)-propionsäuremethylester (s. DE-A-2601548),
   2-(4-(4-Brom-2-fluorphenoxy)-phenoxy)-propionsäuremethylester (s. US-A-4808750), 2-(4-(2-Chlor-4-trifluormethylphenoxy)-phenoxy)-propionsäuremethylester (s. DE-A-2433067),
   2-(4-(2-Fluor-4-trifluormethylphenoxy)-phenoxy)-propionsäuremethylester (s. US-A-4808750),
   2-(4-(2,4-Dichlorbenzyl)-phenoxy)propionsäuremethylester (s. DE-A-2417487),
   4-(4-(4-Trifluormethylphenoxy)-phenoxy)-pent-2-en-säureethylester,
   2-(4-(4-Trifluormethylphenoxy)-phenoxy)-propionsäuremethylester (s. DE-A-2433067),
A2) "Einkernige" Heteroaryloxy-phenoxy-alkancarbonsäurederivate, z.B.
   2-(4-(3,5-Dichlorpyridyl-2-oxy)-phenoxy)-propionsäureethylester (s. EP-A-2925),
   2-(4-(3,5-Dichlorpyridyl-2-oxy)-phenoxy)-propionsäurepropargylester (EP-A-3114),
   2-(4-(3-Chlor-5-trifluormethyl-2-pyridyloxy)-phenoxy-propionsäure-methylester (s. EP-A-3890),
   2-(4-(3-Chlor-5-trifluormethyl-2-pyridyloxy)-phenoxy)-propionsäure-ethylester (s. EP-A-3890),
   2-(4-(5-Chlor-3-fluor-2-pyridyloxy)-phenoxy)-propionsäurepropargylester (EP-A-191736),
   2-(4-(5-Trifluormethyl-2-pyridyloxy)-phenoxy)-propionsäurebutylester (Fluazifopbutyl),
A3) "Zweikernige" Heteroaryloxy-phenoxy-alkancarbonsäurederivate, z.B. 2-(4-(6-Chlor-2-chinoxalyloxy)-phenoxy)-propionsäuremethylester und -ethylester (Quizalofop-methyl und -ethyl),
   2-(4-(6-Fluor-2-chinoxalyloxy)-phenoxy)-propionsäuremethylester (s. J. Pest. Sci. Vol. 10, 61 (1985)),
   2-(4-(6-Chlor-2-chinoxalyloxy)-phenoxy)-propionsäure und -2-isopropylidenaminooxyethylester (Propaquizafop u. Ester),
   2-(4-(6-Chlorbenzoxazol-2-yl-oxy)-phenoxy)-propionsäureethylester (Fenoxapropethyl), dessen D(+) Isomer (Fenoxaprop-P-ethyl) und
   2-(4-(6-Chlorbenzthiazol-2-yloxy)-phenoxypropionsäureethylester (s. DE-A-2640730),
   2-(4-(6-Chlorchinoxalyloxy)-phenoxy-propionsäure-tetrahydrofur-2-ylmethyl-ester (s. EP-A 323 727),
B) Herbizide aus der Suffonylhamstoff-Reihe, wie z.B. Pyrimidin- oder Triazinylaminocarbonyl-[benzol-, pyridin-, pyrazol-, thiophen- und
   (alkylsulfonyl)alkylamino-]-sulfamide. Bevorzugt als Substituenten am Pyrimidinring oder Triazinring sind Alkoxy, Alkyl, Haloalkoxy, Haloalkyl, Halogen oder Dimethylamino, wobei alle Substituenten unabhängig voneinander kombinierbar sind. Bevorzugte Substituenten im Benzol-, Pyridin-, Pyrazol-, Thiophen- oder (Alkylsulfonyl)alkylamino-Teil sind Alkyl, Alkoxy, Halogen, Nitro, Alkoxycarbonyl, Aminocarbonyl, Alkylaminocarbonyl, Dialkylaminocarbonyl, Alkoxyaminocarbonyl, Alkyl, Alkoxyaminocarbonyl, Haloalkoxy, Haloalkyl, Alkylcarbonyl, Alkoxyalkyl, (Alkansulfonyl)alkylamino. Geeignete Sulfonylharnstoffe sind beispielsweise
B1) Phenyl- und Benzylsulfonylharnstoffe und verwandte Verbindungen, z.B.
   1-(2-Chlorphenylsulfonyl)-3-(4-methoxy-6-methyl-1,3,5-triazin-2-yl)harnstoff (Chlorsulfuron),
   1-(2-Ethoxycarbonylphenylsulfonyl)-3-(4-chlor-6-methoxypyrimidin-2-yl)harnstoff (Chlorimuron-ethyl),
   1-(2-Methoxyphenylsulfonyl)-3-(4-methoxy-6-methyl-1,3,5-triazin-2-yl)harnstoff (Metsulfuron-methyl),
   1-(2-Chlorethoxy-phenylsulfonyl)-3-(4-methoxy-6-methy)-1,3,5-triazin-2-yl)harnstoff (Triasulfuron),
   1-(2-Methoxycarbonyl-phenylsulfonyl)-3-(4,6-dimethyl-pyrimidin-2-yl)harnstoff (Sutfometuron-methyl),
   1-(2-Methoxycarbonylphenylsulfonyl)-3-(4-methoxy-6-methyl-1,3,5-triazin-2-yl)-3-methylhamstoff (Tribenuron-methyl),
   1-(2-Methoxycarbonylbenzylsulfonyl)-3-(4,6-dimethoxy-pyrimidin-Z-yl)harnstoff (Bensulfuron-methyl),
   1-(2-Methoxycarbonylphenylsulfonyl)-3-(4,6-bis-(difluormethoxy)pyrimidin-2-yl)harnstoff (Primisulfuron-methyl),
   3-(4-Ethyl-6-methoxy-1,3,5-triazin-2-yl)-1-(2,3-dihydro-1,1-dioxo-2-methylbenzo[b]thiophen-7-sulfonyl)-harnstoff (s. EP-A-79683),
   3-(4-Ethoxy-6-ethyl-1,3,5-triazin-2-yl)-1-(2,3-dihydro-1,1-dioxo-2-methylbenzo[b]thiophen-7-sulfonyl)-harnstoff (s. EP-A-79683),
B2) Thienylsulfonylharnstoffe, z.B. 1-(2-Methoxycarbonylthiophen-3-yl)-3-(4-methoxy-6-methyl-1,3,5-triazin-2-yl)harnstoff (Thifensuffuron-methyl),
B3) Pyrazolylsulfonylharnstoffe, z.B.
   1-(4-Ethoxycarbonyl-1-methylpyrazol-5-yl-sulfonyl)-3-(4,6-dimethoxypyrimidin-2-yl)harnstoff (Pyrazosulfuron-methyl),
   3-Chlor-5-(4,6-dimethoxypyrimidin-2-ylcarbamoylsulfamoyl)-1-methyl-pyrazol-4-carbonsäuremethylester (s. EP 282613),
   5-(4,6-Dimethylpyrimidin-2-yl-carbamoylsulfamoyl)-1-(2-pyridyl)-pyrazol-4-carbonsäuremethylester (NC-330, s. Brighton Crop Prot. Conference - Weeds - 1991, Vol. 1, 45 ff.),
B4) Sulfondiamid-Derivate, z.B.
   3-(4,6-Dimethoxypyrimidin-2-yl)-1-(N-methyl-N-methylsulfonylaminosulfonyl)harnstoff (Amidosulfuron) und Strukturanaloge (s. EP-A-0131258 und Z. Pfl. Krankh. Pfl. Schutz 1990, Sonderheft XII, 489-497),
B5) Pyridylsulfonylharnstoffe, z.B.
   1-(3-N,N-Dimethylaminocarbonylpyridin-2-yl-sulfonyl)-3-(4,6-dimethoxypyrimidin-2-yl)harnstoff (Nicosulfuron),
   1-(3-Ethylsulfonylpyridin-2-yl-sulfonyl)-3-(4,6-dimethoxy-pyrimidin-2-yl)harnstoff (DPX-E 9636, s. Brighton Crop Prot. Conf. - Weeds - 1989, S. 23 ff.), Pyridylsulfonylharnstoffe, wie sie in DE-A-4000503 und DE-A-4030577 beschrieben sind, vorzugsweise solche der Formel 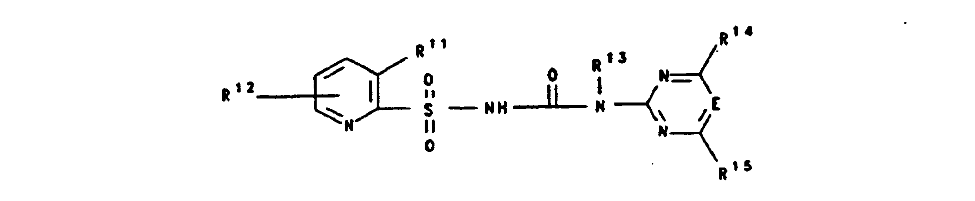
   worin
   - E: CH oder N vorzugsweise CH,
   - R¹¹: Iod oder NR¹⁶R¹⁷,
   - R¹²: H, Halogen, Cyano, C₁-C₃-Alkyl, C₁-C₃-Alkoxy, C₁-C₃-Haloalkyl, C₁-C₃-Haloalkoxy, C₁-C₃-Alkylthio, (C₁-C₃-Alkoxy)-C₁-C₃-alkyl, (C₁-C₃-Alkoxy)-carbonyl, Mono- oder Di-(C₁-C₃-alkyl)-amino, C₁-C₃-Alkyl-sulfinyl oder -sulfonyl, SO₂-NR^{a}R^{b} oder CO-NR^{a}R^{b}, insbesondere H,
   - R^{a},R^{b}: unabhängig voneinander H, C₁-C₃-Alkyl, C₁-C₃-Alkenyl, C₁-C₃-Alkinyl oder zusammen -(CH₂)₄-, -(CH₂)₅- oder (CH₂)₂-O-(CH₂)₂-,
   - R¹³: H oder CH₃,
   - R¹⁴: Halogen, C₁-C₂-Alkyl, C₁-C₂-Alkoxy, C₁-C₂-Haloalkyl, vorzugsweise CF₃, C₁-C₂-Haloalkoxy, vorzugsweise OCHF₂ oder OCH₂CF₃,
   - R¹⁵: C₁-C₂-Alkyl, C₁-C₂-Haloalkoxy, vorzugsweise OCHF₂, oder C₁-C₂-Alkoxy, und
   - R¹⁶: C₁-C₄-Alkyl und
   - R¹⁷: C₁-C₄-Alkylsulfonyl oder
   - R¹⁶ und R¹⁷: gemeinsam eine Kette der Formel -(CH₂)₃SO₂- oder -(CH₂)₄SO₂ bedeuten,
   z.B. 3-(4,6-Dimethoxypyrimidin-2-yl)-1-(3-N-methylsulfonyl-N-methylaminopyridin-2-yl)sulfonylharnstoff, oder deren Salze,
B6) Alkoxyphenoxysuffonylhamstoffe, wie sie in EP-A-0342569 beschrieben sind, vorzugsweise solche der Formel 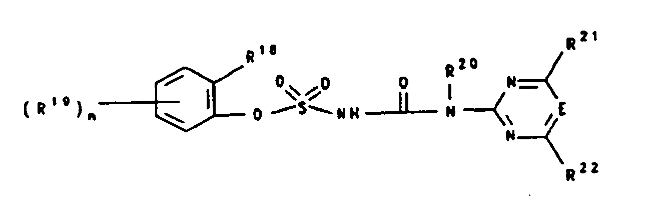
   worin
   - E: CH oder N, vorzugsweise CH,
   - R¹⁸: Ethoxy, Propoxy oder Isopropoxy,
   - R¹⁹: Wasserstoff, Halogen, NO₂, CF₃, CN, C₁-C₄-Alkyl, C₁-C₄-Alkoxy, C₁-C₄-Alkylthio oder (C₁-C₃-Alkoxy)-carbonyl, vorzugsweise in 6-Position am Phenylring,
   - n: 1, 2 oder 3, vorzugsweise 1,
   - R²⁰: Wasserstoff, C₁-C₄-Alkyl oder C₃-C₄-Alkenyl,
   - R²¹,R²²: unabhängig voneinander Halogen, C₁-C₂-Alkyl, C₁-C₂-Alkoxy, C₁-C₂-Hatoalkyl, C₁-C₂-Haloalkoxy oder (C₁-C₂-Alkoxy)-C₁-C₂-alkyl, vorzugsweise OCH₃ oder CH₃, bedeuten, z.B. 3-(4,6-Dimethoxypyrimidin-2-yl)-1-(2-ethoxyphenoxy)-sulfonylharnstoff, oder deren Salze,

   und andere verwandte Sulfonylharnstoffderivate und Mischungen daraus,
C) Chloracetanilid-Herbizide wie
   N-Methoxymethyl-2,6-diethyl-chloracetanilid (Alachlor),
   N-(3'-Methoxyprop-2'-yl)-2-methyl-6-ethyl-chloracetanilid (Metolachlor),
   N-(3-Methyl-1,2,4-oxdiazol-5-yl-methyl)-chloressigsäure-2,6-dimethylanilid,
   N-(2,6-Dimethylphenyl)-N-(1-pyrazolylmethyl)-chloressigsäureamid (Metazachlor),
D) Thiocarbamate wie
   S-Ethyl-N,N-dipropykhiocarbamat (EPTC) oder
   S-Ethyl-N,N-diisobutylthiocarbamat (Butylate),
E) Cyclohexandion-Derivate wie
   3-(1-Allyloxyiminobutyl)-4-hydroxy-6,6-dimethyl-2- oxocyclohex-3-encarbonsäuremethylester (Alloxydim),
   2-(1-Ethoximinobutyl)-5-(2-ethylthiopropyl)-3-hydroxy-cyclohex-2-en-1-on (Sethoxydim),
   2-(1-Ethoximinobutyl)-5-(2-phenylthiopropyl)-3-hydroxy-cyclohex-2-en-1-on (Cloproxydim),
   2-(1-(3-Chlorallyloxy)iminobutyl)-5-[2-(ethylthio)propyl]-3-hydroxy-cyclohex-2-en-1-on,
   2-(1-(3-Chlorallyloxy)iminopropyl)-5-[2-(ethylthio)propyl]-3-hydroxy-cyclohex-2-en-1-on (Clethodim),
   2-(1-(Ethoxyimino)-butyl)-3-hydroxy-5-(thian-3-yl)-cyclohex-2-enon (Cycloxydim), oder
   2-(1-Ethoxyiminopropyl)-5-(2,4,6-trimethylphenyl)-3-hydroxy-cyclohex-2-en-1-on (Tratkoxydim),
F) 2-(4-Alkyl-5-oxo-2-imidazolin-2-yl)-benzoesäurederivate oder 2-(4-Alkyl-5-oxo-2-imidazolin-2yl)-heteroarylcarbonsäurederivate wie z.B.
   2-(4-Isopropyl-4-methyl-5-oxo-2-imidazolin-2-yl)-5-methylbenzoesäuremethylester und
   2-(4-Isopropyl-4-methyl-5-oxo-2-imidazolin-2-yl)-4-methylbenzoesäure (Imazamethabenz),
   5-Ethyl-2-(4-isopropyl-4-methyl-5-oxo-2-imidazolin-2-yl)-pyridin-3-carbonsäure (Imazethapyr),
   2-(4-Isopropyl-4-methyl-5-oxo-2-imidazolin-2-yl)-chinolin-3-carbonsäure (Imazaquin),
   2-(4-Isopropyl-4-methyl-5-oxo-2-imidazolin-2-yl)-pyridin-3-carbonsäure (Imazapyr),
   5-Methyl-2-(4-Isopropyl-4-methyl-5-oxo-2-imidazolin-2yl)-pyridin-3-carbonsäure (Imazethamethapyr),
G) Triazolopyrimidinsulfonamidderivate, z. B.
   N-(2,6-Difluorphenyl)-7-methyl-1,2,4-triazolo-(1,5-c)-pyrimidin-2-sulfonamid (Flumetsulam),
   N-(2,6-Dichlor-3-methylphenyl)-5,7-dimethoxy-1,2,4-triazolo-(1,5-c)-pyrimidin-2-sulfonamid.
   N-(2,6-Difluorphenyl)-7-fluor-5-methoxy-1,2,4-triazolo-(1,5-c)-pyrimidin-2-sulfonamid
   N-(2,6-Dichlor-3-methylphenyl)-7-chlor-5-methoxy-1,2,4-triazolo-(1,5-c)-pyrimidin-2-sulfonamid,
   N-(2-Chlor-6-methoxycarbonyl)-5,7-dimethyl-1,2,4-triazolo-(1,5-c)-pyrimidin-2-sulfonamid
   (siehe z. B. EP-A-343 752, US- 4 988 812),
H) Benzoylcyctohexandionderivate, z. B.
   2-(2-Chlor-4-methylsulfonylbenzoyl)-cyclohexan-1,3-dion (SC-0051, s.EP-A-137963),
   2-(2-Nitrobenzoyl)-4,4-dimethyl-cyclohexan-1,3-dion (s. EP-A-274634),
   2-(2-Nitro-3-methylsulfonylbenzoyl)-4,4-dimethyl-cyclohexan-1,3-dion (s. WO-91/13548),
J) Pyrimidinyloxy-pyrimidincarbonsäure- bzw. Pyrimidinyloxy-benzoesäure-Derivate, z.B.
   3-(4,6-Dimethoxypyrimidin-2-yl)-oxy-pyridin-2-carbonsäurebenzylester
   (EP-A-249 707),
   3-(4,6-Dimethoxypyrimidin-2-yl)-oxy-pyridin-2-carbonsäuremethylester
   (EP-A-249 707),
   2,6-Bis[(4,6-dimethoxypyrimidin-2-yl)-oxy]-benzoesäure (EP-A-321 846),
   2,6-Bis[(4,6-dimethoxypyrimidin-2-yl)-oxy]-benzoesäure-(1-ethoxycarbonyloxyethyl)-ester (EP-A-472 113) und
K) S-(N-Aryl-N-alkyl-carbamoylmethyl)-dithiophosphorsäureester wie
   S-[N-(4-Chlorphenyl)-N-isopropyl-carbamoylmethyl]-O,O-dimethyl-dithiophosphat (Anilofos).

Die obengenannten Herbizide der Gruppen A bis K sind dem Fachmann bekannt und in der Regel in "The Pesticide Manual", British Crop Protection Council, 9. Auflage 1991 oder 8. Auflage 1987 oder in "Agricultural Chemicals Book II, Herbicides", by W.T. Thompson, Thompson Publications, Fresno CA, USA 1990 oder in "Farm Chemicals Handbook '90", Meister Publishing Company, Willoughby OH, USA 1990 beschrieben. Imazethamethapyr ist aus Weed Techn. 1991, Vol. 5, 430-438 bekannt.

Die herbiziden Wirkstoffe und die erwähnten Safener können zusammen (als fertige Formulierung oder im Tank-mix-Verfahren) oder in beliebiger Reihenfolge nacheinander ausgebracht werden. Das Gewichtsverhältnis Safener:Herbizid kann innerhalb weiter Grenzen variieren und ist vorzugsweise im Bereich von 1:10 bis 10:1, insbesondere von 1:10 bis 5:1. Die jeweils optimalen Mengen an Herbizid und Safener sind vom Typ des verwendeten Herbizids oder vom verwendeten Safener sowie von der Art des zu behandelnden Pflanzenbestandes abhängig und lassen sich von Fall zu Fall durch entsprechende Vorversuche ermitteln.

Haupteinsatzgebiete für die Anwendung der Safener sind vor allem Getreidekulturen (Weizen, Roggen, Gerste, Hafer), Reis, Mais, Sorghum, aber auch Baumwolle und Sojabohne, vorzugsweise Getreide, Reis und Mais.

Ein besonderer Vorteil der erfindungsgemäßen Safener der Formel I ist bei deren Kombination mit Herbiziden aus der Gruppe der Sulfonylharnstoffe und/oder Imidazolinone sowie mit Herbiziden vom Typ der Phenoxyphenoxy- und Heteroaryloxy-phenoxy-alkancarbonsäurederivate festzustellen.

Einige Herbizide dieser Strukturklassen können speziell in Getreidekulturen und/oder Mais sowie Reis nicht oder nicht genügend selektiv eingesetzt werden. Durch die Kombination mit den erfindungsgemäßen Safenern sind auch bei diesen Herbiziden in Getreide, Mais oder Reis hervorragende Selektivitäten zu erreichen.

Die Safener der Formel I können je nach ihren Eigenschaften zur Vorbehandlung des Saatgutes der Kulturpflanze (Beizung der Samen) verwendet werden oder vor der Saat in die Saatfurchen eingebracht oder zusammen mit dem Herbizid vor oder nach dem Auflaufen der Pflanzen angewendet werden. Vorauflaufbehandlung schließt sowohl die Behandlung der Anbaufläche vor der Aussaat als auch die Behandlung der angesäten, aber noch nicht bewachsenen Anbauflächen ein. Bevorzugt ist die gemeinsame Anwendung mit dem Herbizid. Hierzu können Tankmischungen oder Fertigformulierungen eingesetzt werden.

Die benötigten Aufwandmengen der Safener können je nach Indikation und verwendetem Herbizid innerhalb weiter Grenzen schwanken und liegen in der Regel im Bereich von 0,001 bis 5 kg, vorzugsweise 0,005 bis 0,5 kg Wirkstoff je Hektar.

Gegenstand der vorliegenden Erfindung ist deshalb auch ein Verfahren zum Schutz von Kulturpflanzen vor phytotoxischen Nebenwirkungen von Herbiziden, das dadurch gekennzeichnet ist, daß eine wirksame Menge einer Verbindung der Formel I vor, nach oder gleichzeitig mit dem Herbizid auf die Pflanzen, Pflanzensamen oder die Anbaufläche appliziert wird.

Gegenstand der Erfindung sind auch pflanzenschützende Mittel, die einen Wirkstoff der Formel I und übliche Formulierungshilfsmittel enthalten, sowie herbizide Mittel, die einen Wirkstoff der Formel I und ein Herbizid sowie im Bereich des Pflanzenschutzes übliche Formulierungshilfsmittel enthalten.

Die Verbindungen der Formel I und deren Kombinationen mit einem oder mehreren der genannten Herbizide können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), wasserlösliche Pulver (SP), wasserlösliche Konzentrate (SL), konzentrierte Emulsionen (EW) wie Öl-in-Wasser und Wasser-in- Öl-Emulsionen, versprühbare Lösungen oder Emulsionen, Kapselsuspensionen (CS), Dispersionen auf Öl- oder Wasserbasis (SC), Suspoemulsionen, Suspensionskonzentrate, Stäubemittel (DP), ölmischbare Lösungen (OL), Beizmittel, Granulate (GR) in Form von Mikro-, Sprüh-, Aufzugs- und Adsorptionsgranulaten, Granulate für die Boden- bzw. Streuapplikation, wasserlösliche Granulate (SG), wasserdispergierbare Granulate (WG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie" Band 7, C. Hauser Verlag München, 4. Aufl. 1986; Wade van Valkenburg, "Pesticide Formulations", Marcel Dekker N.Y., 1973; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marsden, "Solvents Guide", 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler "Chemische Technolgie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole und Fettamine, Fettalkoholpolyglykolethersulfate, Alkansulfonate oder Alkylarylsulfonate und Dispergiermittel, z.B. ligninsulfonsaures Natrium, 2,2-dinaphthylmethan-6,6'-disulfonsaures Natrium, dibutylnaphthalinsulfonsaures Natrium oder auch oleoylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte (z.B. Blockpolymere), Alkylpolyether, Sorbitanfettsäureester, Polyoxyethylensorbitanfettsäureester oder Polyoxethylensorbitester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Die agrochemischen Zubereitungen enthalten in der Regel 0,1 bis 99 Gewichtsprozent, insbesondere 0,1 bis 95 Gew.-%, Wirkstoffe der Formel I (Antidot) oder des Antidot/Herbizid-Wirkstoffgemischs und 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

In Spritzpulvern beträgt die Wirkstoffkonzentration z.B. etwa 10 bis 90 Gew.%, der Rest zu 100 Gew.-% besteht aus üblichen Formulierungsbestandteilen. Bei emulgierbaren Konzentraten beträgt die Wirkstoffkonzentration etwa 1 bis 80 Gew.-% Wirkstoffe. Staubförmige Formulierungen enthalten etwa 1 bis 20 Gew.-% an Wirkstoffen, versprühbare Lösungen etwa 0,2 bis 20 Gew.-% Wirkstoffe. Bei Granulaten wie wasserdispergierbaren Granulaten hängt der Wirkstoffgehalt zum Teil davon ab, ob die wirksame Verbindung flüssig oder fest vorliegt. In der Regel liegt der Gehalt bei den in Wasser dispergierbaren Granulaten zwischen 10 und 90 Gew.-%.

Daneben enthalten die genannten Wirkstofformulierungen gegebenenfalls die jeweils üblichen Haft-, Netz-, Dispergier- , Emulgier-, Penetrations-, Lösungsmittel, Füll- oder Trägerstoffe.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser. Staubförmige Zubereitungen, Granulate sowie versprühbare Lösungen werden vor der Anwendung üblicherweise nicht mehr mit weiteren inerten Stoffen verdünnt. Mit den äußeren Bedingungen wie Temperatur, Feuchtigkeit, der Art des verwendeten Herbizids u.a. variiert die erforderliche Aufwandmenge der "Antidots".

Folgende Beispiele dienen zur Erläuterung der Erfindung:
A. Formulierungsbeispiele
   a) Ein Stäubmittel wird erhalten, indem man 10 Gew.-Teile einer Verbindung der Formel I oder eines Wirkstoffgemischs aus einem Herbizid und eine Verbindung der Formel I und 90 Gew.-Teile Talkum als Inertstoff mischt und in einer Schlagmühle zerkleinert.
   b) Ein in Wasser leicht dispergierbares, benetzbares Pulver wird erhalten, indem man 25 Gewichtsteile einer Verbindung der Formel I oder eines Wirkstoffgemischs aus einem Herbizid und einem Safener der Formel I, 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
   c) Ein in Wasser leicht dispergierbares Dispersionskonzentrat wird erhalten, indem man 20 Gewichtsteile einer Verbindung der Formel I oder eines Wirkstoffgemischs aus einem Herbizid und einem Safener der Formel I, 6 Gew.-Teilen Alkylphenolpolyglykolether (^{R}Triton X 207), 3 Gew.-Teilen Isotridecanolpolyglykolether (8 EO) und 71 Gew.- Teilen paraffinischem Mineralöl (Siedebereich z.B. ca. 255 bis über 277°C) mischt und in einer Reibkugelmühle auf eine Feinheit von unter 5 Mikron vermahlt.
   d) Ein emulgierbares Konzentrat wird erhalten aus 15 Gew.-Teilen einer Verbindung der Formel I oder eines Wirkstoffgemischs aus einem Herbizid und einem Safener der Formel I, 75 Gew.-Teilen Cyclohexanon als Lösemittel und 10 Gew.-Teilen oxethyliertem Nonylphenol als Emulgator.
   e) Ein in Wasser dispergierbares Granulat wird erhalten, indem man 75 Gew.-Teile einer Verbindung der Formel I oder eines Wirkstoffgemischs aus einem Herbizid und einem Safener der Formel I,
      10 " ligninsulfonsaures Calcium,
      5 " Natriumlaurylsulfat,
      3 ° Polyvinylalkohol und
      7 " Kaolin
      mischt, auf einer Stiftmühle mahlt und das Pulver in einem Wirbelbett durch Aufsprühen von Wasser als Granulierflüssigkeit granuliert.
   f) Ein in Wasser dispergierbares Granulat wird auch erhalten, indem man 25 Gew.-Teile einer Verbindung der Formel I oder eines Wirkstoffgemischs aus einem Herbizid und einem Safener der Formel I,
      5 " 2,2'-dinaphthylmethan-6,6'-disulfonsaures Natrium,
      2 " oleoylmethyltaurinsaures Natrium,
      1 " Polyvinylalkohol,
      17 " Calciumcarbonat und
      50 " Wasser
      auf einer Kolloidmühle homogensiert und vorzerkleinert, anschließend auf einer Perlmühle mahlt und die so erhaltene Suspension in einem Sprühturm mittels einer Einstoffdüse zerstäubt und trocknet.

### B. Herstellungsbeispiele

### 1. 2-Phenoxymalonsäurediethylester (Beispiel 2 aus Tabelle 1):

22,1 g (160 mmol) Kaliumcarbonat wurden in 30 ml Aceton suspendiert, mit 7,5 g (80 mmol) Phenol in 100 ml Aceton versetzt und 1 h auf Rückflußtemperatur erhitzt. Anschießend wurden 15,5 g (80 mmol) 2-Chlomnalonsäurediethylester in 100 ml Aceton zugetropft und das Gemisch 10 h unter Rückfluß gekocht. Man engte im Vakuum ein und nahm den Rückstand in Methylenchlorid auf. Die organische Phase wurde mit gesättigter NaHCO₃-Lösung und gesättigter NaCl-Lösung gewaschen, über Magnesiumsulfat getrocknet und eingeengt. Säulenchromatographie (Kieselgel, Heptan/Diethylether 2:1) des Rückstandes ergab 15,5 (77 % d. Th.) 2-Phenoxymalonsäurediethylester als farblose Flüssigkeit.

### 2. 2-(3,4-Dichlorphenoxy)-3-keto-butansäureethylester (Beispiel 38 aus Tabelle 1):

13,0 g (80mmol) 3,4-Dichlorphenol und 12,2 g (88 mmol) Kaliumcarbonat wurden in 400 ml Aceton 30 min auf Rückflußtemperatur erhitzt. Anschließend wurden 15,8 g (96 mmol) 2-Chloracetessigsäureethylester zugetropft und das Gemisch 8 h unter Rückfluß gekocht. Man engte im Vakuum ein und versetzte den Rückstand mit Wasser. Die wäßrige Phase wurde dreimal mit Ethylacetat extrahiert, die vereinigten organischen Phasen über Magnesiumsulfat getrocknet und eingeengt. Säulenchromatographie des Rückstandes ergab 15,8 g (68% d. Th.) 2- (3,4-Dichlorphenoxy)-3-keto-butansäureethylester als Öl.

In den nachfolgenden Tabellen 1 und 2 sind die obengenannten Herstellungsbeispiele mit weiteren Beispielen für Verbindungen der Formel I aufgeführt, die in analoger Weise hergestellt werden.

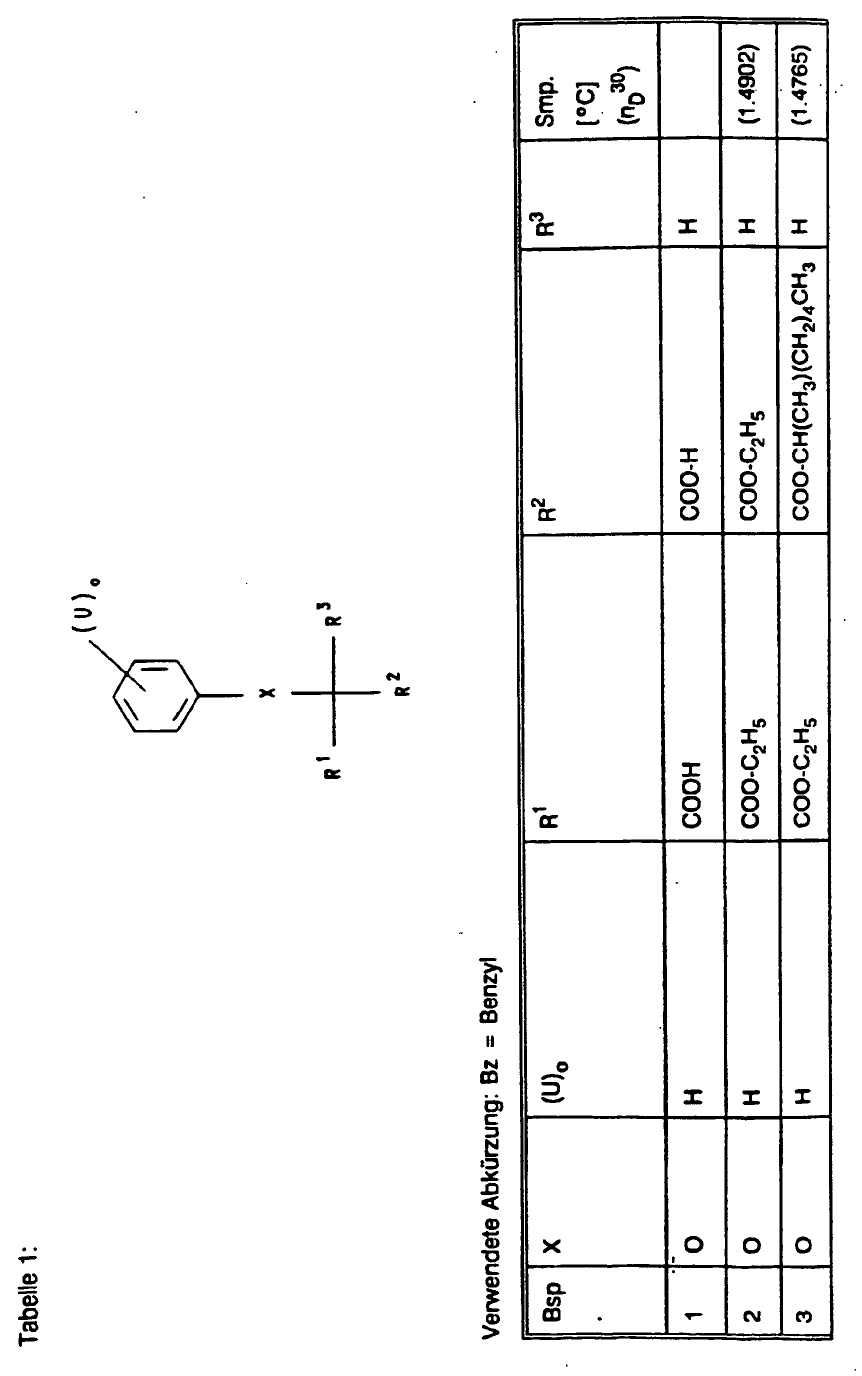

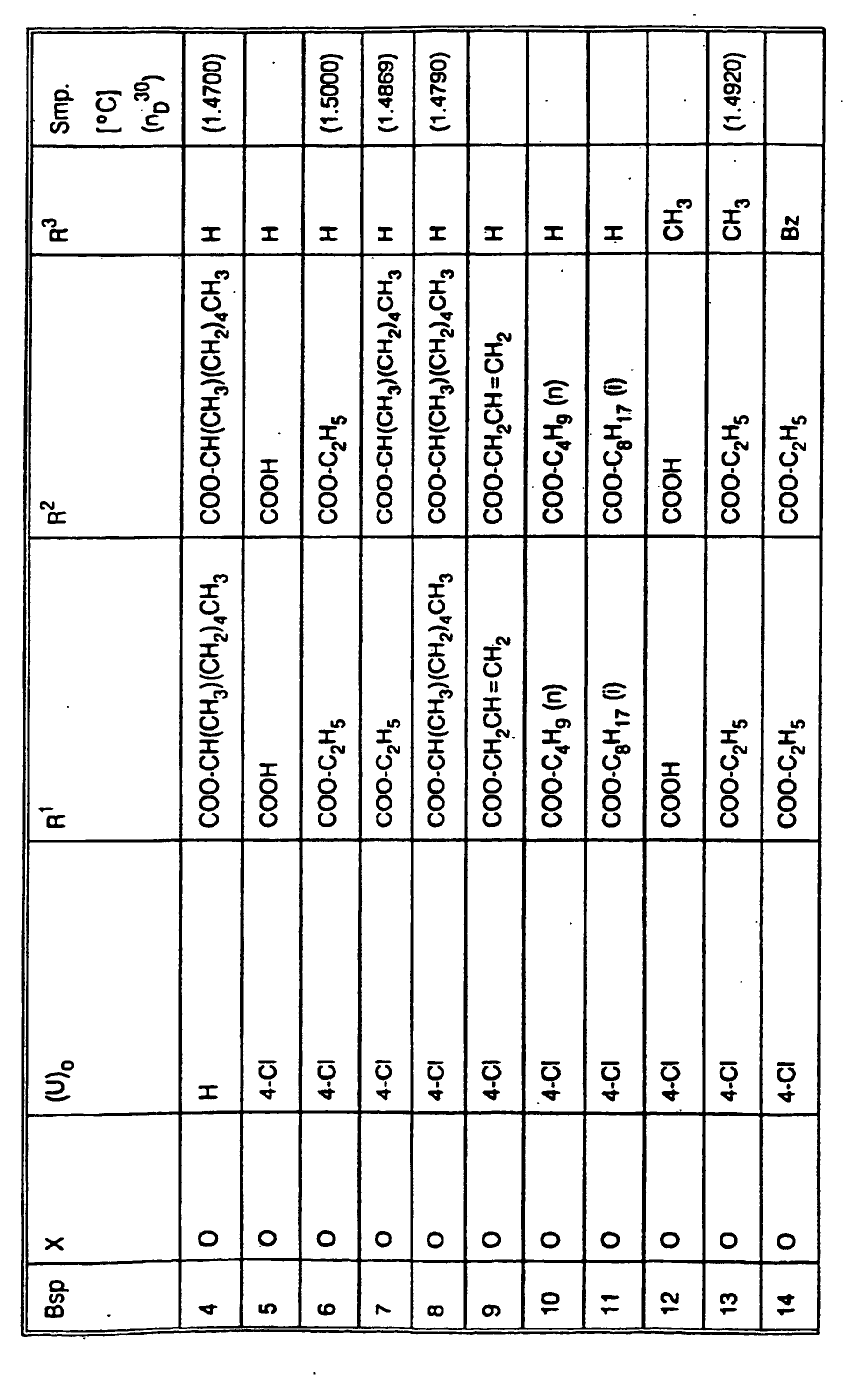

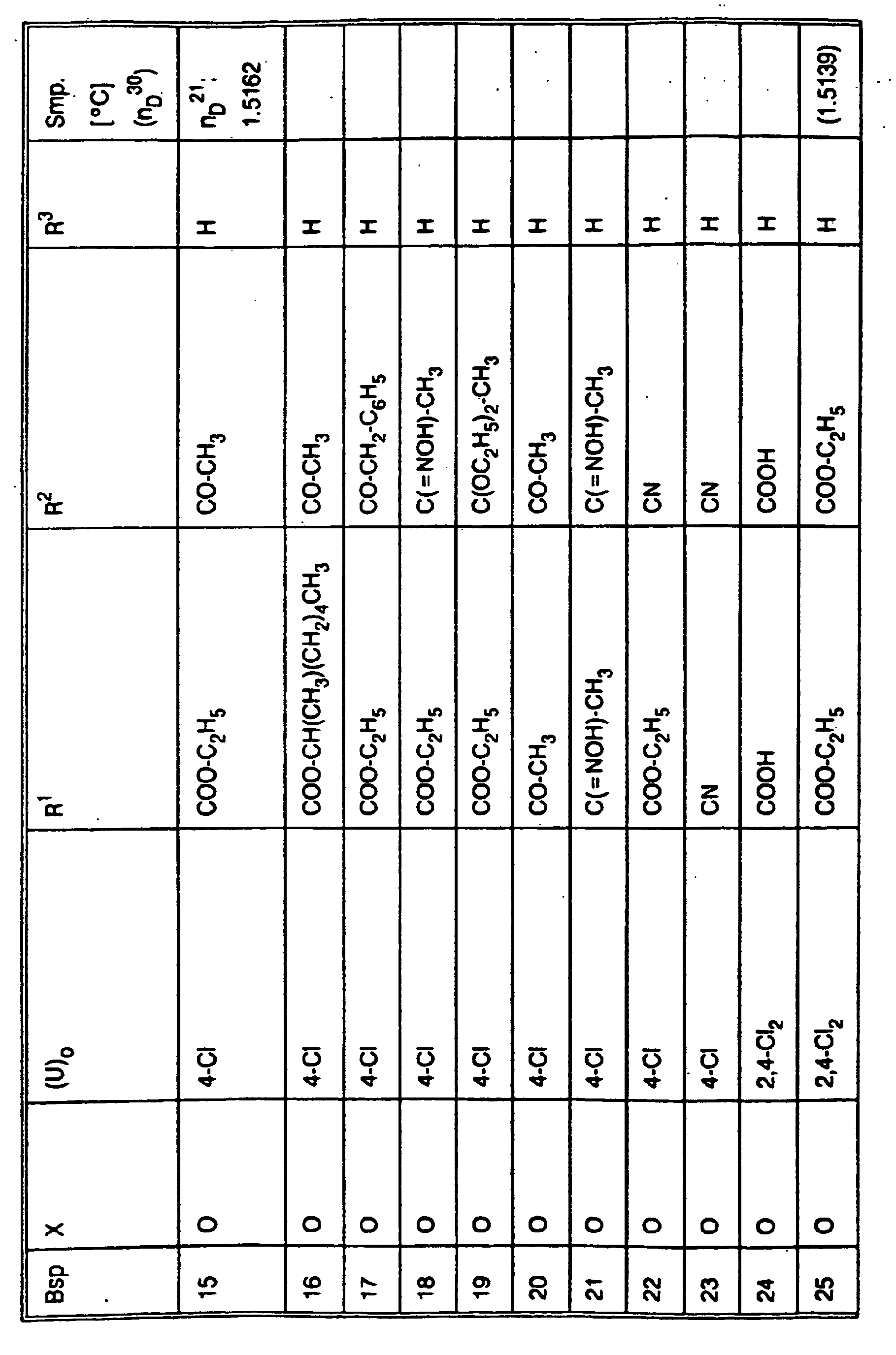

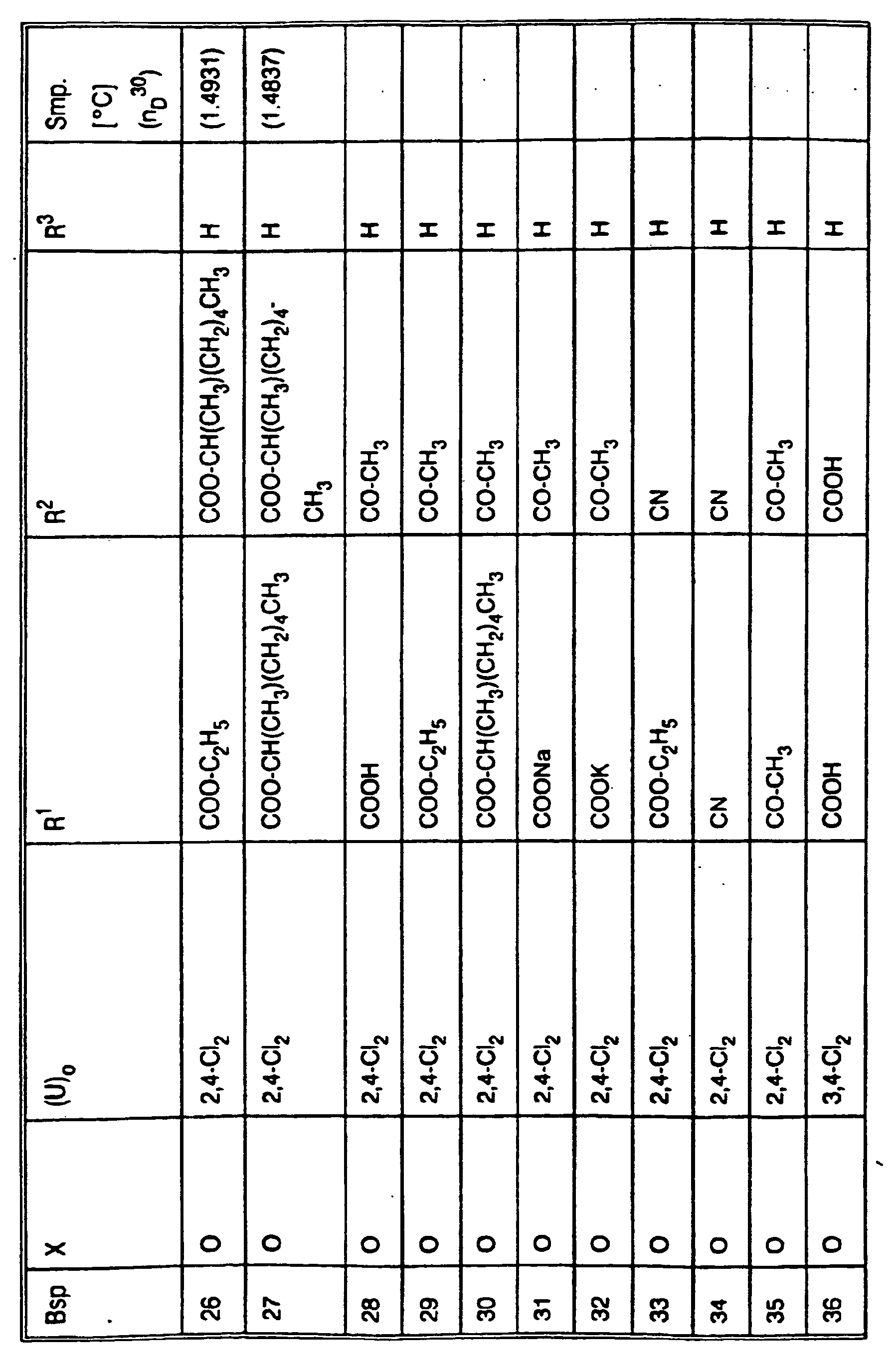

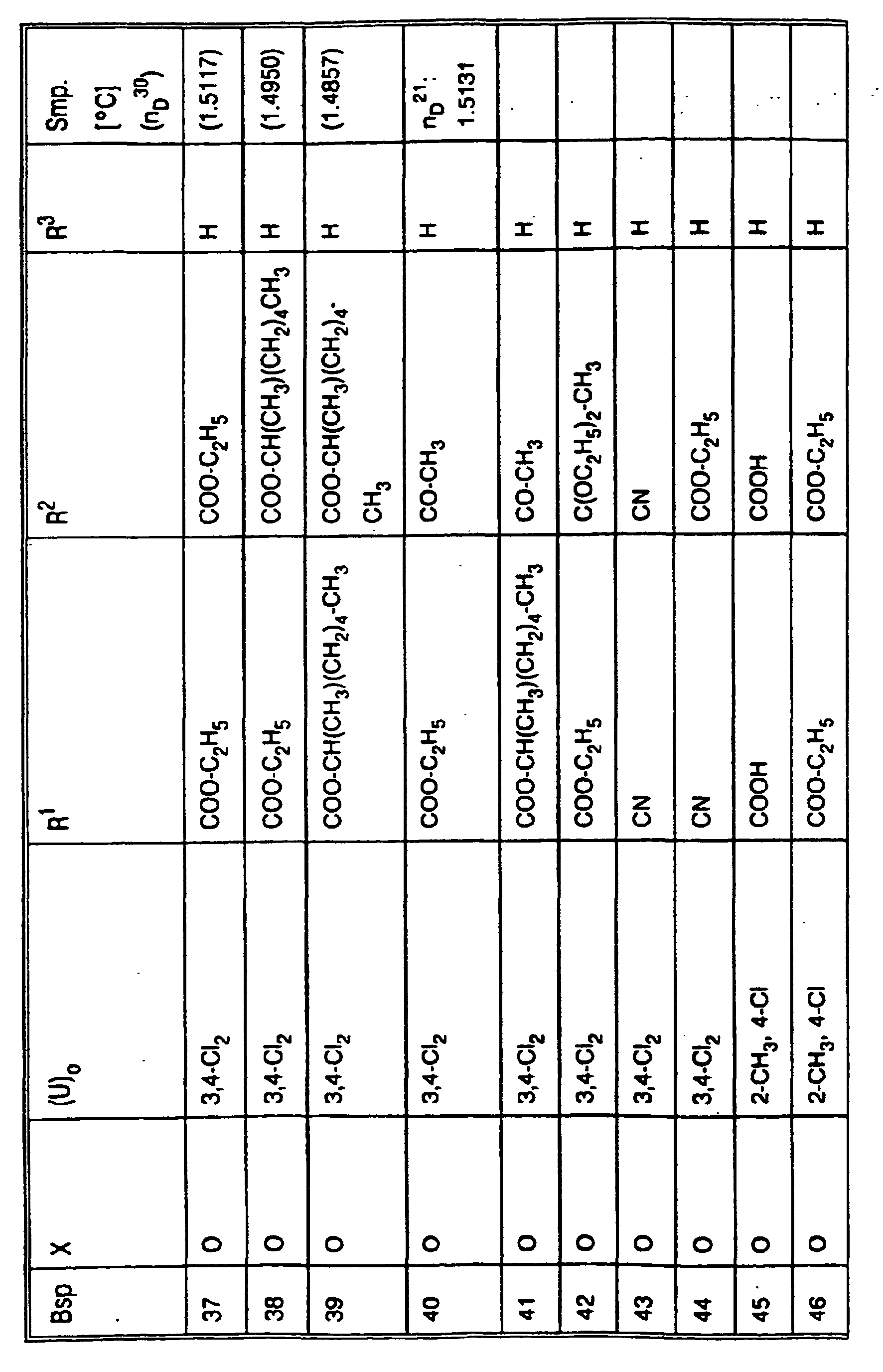

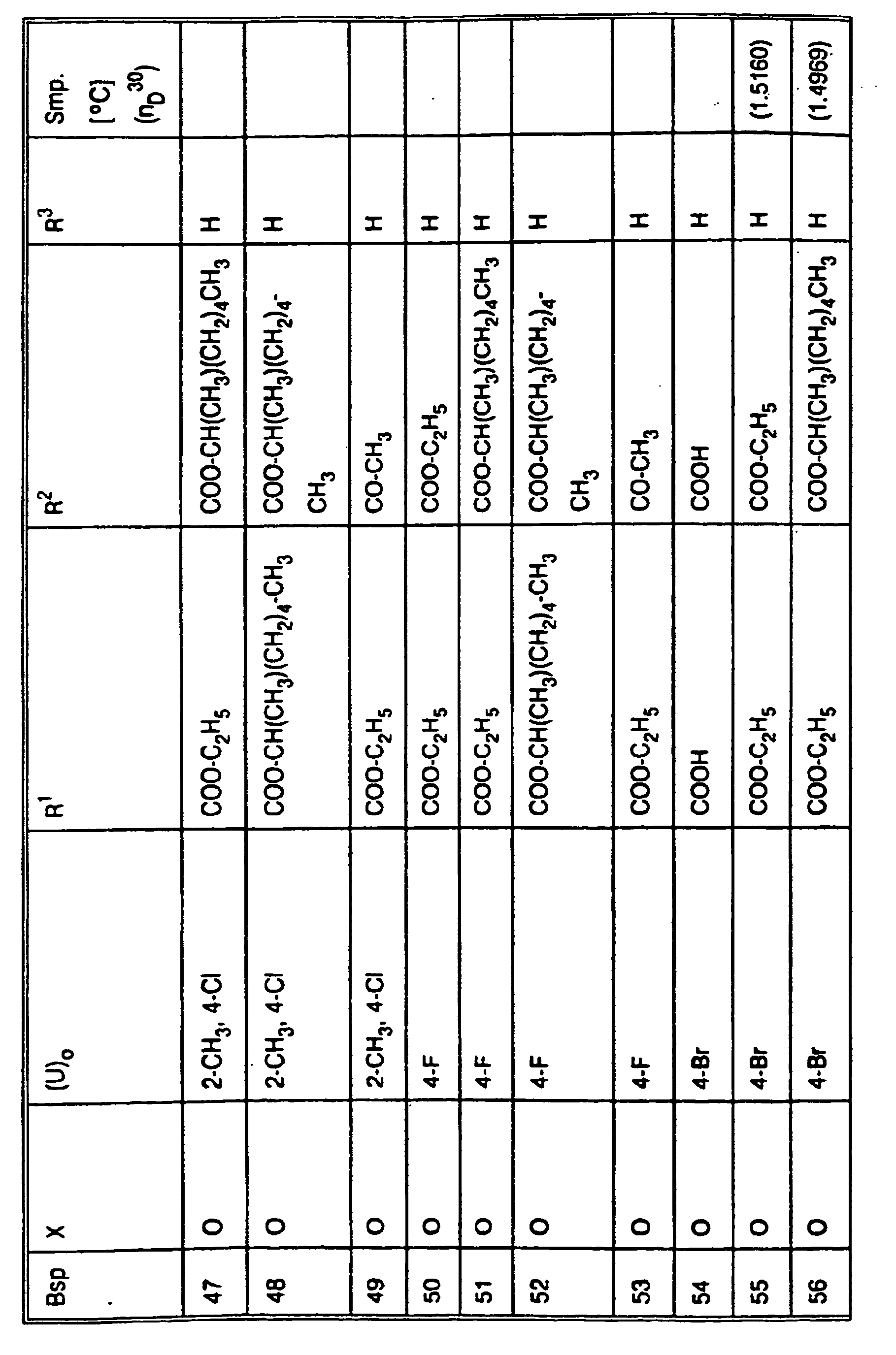

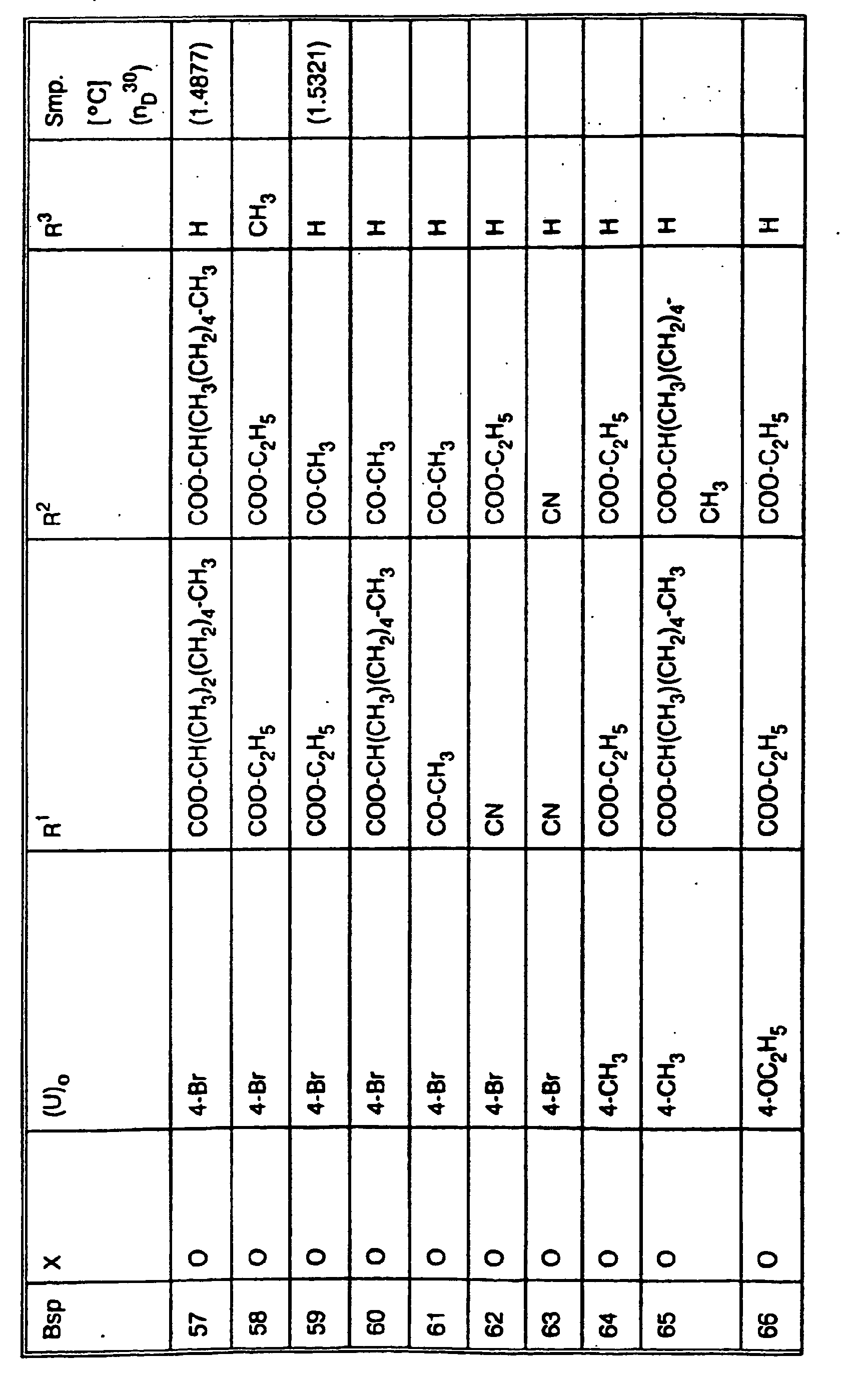

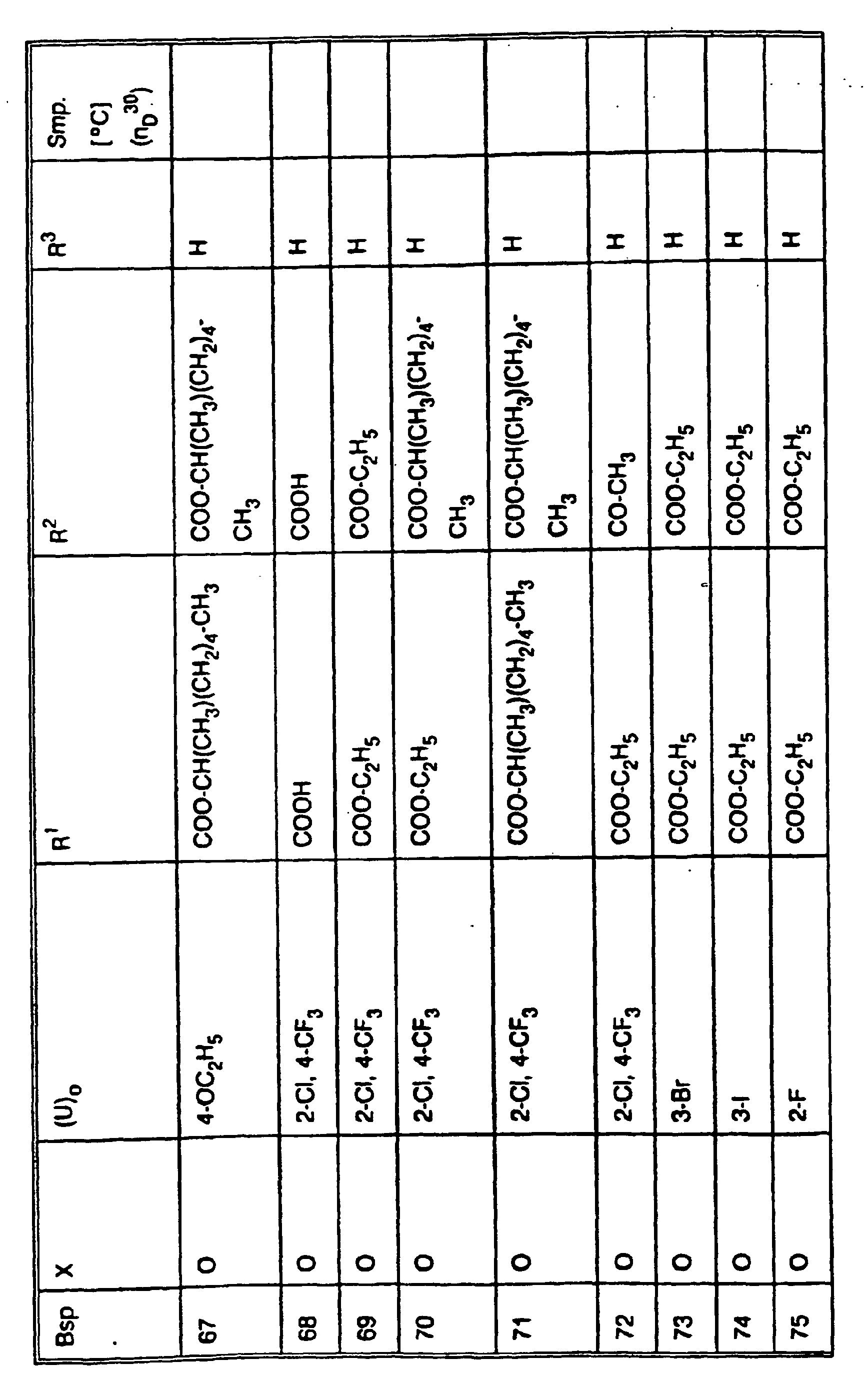

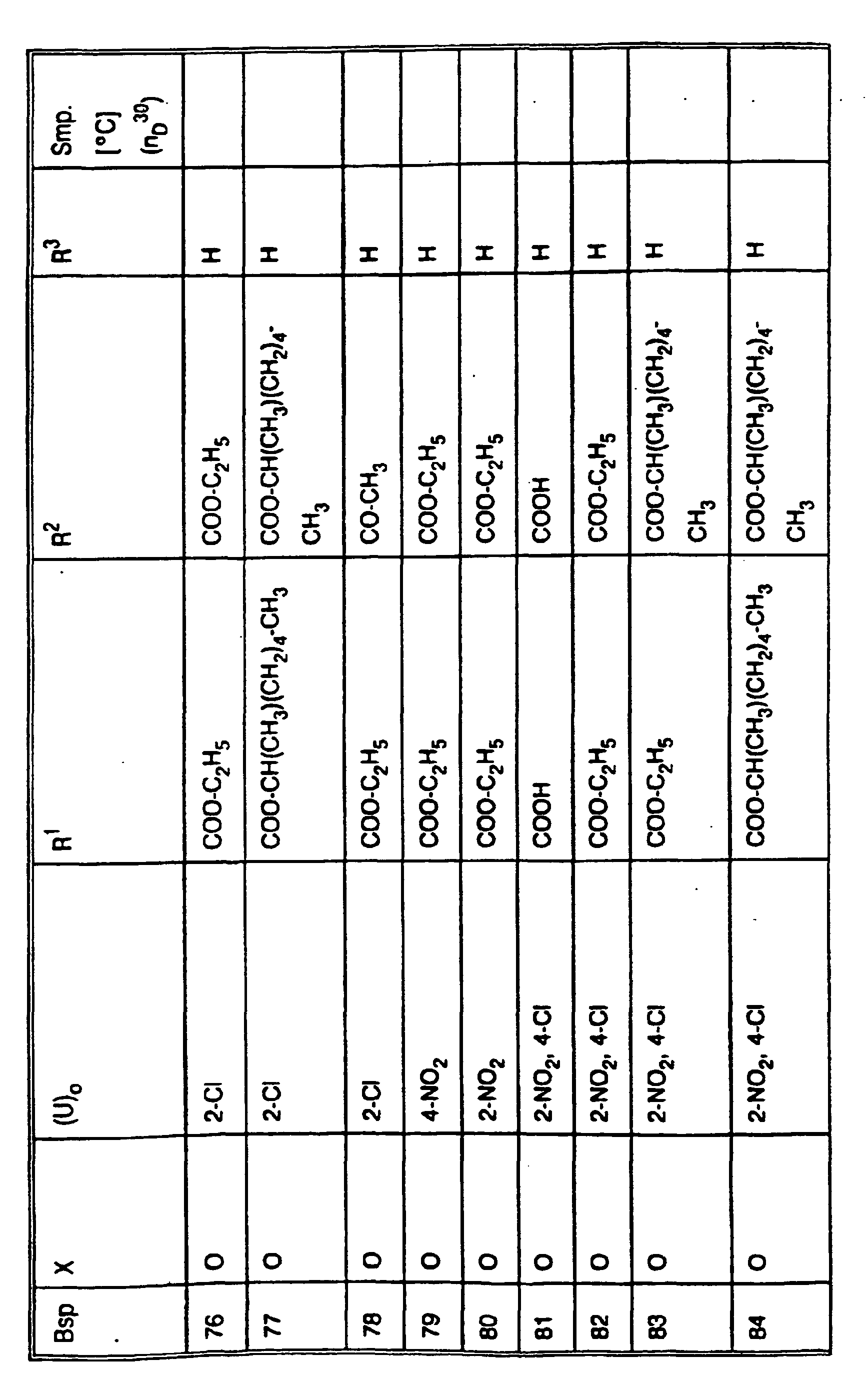

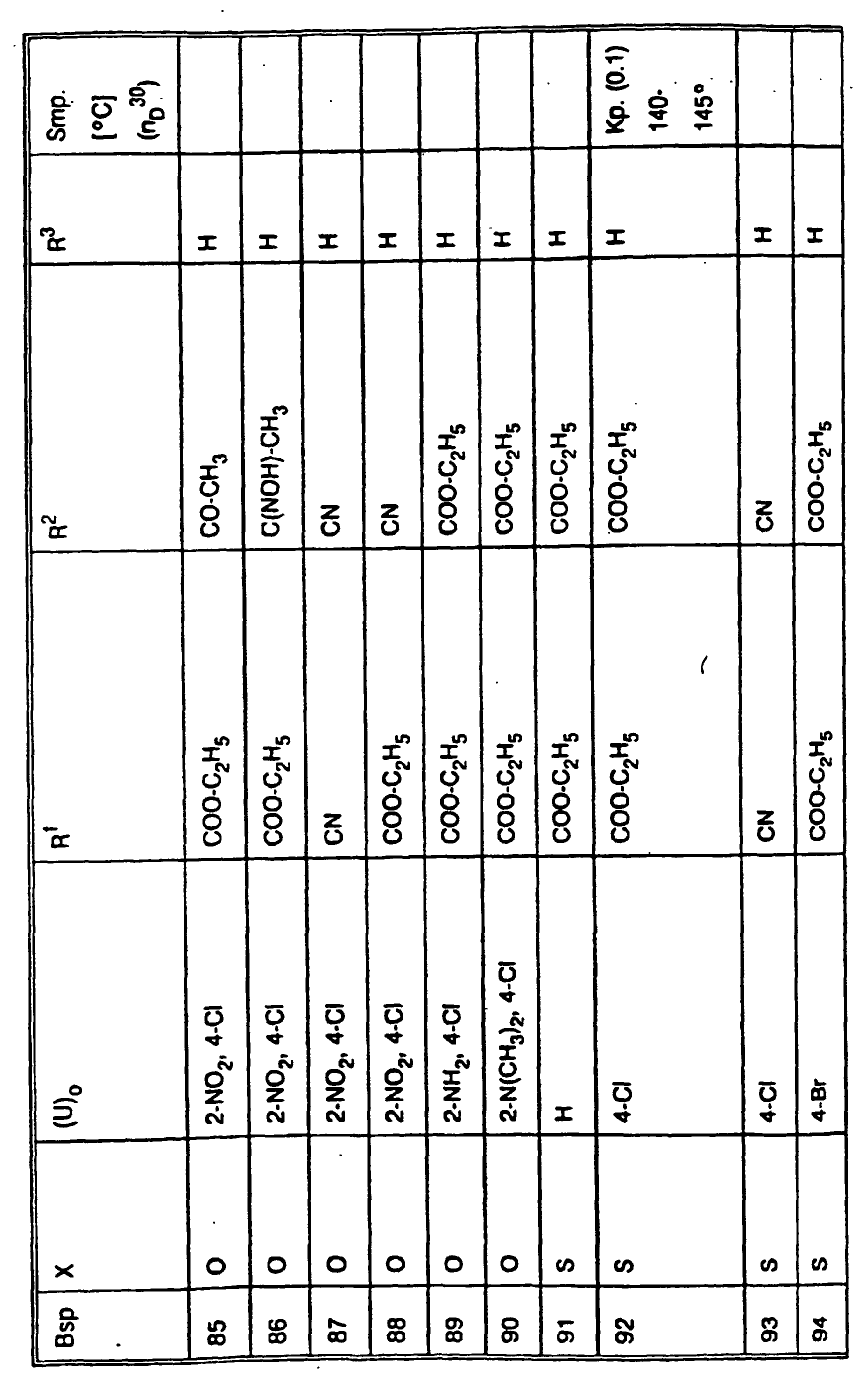

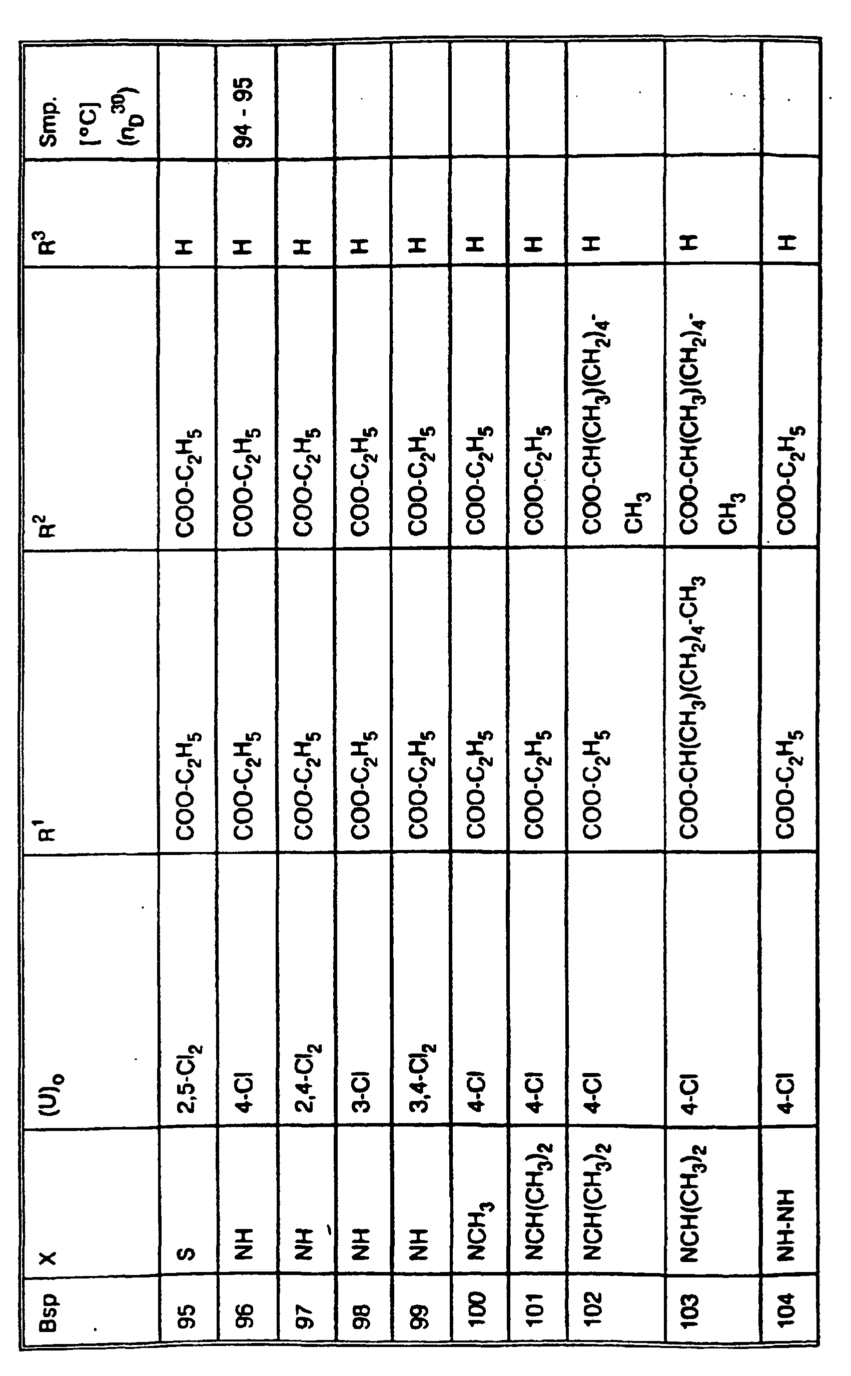

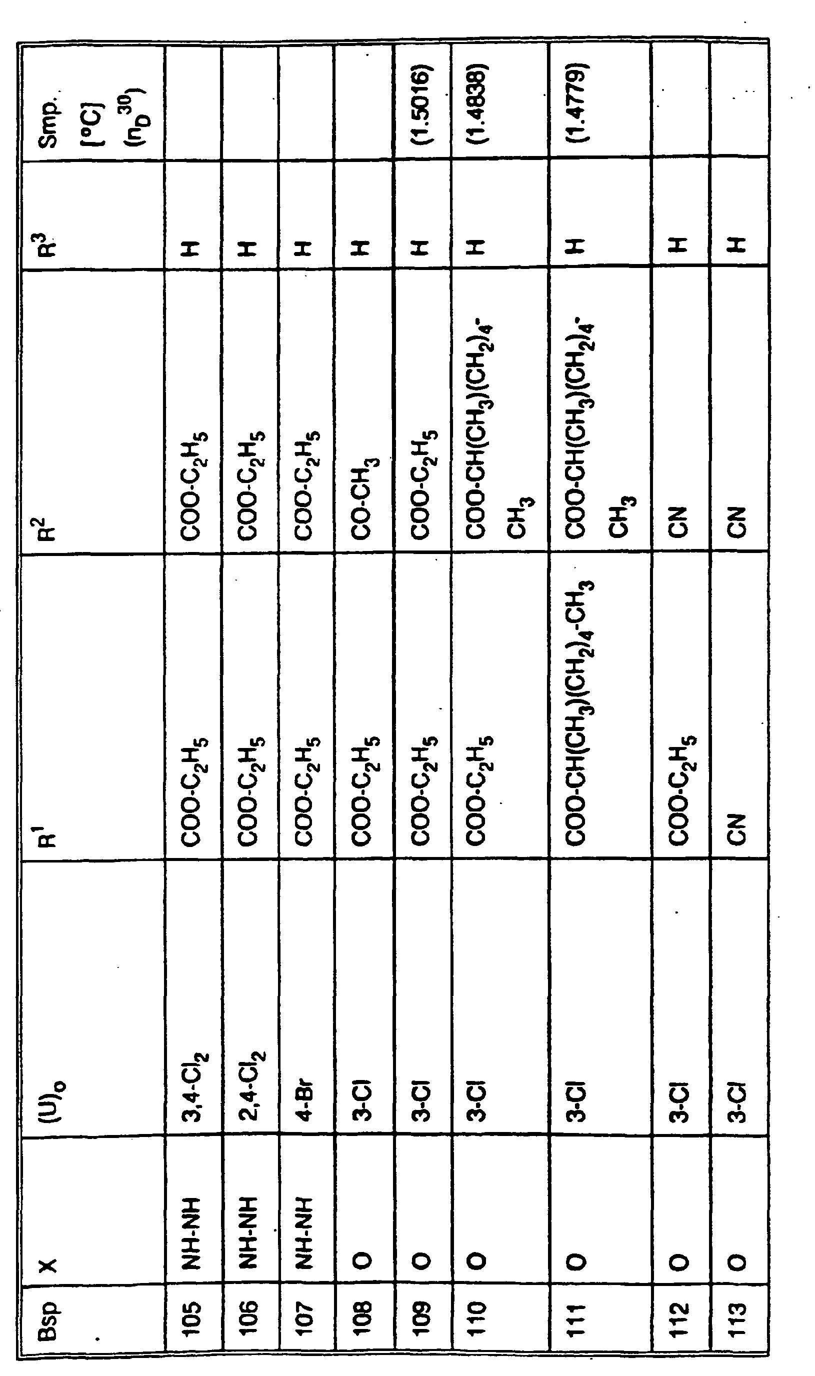

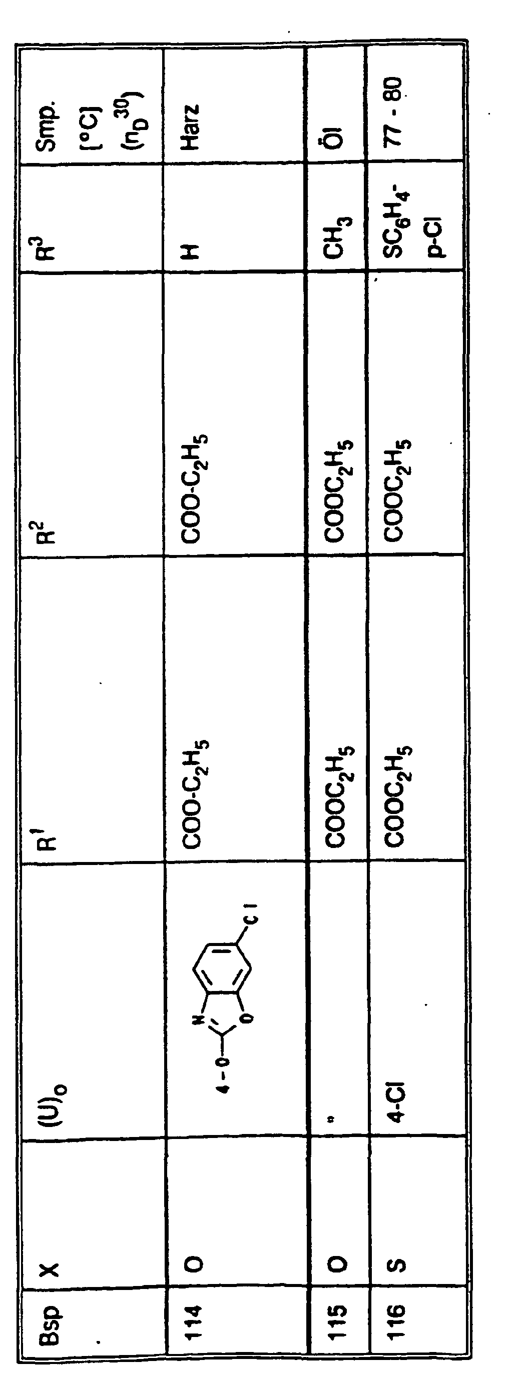

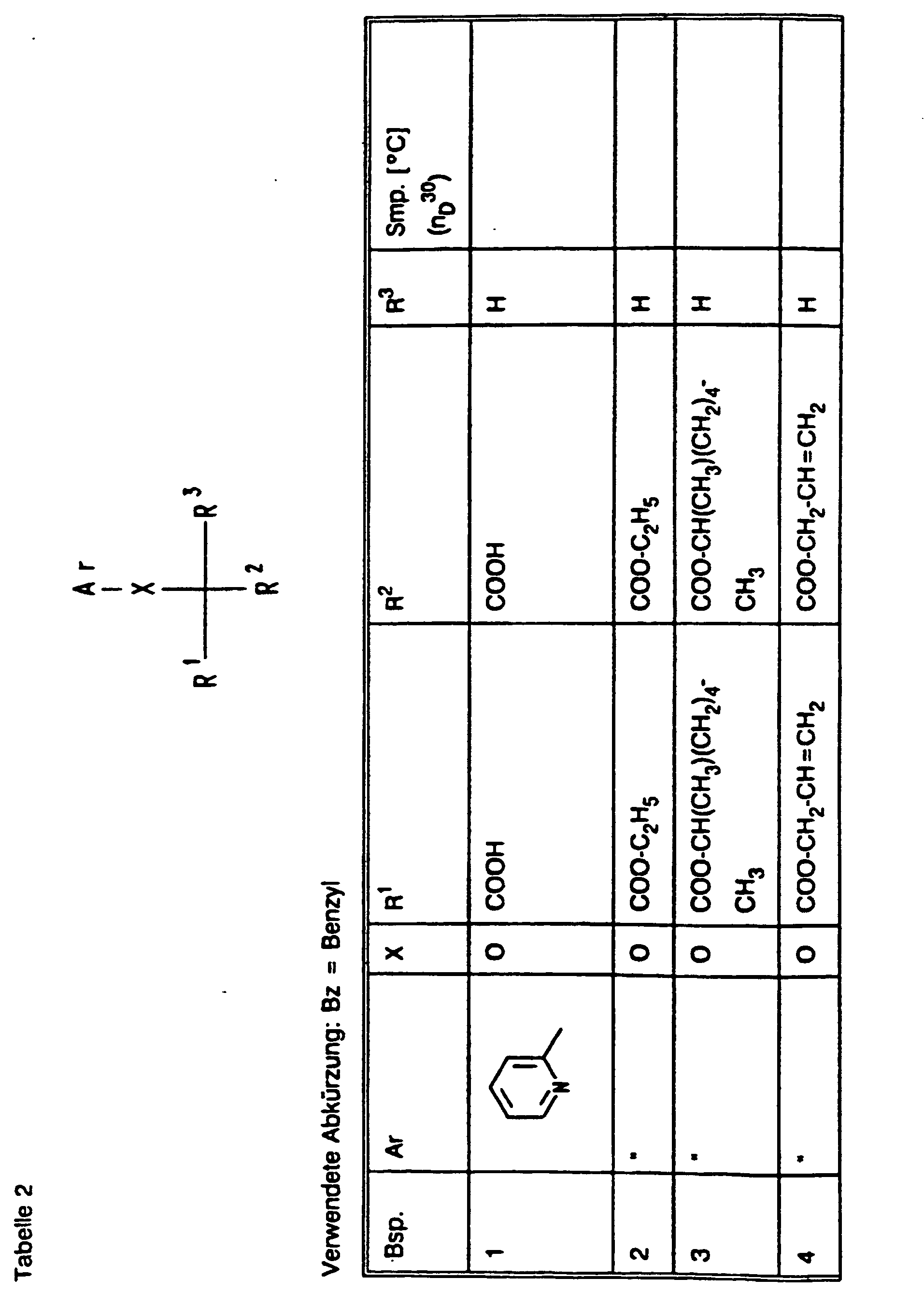

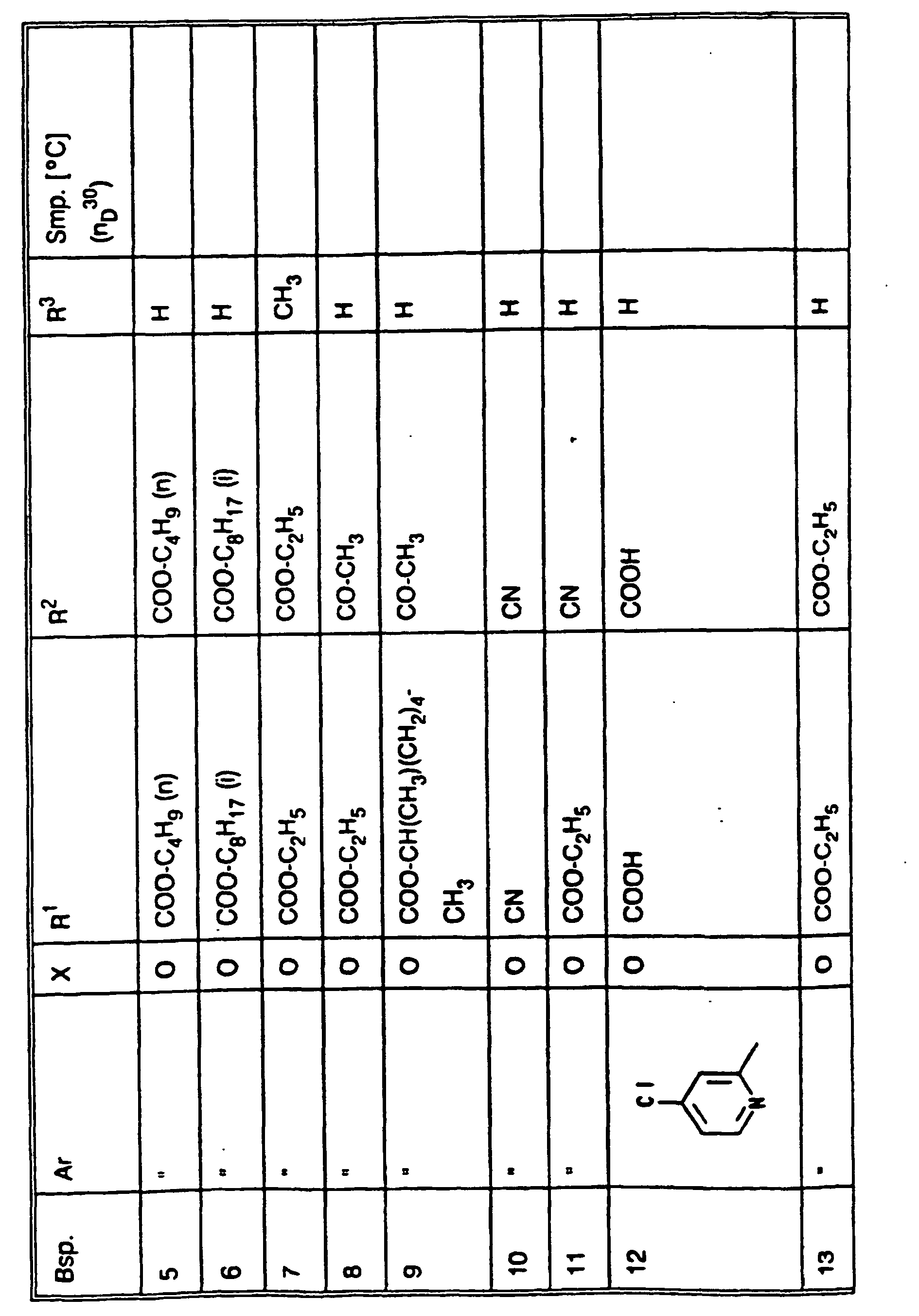

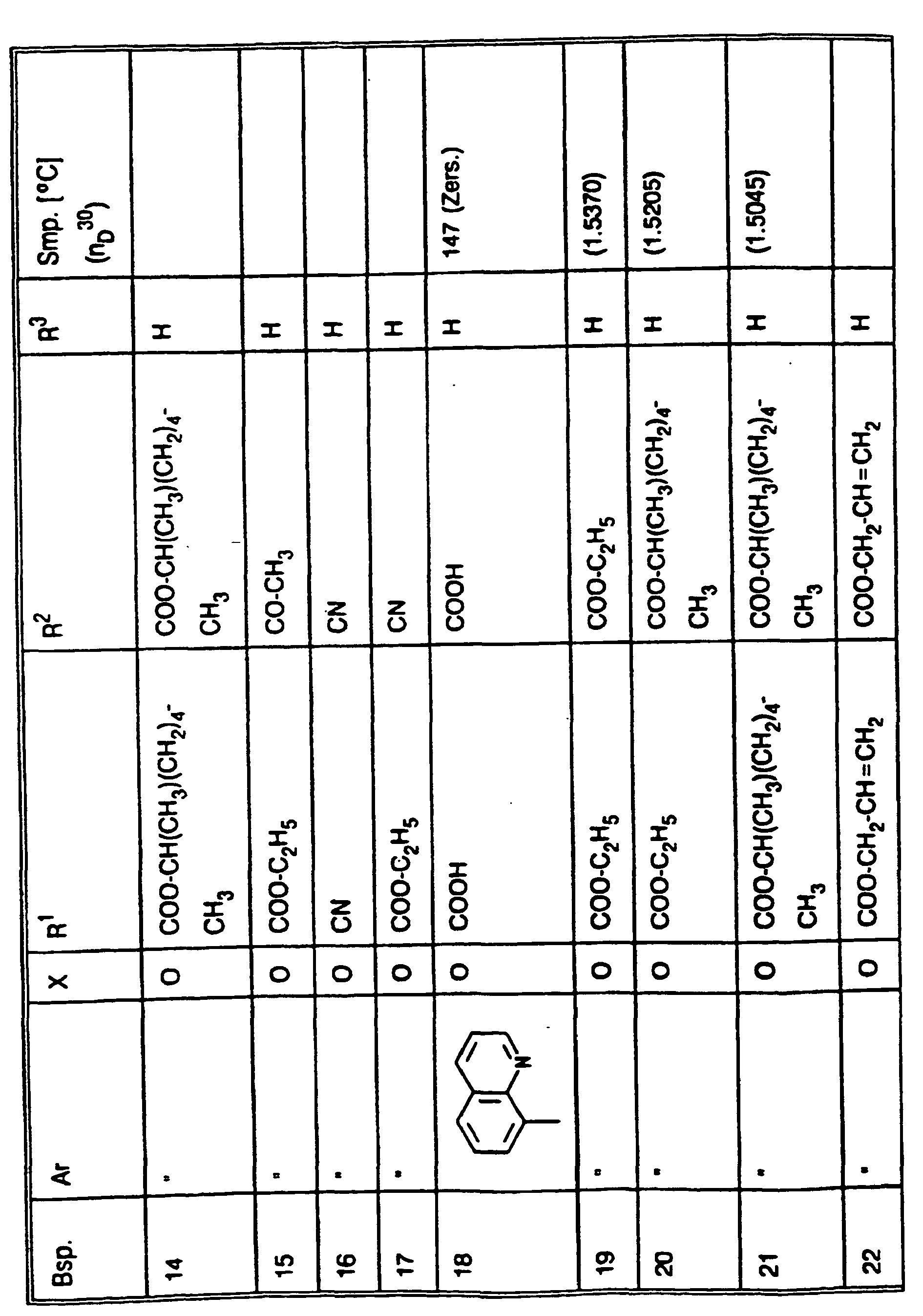

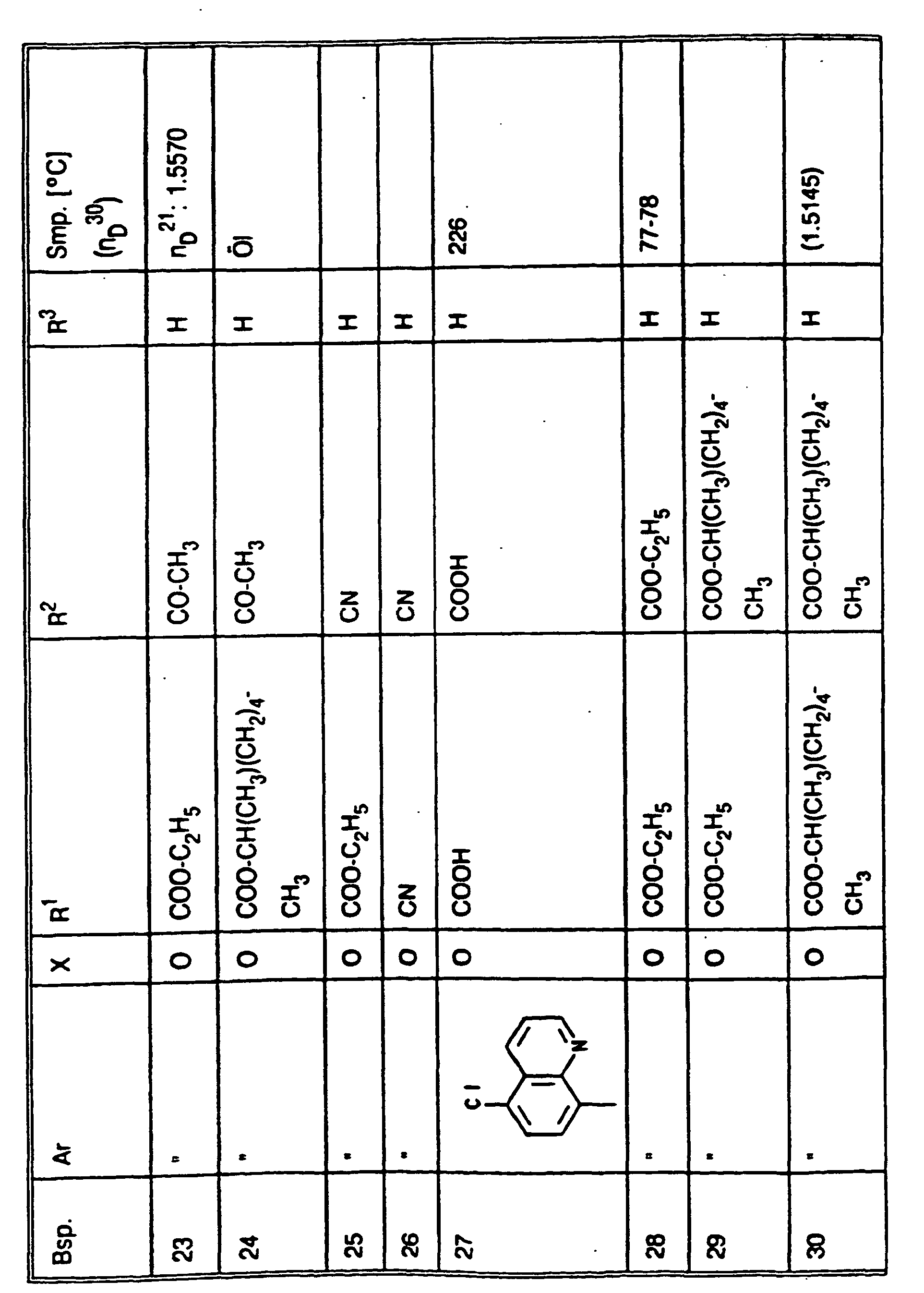

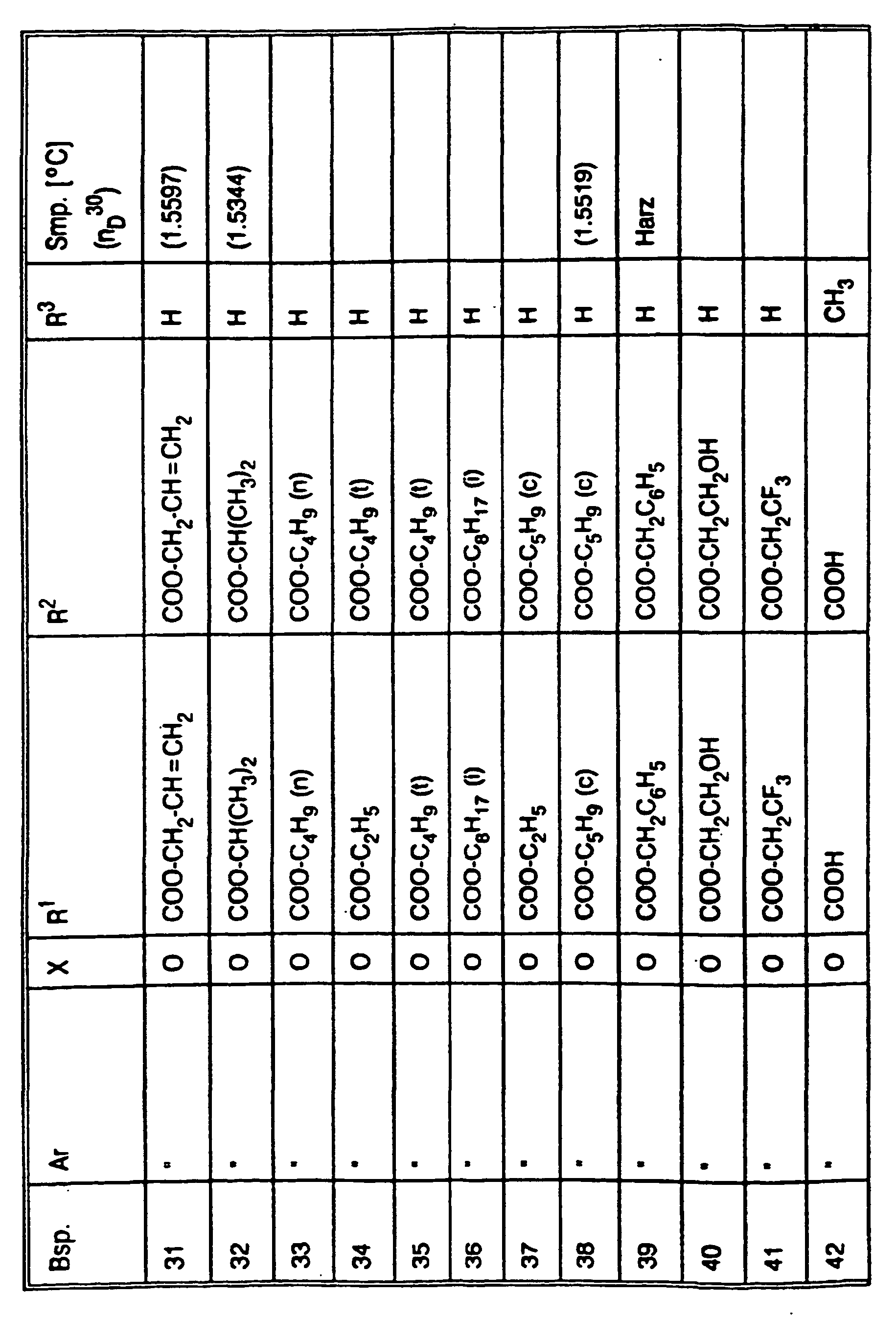

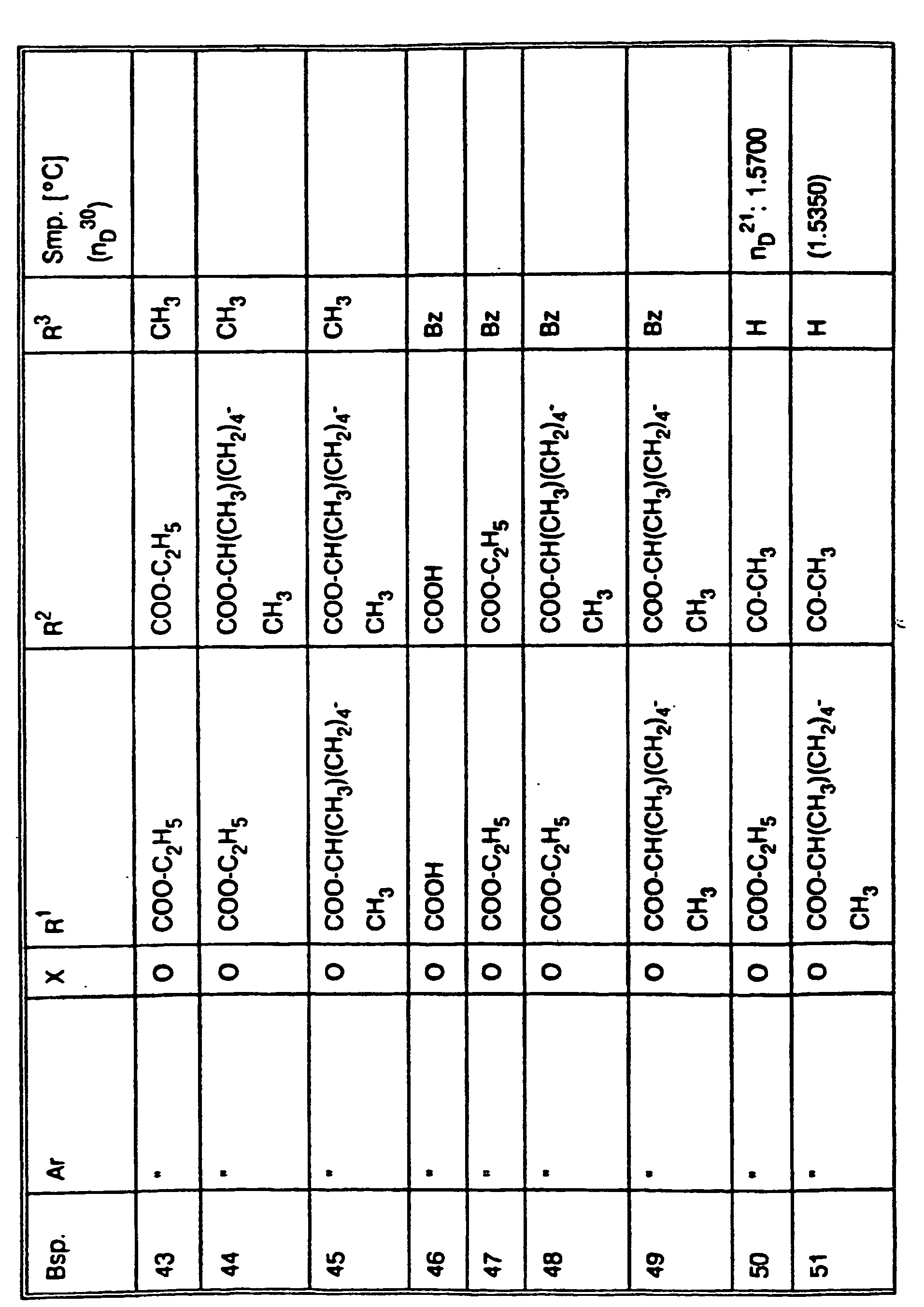

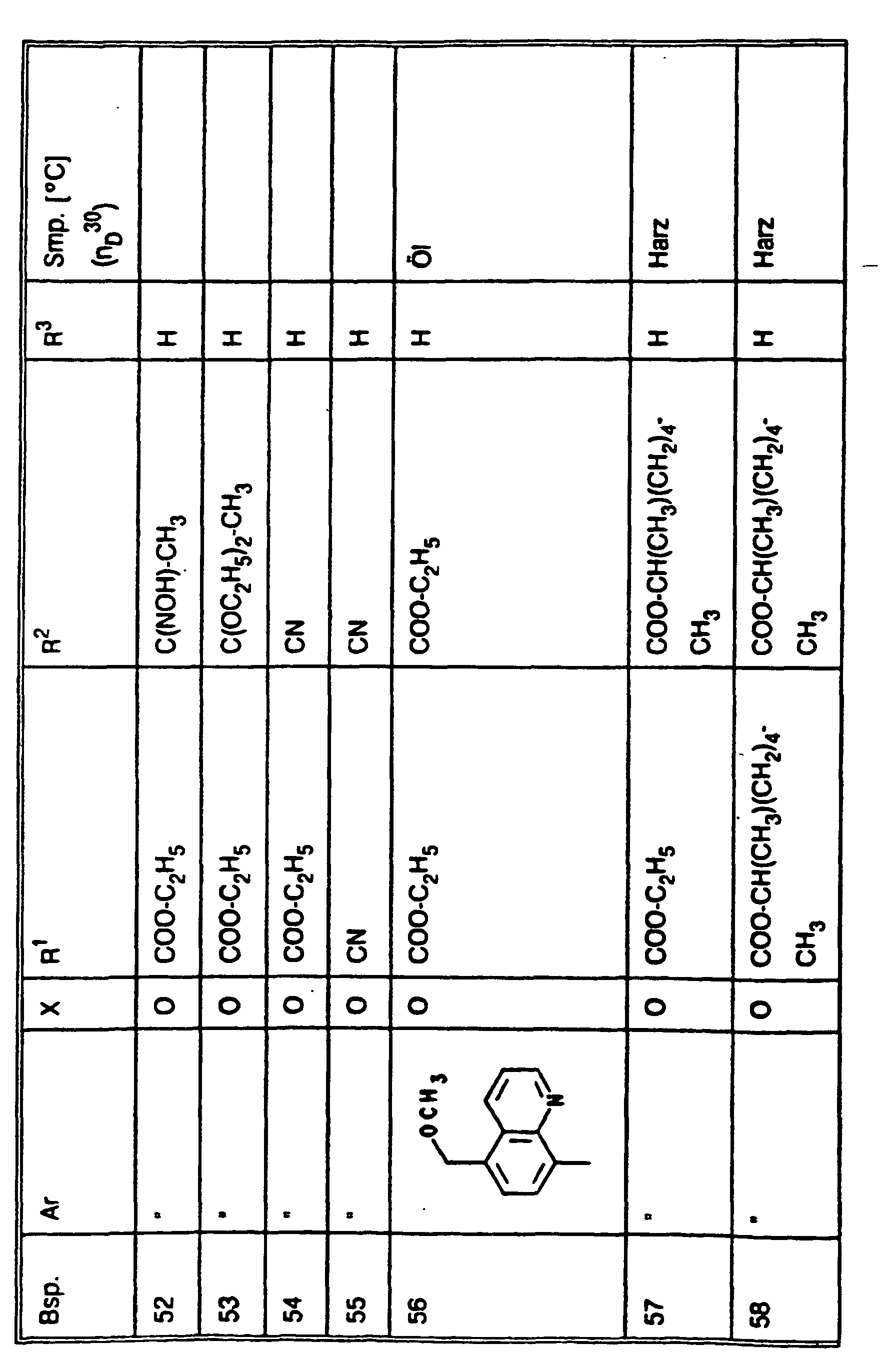

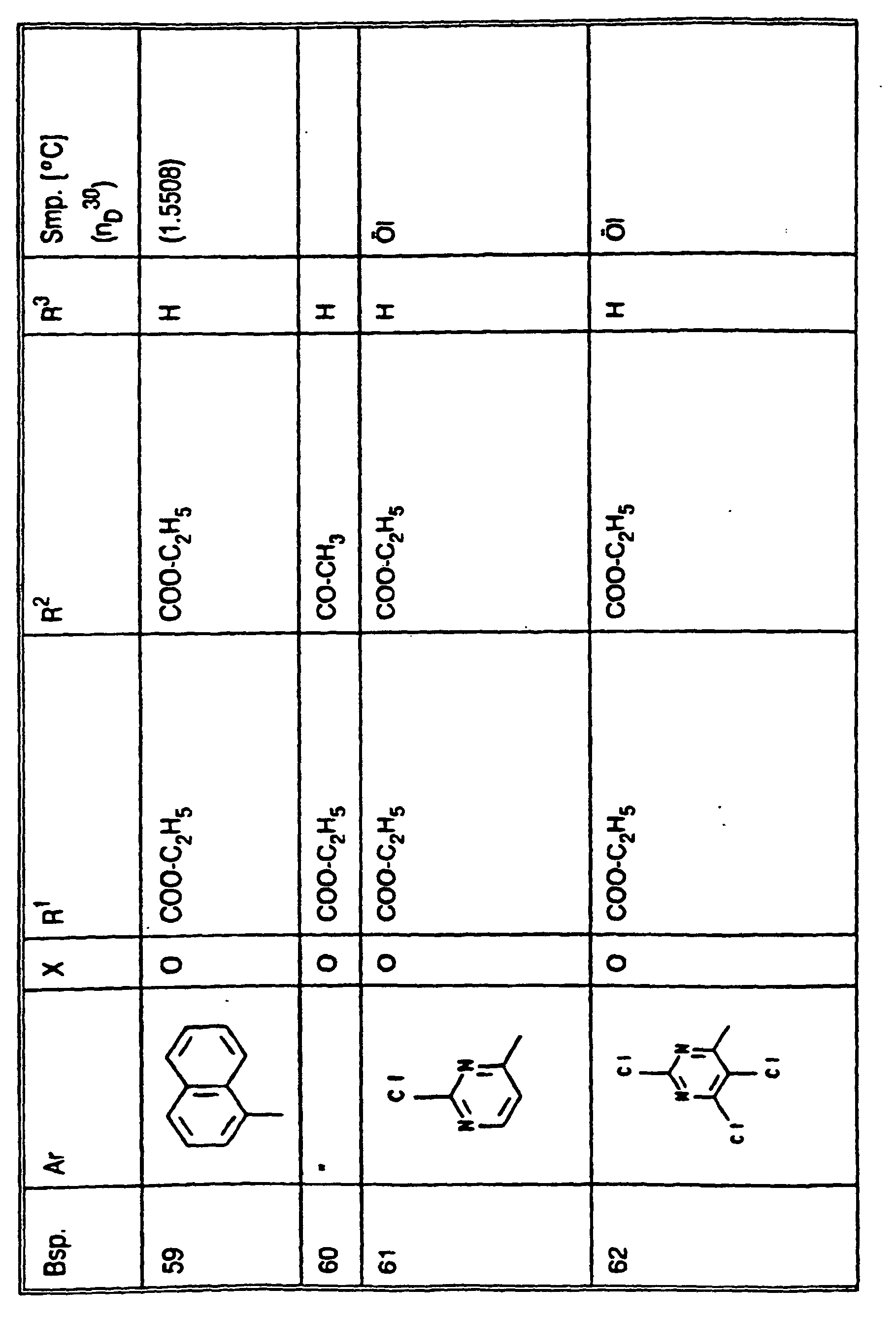

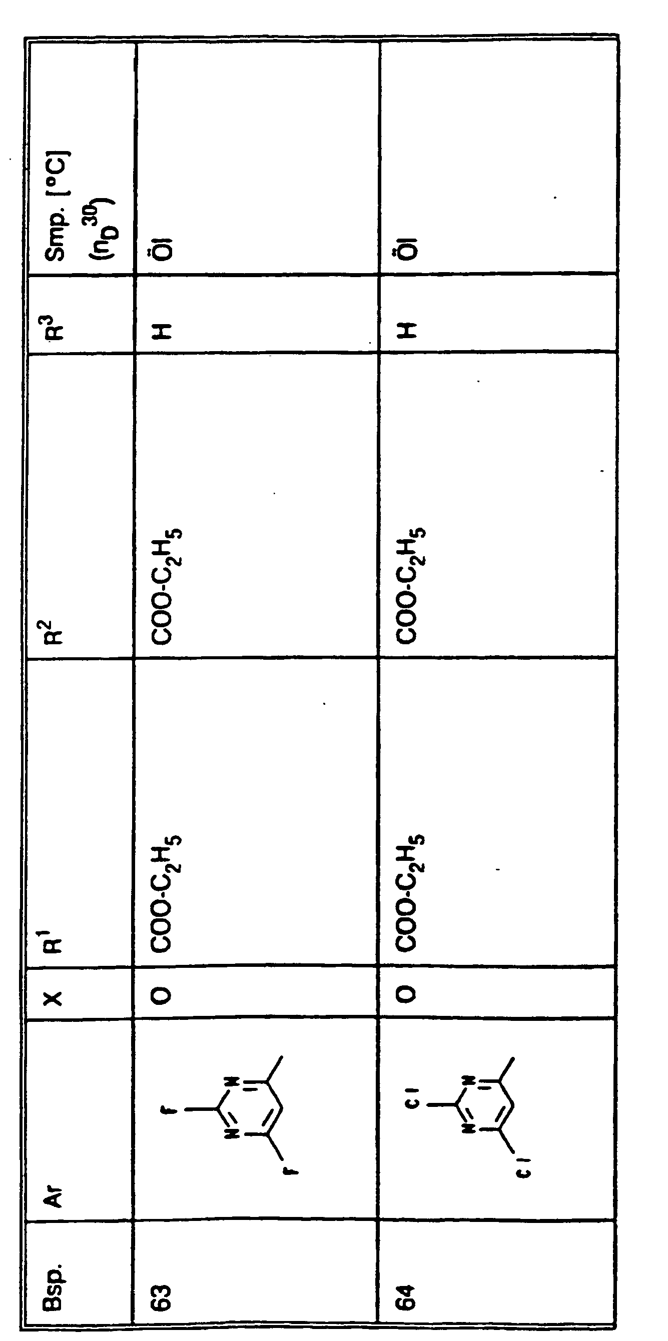

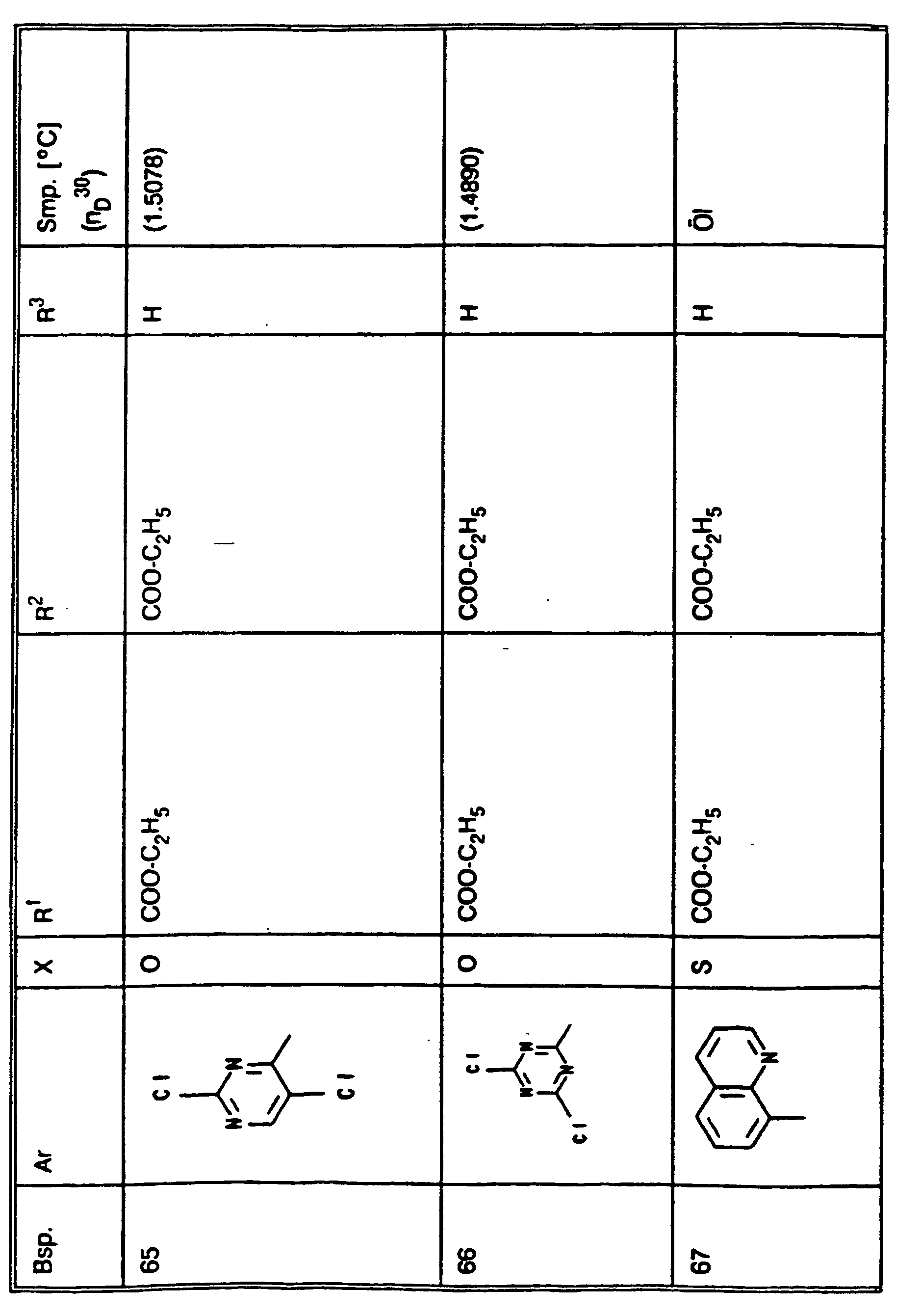

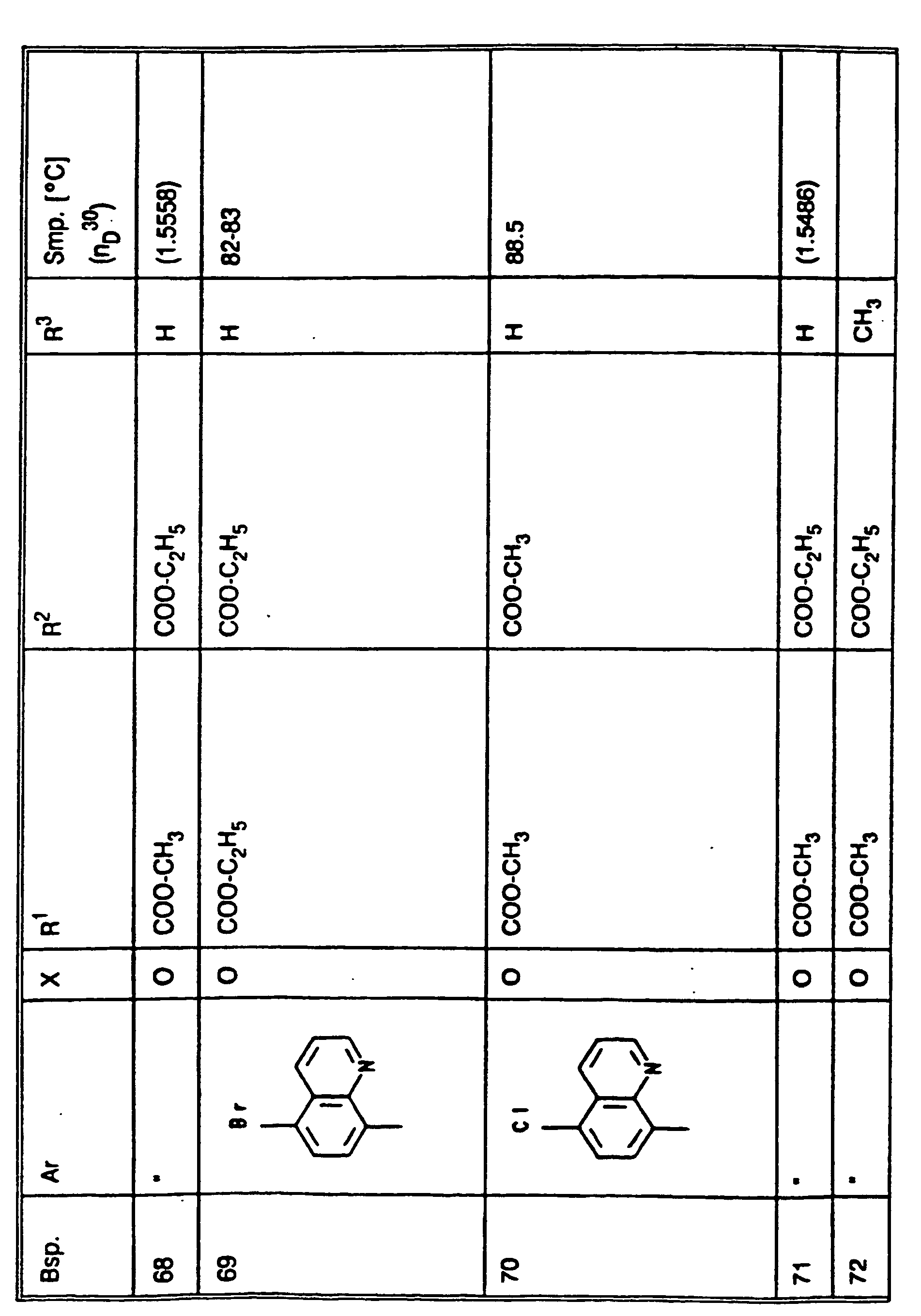

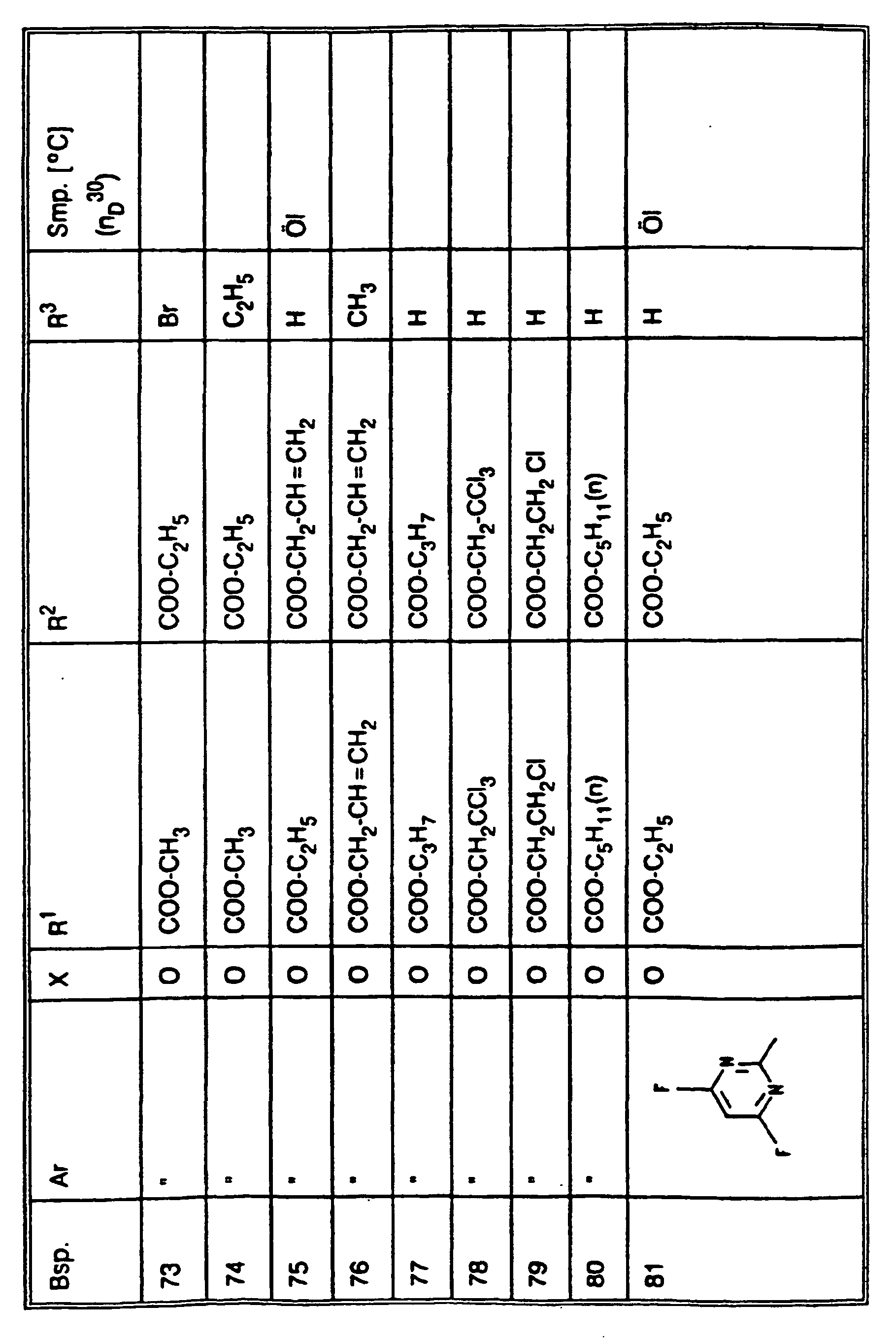

### C. Biologische Beispiele

### Beispiel 1

Samen von Weizen und Gerste wurden in sandiger Lehmerde in Plastiktöpfen ausgelegt, im Gewächshaus bis zum 3- bis 4- Blattstadium herangezogen und dann nacheinander mit den erfindungsgemäßen Verbindungen und den Herbiziden im Nachlaufverfahren behandelt. Die Herbizide und die Verbindungen der Formel I wurden dabei in Form wäßriger Suspensionen bzw. Emulsionen mit einer Wasseraufwandmenge von umgerechnet 300 l/ha ausgebracht. 3-4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wurde. Die Bewertung erfolgte in Prozentwerten im Vergleich zu unbehandelten Kontrollen. Einige Versuchsergebnisse sind in Tabellen 3 und 4 zusammengestellt.

**Tabelle 3: Safener-Wirkung an Weizen und Gerste**

| Wirkstoffe | Aufwandmenge [g a.i./ha] | | Schädigung [%] | | |
|---|---|---|---|---|---|
| | Herbizid | Safener | TA | HV | TD |
| H1 | 400 | - - | 40 | 98 | 98 |
| | 200 | - - | 30 | 90 | 95 |
| | 100 | - - | 10 | 80 | 95 |
| H1 + Bsp.28/Tab 2 | 400 | 50 | 10 | 20 | 10 |
| | 200 | 25 | 0 | 0 | 0 |
| | 100 | 12 | 0 | 0 | 0 |
| H6 + Bsp. 31/Tab 2 | 400 | 50 | 10 | 25 | 15 |
| | 200 | 25 | 0 | 15 | 0 |
| | 100 | 12 | 0 | 0 | 0 |
| H6 + Bsp. 27/Tab 2 | 400 | 100 | 15 | 25 | 20 |
| | 200 | 50 | 0 | 10 | 5 |
| | 100 | 25 | 0 | 0 | 0 |
| H2 | 1800 | - - | - - | 40 | - - |
| | 900 | - - | - - | 10 | - - |
| H2 + Bsp. 71 /Tab 2 | 1800 | 225 | - - | 0 | - - |
| | 900 | 112 | - - | 0 | - - |
| H3 | 50 | - - | 70 | 60 | - - |
| | 25 | - - | 80 | 30 | - - |
| | 12 | - - | 15 | 20 | - - |
| H3 + | 50 | 25 | 20 | 10 | - - |
| Bsp 28/Tab 2 | 25 | 12 | 10 | 5 | - - |
| | 12 | 6 | 0 | 0 | - - |
| H3 + Bsp 71/Tab 2 | 50 | 25 | 15 | 10 | - - |
| | 25 | 12 | 0 | 0 | - - |
| | 12 | 6 | 0 | 0 | - - |
| H3 + Bsp 75/Tab 2 | 50 | 25 | 25 | 20 | - - |
| | 25 | 12 | 5 | 5 | - - |
| | 12 | 6 | 0 | 0 | - - |
| H3 + Bsp 31/Tab 2 | 50 | 25 | 25 | 20 | - - |
| | 25 | 12 | 15 | 10 | - - |
| | 12 | 6 | 0 | 0 | - - |

| | | | | | |
|---|---|---|---|---|---|
| Zu Tabelle 3: Testbedingungen: Applikation im 4-Blattstadium; Bonitur nach 4 Wochen; 4 Wiederholungen | | | | | |

| Abkürzungen: | |
|---|---|
| H1 = | Fenoxaprop-P-ethyl |
| H2 = | Diclofop-methyl |
| H3 = | 4-Jod-2-[3-(4-methoxy-6-methyl-1,3,5-triazin-2-yl)-ureidosulfonyl]-benzoesäuremethylester, Natriumsalz |
| HV = | Hordeum vulgare (Gerste) |
| TA = | Triticum aestivum (Weizen) |
| TD = | Triticum durum (Hartweizen) |
| Bsp Nr./Tab Nr. = | Safener Nr. aus Tabelle Nr. |
| - - = | nicht appliziert (bei Safener) bzw. nicht getestet (bei Pflanzenkultur) |

**Tabelle 4: Safener-Wirkung an Gerste**

| Wirkstoff(e) | Aufwandmenge [g a.i./ha] | | HV |
|---|---|---|---|
| | Herbizid | Safener | |
| H1 | 200 | | 85 |
| H 1 + Bsp. 27/Tab 2 | 200 | 1250 | 50 |
| H1 + Bsp. 30/Tab 2 | 200 | 1250 | 65 |
| H1 + Bsp. 50/Tab 2 | 200 | 1250 | 30 |
| H1 + Bsp. 64/Tab 2 | 200 | 1250 | 35 |
| H1 + Bsp. 70/Tab 2 | 200 | 1250 | 30 |
| H1 + Bsp. 32/Tab 2 | 200 | 1250 | 50 |
| H1 + Bsp. 75/Tab 2 | 200 | 1250 | 33 |
| H1 + Bsp. 39/Tab 2 | 200 | 1250 | 25 |
| H1 + Bsp. 19/Tab 2 | 200 | 1250 | 60 |
| H1 + Bsp. 51/Tab 2 | 200 | 1250 | 50 |

| | | | |
|---|---|---|---|
| Testbedingungen: Applikation im Dreiblattstadium; Bonitur nach 2 - 3 Wochen; 4 Wiederholungen | | | |
| Abkürzungen: siehe Abkürzungen zu Tabelle 3 | | | |

Selbst bei starken Überdosierungen des Herbizids werden bei den Kulturpflanzen auftretende schwere Schädigungen deutlich reduziert, geringere Schäden völlig aufgehoben.

Mischungen aus Herbiziden und erfindungsgemäßen Verbindungen eignen sich deshalb in ausgezeichneter Weise zur selektiven Unkrautbekämpfung in Getreidekulturen.

### Beispiel 2

Maispflanzen, Unkräuter und Ungräser wurden im Freiland oder im Gewächshaus in Plastiktöpfen bis zum 4- bis 5-Blattstadium herangezogen und nacheinander mit Herbiziden und erfindungsgemäßen Verbindungen der Formel I im Nachauflaufverfahren behandelt. Die Wirkstoffe wurden dabei in Form wäßriger Suspensionen bzw. Emulsionen mit einer Wasseraufwandmenge von umgerechnet 300 l/ha ausgebracht. 4 Wochen nach der Behandlung wurden die Pflanzen visuell auf jede Art von Schädigung durch die ausgebrachten Herbizide bonitiert, wobei insbesondere das Ausmaß der anhaltenden Wachstumshemmung berücksichtigt wird. Die Bewertung erfolgte in Prozentwerten im Vergleich zu unbehandelten Kontrollen.
Einige Ergebnisse sind in Tabellen 5 bis 7 zusammengestellt.

**Tabelle 5: Safener-Wirkung an Mais (Zea mays)**

| Wirkstoff(e) | Aufwandmenge [g a.i./ha] | | Schädigung Mais [%] | |
|---|---|---|---|---|
| | Herbizid | Safener | Sorte Alois | Sorte Felix |
| H4 | 300 | - - | 60 | 60 |
| | 150 | - - | 55 | 50 |
| | 75 | - - | 40 | 30 |
| | 38 | - - | 20 | 0 |
| H4 + Bsp 28/Tab 2 | 300 | 150 | 30 | 25 |
| | 150 | 75 | 10 | 15 |
| | 75 | 38 | 0 | 0 |
| | 38 | 19 | 0 | 0 |
| H4 + Bsp 31 /Tab 2 | 300 | 150 | 40 | 30 |
| | 150 | 75 | 15 | 10 |
| | 75 | 38 | 0 | 0 |
| | 38 | 19 | 0 | 0 |
| H5 | 200 | - - | 50 | 45 |
| | 100 | - - | 40 | 35 |
| | 50 | - - | 30 | 25 |
| H5 + Bsp. 28/Tab 2 | 200 | 100 | 20 | 15 |
| | 100 | 50 | 10 | 5 |
| | 50 | 25 | 0 | 0 |
| H5 + Bsp. 71/Tab 2 | 200 | 100 | 20 | 20 |
| | 100 | 50 | 5 | 10 |
| | 50 | 25 | 0 | 0 |

| | | | | |
|---|---|---|---|---|
| Testbedingungen: Applikation im 4-Blattstadium; Bonitur nach 4 Wochen; 4 Wiederholungen | | | | |
| Abkürzungen: siehe Tabelle 3 sowie H4 = 3-(4,6-Dimethoxypyrimidin-2-yl-oxy-pyridin)-2-carbonsäurebenzylester H5 = 5-Ethyl-2-(4-isopropyl-4-methyl-5-oxo-2-imidazolin-2-yl)-pyridin-3-carbonsäure (Imazethapyr) | | | | |

**Tabelle 6: Safener-Wirkung bei Mais (Zea mays)**

| Wirkstoff(e) | Aufwandmenge [g a.i./ha] | | Schädigung [%] bei Maissorten | | |
|---|---|---|---|---|---|
| | Herbizid | Safener | Mutin | Felix | Dea |
| H6 | 80 | - - | 40 | 5 | - - |
| | 40 | - - | 20 | 5 | - - |
| | 20 | - - | 5 | 10 | - - |
| H6 + Bsp. 71/Tab 2 | 80 | 40 | 10 | 5 | - - |
| | 40 | 20 | 0 | 0 | - - |
| | 20 | 10 | 0 | 0 | - - |
| H6 + Bsp. 70/Tab 2 | 80 | 40 | 20 | 15 | - - |
| | 40 | 20 | 5 | 0 | - - |
| | 20 | 10 | 0 | 0 | - - |
| H7 | 60 | - - | 70 | 75 | - - |
| | 30 | - - | 30 | 40 | - - |
| | 15 | - - | 10 | 15 | - - |
| | 8 | - - | 5 | 0 | - - |
| H7 + Bsp. 71/Tab 2 | 60 | 30 | 20 | 25 | - - |
| | 30 | 15 | 5 | 10 | - - |
| | 15 | 7,5 | 0 | 0 | - - |
| | 8 | 4 | 0 | 0 | - - |
| H7 + Bsp. 70/Tab 2 | 60 | 30 | 25 | 25 | - - |
| | 30 | 15 | 10 | 5 | - - |
| | 15 | 7,5 | 0 | 0 | - - |
| | 8 | 4 | 0 | 0 | - - |
| H8 | 200 | - - | 65 | 70 | 35 |
| | 100 | - - | 60 | 65 | 10 |
| | 50 | - - | 30 | 55 | 0 |
| | 25 | - - | 15 | 25 | 0 |
| H8 + Bsp. 31/Tab 2 | 200 | 100 | 40 | 25 | 0 |
| | 100 | 50 | 20 | 10 | 0 |
| | 50 | 25 | 0 | 0 | 0 |
| | 25 | 12 | 0 | 0 | 0 |
| H8 + Bsp. 71/Tab 2 | 200 | 100 | 35 | 30 | 5 |
| | 100 | 50 | 15 | 10 | 0 |
| | 50 | 25 | 0 | 0 | 0 |
| | 25 | 12 | 0 | 0 | 0 |
| H8 + Bsp. 75/Tab 2 | 200 | 100 | 40 | 30 | 5 |
| | 100 | 50 | 15 | 10 | 0 |
| | 50 | 25 | 0 | 0 | 0 |
| | 25 | 12 | 0 | 0 | 0 |
| H8 + Bsp. 28/Tab 2 | 200 | 100 | 30 | 30 | 0 |
| | 100 | 50 | 20 | 10 | 0 |
| | 50 | 25 | 0 | 0 | 0 |
| | 25 | 12 | 0 | 0 | 0 |

| | | | | | |
|---|---|---|---|---|---|
| Testbedingungen: Applikation im 4-Blattstadium; Bonitur nach 4 Wochen; 4 Wiederholungen | | | | | |
| Abkürzungen: siehe Tabelle 3 und wie folgt: H6 = 1-(3-Ethylsulfonylpyridin-2-yl-sulfonyl)-3-(4,6-dimethoxy-pyrimidin-2-yl)-harnstoff (DPX-E 9636, Rimsulfuron) H7 = 1-(2-Methoxycarbonylthiophen-3-yl)-3-(4-methoxy-6-methyl-1,3,5-triazin-2-yl)-hamstoff (Thifensulfuron-methyl) H8 = 3-(4,6-Dimethoxypyrimidin-2-yl)-1-[3-(N-methyl-N-methylsulfonylamino)-2-pyridylsutfonyl]-hamstoff | | | | | |

**Tabelle 7: Safener-Wirkung bei Mais (zea mays)**

| Wirkstoff(e) | Aufwandmenge [g a.i./ha] | | Schädigung bei Mais [%] Sorte Felix |
|---|---|---|---|
| | Herbizid | Safeher | |
| H8 | 75 | - | 75 |
| H8 + Bsp. 50/Tab 2 | 75 | 1250 | 55 |
| H8 + Bsp. 68/Tab 2 | 75 | 1250 | 20 |
| H8 + Bsp. 70/Tab 2 | 75 | 1250 | 55 |
| H8 + Bsp. 19/Tab. 2 | 75 | 1250 | 30 |

| | | | |
|---|---|---|---|
| Testbedingungen: Applikation im Dreiblattstadium; Bonitur nach 3 Wochen; 4 Wiederholungen | | | |
| Abkürzungen: wie in Tabelle 6 | | | |

Die Ergebnisse zeigen, daß die erfindungsgemäßen eingesetzten Verbindungen der Formel I starke Herbizidschäden an den Maispflanzen effektiv reduzieren können. Selbst bei starken Überdosierungen der Herbizide werden bei den Kulturpflanzen auftretende schwere Schädigungen deutlich reduziert und geringere Schäden völlig aufgehoben. Mischungen aus Herbiziden und Verbindungen der Formel eignen sich deshalb in ausgezeichneter Weise zur selektiven Unkrautbekämpfung in Mais.

### Beispiel 3

Reis wurde in Plastiktöpfen ausgesät und im Gewächshaus unter optimalen Wachstumsbedingungen angezogen. Nach dem Auflaufen wurden die Töpfe mit Wasser bis zu 2 cm aufgefüllt und diese Anstauhöhe über die gesamte Versuchsdauer eingehalten. Im 3- bis 4-Blattstadium wurden die Pflanzen dann mit den Herbiziden und den Verbindungen der Formel I behandelt. 3 Wochen nach der Behandlung wurden die Pflanzen auf jede Art von Schädigung durch die Herbizide bonitiert, wobei insbesondere das Ausmaß der anhaltenden Wachstumshemmung und Ausdünnung betrachtet wurde. Die Ergebnisse der Bonituren zeigen, daß die Safener die Herbizidschäden an Reis effektiv reduzieren.
Einige Ergebnisse sind in Tabelle 8 aufgeführt.

**Tabelle 8: Safener-Wirkung bei Reis**

| Wirkstoff(e) | Aufwandmenge [g a.i./ha] | | Schädigung [%] ORSA |
|---|---|---|---|
| | Herbizid | Safener | |
| H1 | 300 | -- | 80 |
| H1 + Bsp. 28/Tab 2 | 300 | 1250 | 35 |
| H1 + Bsp. 27/Tab 2 | 300 | 1250 | 70 |
| H1 + Bsp. 30/Tab 2 | 300 | 1250 | 45 |
| H1 + Bsp. 50/Tab 2 | 300 | 1250 | 70 |
| H1 + Bsp. 64/Tab 2 | 300 | 1250 | 70 |
| H1 + Bap. 70/Tab 2 | 300 | 1250 | 70 |
| H1 + Bsp. 32/Tab 2 | 300 | 1250 | 35 |
| H1 + Bsp. 75/Tab 2 | 300 | 1250 | 30 |
| H1 + Bsp. 31/Tab 2 | 300 | 1250 | 50 |
| H1 + Bsp. 39/Tab 2 | 300 | 1250 | 70 |
| H1 + Bsp. 19/Tab 2 | 300 | 1250 | 45 |
| H1 + Bsp. 51/Tab 2 | 300 | 1250 | 70 |
| H1 + Bsp. 71 /Tab 2 | 300 | 1250 | 40 |

| | | | |
|---|---|---|---|
| Testbedingungen: Applikation im Dreiblattstadium; Bonitur nach 3 Wochen; 4 Wiederholungen | | | |
| Abkürzungen: siehe Tabelle 3 und | | | |
| ORSA = Oryza sativa (Reis) | | | |

Mischungen aus Herbiziden und den erfindungsgemäßen Safenern eignen sich also zur selektiven Unkrautbekämpfung in Reis. Die herbizide Wirksamkeit der eingesetzten Herbizide gegen Schadpflanzen wird durch die Zugabe der erfindungsgemäßen Safener nicht beeinträchtigt; sie entspricht bei den verwendeten Aufwandmengen den Vergleichswerten, wie sie bei der Anwendung der Herbizide alleine erzielt werden.

### Beispiel 4

Reis wurde im Gewächshaus in Töpfen in Sandboden ausgesät und bis zu einer Wuchshöhe von 24 - 25 cm herangezogen. Dann wurde der Reis in einen mit Wasser bedeckten Boden verpflanzt und 3 Tage nach dem Verpflanzen mit einem Herbizid oder einer Herbizid/Safener-Kombination mittels Gießapplikaton behandelt. Vier Wochen nach der Applikation wurden Pflanzenschäden im Vergleich zu unbehandelten Kontrollen optisch bonitiert (Ergebnisse siehe Tabelle 9).

**Tabelle 9: Safener-Wirkung bei verpflanztem Reis**

| Wirkstoff(e) | Aufwandmenge [g a.i./ha] | | ORSA-T |
|---|---|---|---|
| | Herbizid | Safener | |
| H9 | 450 | - | 50 |
| H9 + Bsp. 28/Tab 2 | 450 | 225 | 33 |
| | 450 | 450 | 33 |

| | | | |
|---|---|---|---|
| Abkürzungen wie in Tabelle 3 und wie folgt | | | |
| : ORSA-T = Oryza sativa (verpflanzt) | | | |
| H9 = Anilofos | | | |

Das Beispiel von Tabelle 4 veranschaulicht die Safener-Wirkung der Verbindungen der Formel (I) bei einem Herbizid, das im Vergleich zu den Herbiziden H1 bis H8 strukturell ganz andersartig ist.

## Patentansprüche

1. Verwendung von Verbindungen der Formel I deren Salze, 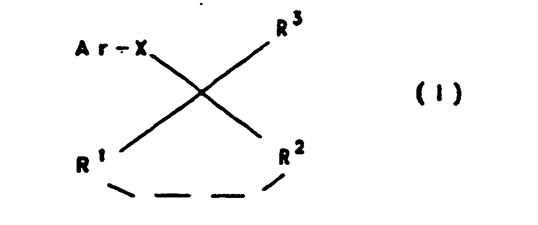 worin
R¹ und R² unabhängig voneinander Reste der Formel 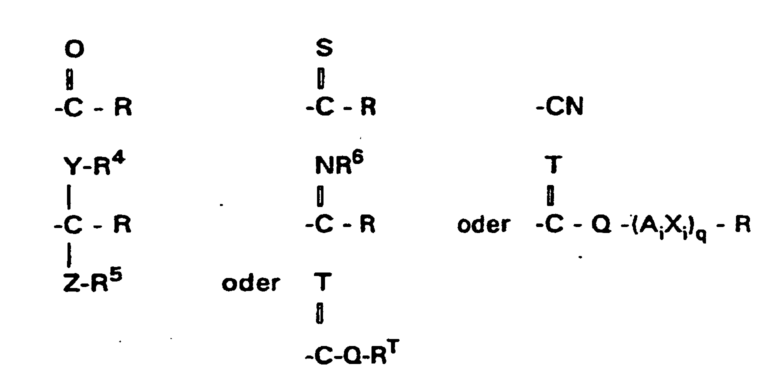 worin R, R^{T}, R⁴, R⁵, R⁶, Y, T, Z, Q, Aᵢ, Xᵢ, q wie weiter unten definiert sind, oder
R¹ und R² miteinander verbunden sind und gemeinsam eine Gruppe der Formel
-CO-Q¹-D-Q²-CO-
worin
Q¹, Q² unabhängig voneinander wie Q definiert sind und
D eine divalente Gruppe der Formel CR'R" oder C=O, wobei R' und R" unabhängig voneinander Wasserstoff oder C₁-C₄-Alkyl bedeuten,
R³ Wasserstoff, Halogen, C₁-C₁₈-Alkyl, C₂-C₈-Alkenyl, C₂-C₈-Alkinyl, C₁-C₁₈-Alkoxy, C₂-C₈-Alkenyloxy. C₂-C₈-Alkinyloxy, C₁-C₁₈-Alkylthio, C₂-C₈-Alkenylthio, C₂-C₈-Alkinylthio, wobei jeder der letztgenannten 9 Reste jeweils unsubstituiert oder durch einen oder mehrere Reste aus der Gruppe Halogen, Nitro und Cyano substituiert ist, oder C₃-C₁₂-Cycloalkyl, das unsubstituiert oder durch einen oder mehrere Reste aus der Gruppe C₁-C₄-Alkyl, Halogen, Nitro und Cyano substituiert ist, oder SiR^{a}R^{b}R^{c}, worin R^{a}, R^{b} und R^{c} unabhängig voneinander C₁-C₄-Alkyl, C₂-C₄-Alkenyl, C₂-C₄-Alkinyl oder unsubstituiertes oder substituiertes Phenyl bedeuten, oder einen Rest der Formel Ar'X'-, worin Ar' und X' analog Ar bzw. X definiert sind,
X O, S, NH-NH oder NR^{d}, wobei R^{d} analog R⁴ definiert ist, oder -CH₂O-, -CH₂S-, -CH(Ar)O- oder -CH(Ar)S-
Ar einen aromatischen Rest,
R Wasserstoff oder einen aliphatischen, aromatischen, heteroaromatischen, araliphatischen oder heteroaraliphatischen Rest mit 1 bis 30 C-Atomen und mit gegebenenfalls einer oder mehreren funktionellen Gruppen,
R^{T} einen Rest der Formel -CO-R, -CS-R, -NR^{f}R^{g}, -N=CR^{h}Rⁱ oder SiR^{a}R^{b}R^{c},
wobei R die genannte Bedeutung hat und R^{f}, R^{g}, R^{h} und Rⁱ unabhängig voneinander Wasserstoff, C₁-C₄-Alkyl, C₂-C₄-Alkenyl, C₂-C₄-Alkinyl, Benzyl, Phenyl oder substituiertes Phenyl sind oder R^{f} und R^{g} gemeinsam mit dem N-Atom einen 5- oder 6-gliedrigen Heterocyclus, der noch bis zu 2 weitere Heteroatome aus der Gruppe N, O und S enthalten und durch C₁-C₄-Alkyl substituiert sein kann, bedeuten und
R^{a}, R^{b} und R^{c}unabhängig voneinander C₁-C₄-Alkyl, C₂-C₄-Alkenyl, C₂-C₄-alkinyl, Phenyl oder substituiertes Phenyl sind,
Y und Z unabhängig voneinander Sauerstoff, Schwefel in seinen verschiedenen Oxidationstufen, oder -NR^{e}, worin R^{e} analog R⁴ definiert ist,
R⁴ und R⁵ gleich oder verschieden sind und unabhängig voneinander Wasserstoff, C₁-C₆-Alkyl, C₂-C₆-Alkenyl, C₂-C₆-Alkinyl, (C₁-C₄-Alkyl)-carbonyl, wobei jeder der 4 letztgenannten Reste unsubstituiert oder durch einen oder mehrere Substituenten aus der Gruppe enthaltend Halogen, C₁-C₆-Haloalkoxy, Nitro, Cyano, Hydroxy, C₁-C₈-Alkoxy und C₁-C₈-Alkoxy, worin eine oder mehrere, vorzugsweise bis zu drei nicht direkt aneinander gebundene CH₂-Gruppen durch Sauerstoff ersetzt sind, und C₁-C₈-Alkylthio, C₁-C₆-Alkylsulfonyl, C₂-C₈-Alkenylthio, C₂-C₈-Alkinylthio, C₂-C₈-Alkenyloxy, C₂-C₈-Alkinyloxy, C₃-C₇-Cycloalkyl, C₃-C₇-Cycloalkoxy sowie Amino-, Mono- und Di-(C₁-C₄-alkyl)-amino substituiert ist, oder Formyl, SiR^{a}R^{b}R^{c}, worin R^{a}, R^{b} und R^{c} unabhängig voneinander C₁-C₄-Alkyl, C₂-C₄-Alkenyl, C₂-C₄-Alkinyl oder unsubstituiertes oder substituiertes Phenyl bedeuten, oder C₃-C₈-Cycloalkyl, C₃-C₈-Cycloalkenyl, Heterocyclyl mit 3 bis 7 Ringatomen, Aryl, Heteroaryl oder Arylcarbonyl, wobei jeder der letztgenannten 6 Reste unsubstituiert oder durch einen oder mehrere Reste aus der Gruppe C₁-C₈-Alkyl, Halogen, C₁-C₈-Hatoalkoxy, Nitro, Cyano, Hydroxy, C₁-C₈-Alkoxy und C₁-C₈-Alkoxy, worin eine oder mehrere, vorzugsweise bis zu drei nicht direkt aneinander gebundene CH₂-Gruppen durch Sauerstoff ersetzt sind, und C₁-C₈-Alkylthio, C₁-C₆-Alkylsulfonyl, C₂-C₈-Alkenylthio, C₂-C₈-Alkinylthio, C₂-C₈-Alkenyloxy, C₂-C₈-Alkinyloxy, C₃-C₇-Cycloalkyl, C₃-C₇-Cycloalkoxy sowie Amino-, Mono- und Di-(C₁-C₄-alkyl)-amino substituiert ist, oder
R⁴ und R⁵ gemeinsam eine C₂-C₄-Alkylen-kette oder C₂-C₄-Alkenylen-kette, welche unsubstituiert oder durch 1 oder 2 Reste aus der Gruppe Methyl, Ethyl, Methoxy, Ethoxy und Halogen substituiert ist,
R⁶ Wasserstoff, C₁-C₄-Alkyl, C₂-C₄-Alkenyl, C₂-C₄-Alkinyl, C₆-C₁₂-Aryl, Heteroaryl, Benzyl, C₁-C₄-Alkoxy, Acyloxy, Hydroxy, -NH-CO-NH₂, -NH-CS-NH₂, Mono- und Di-(C₁-C₄-alkyl)-amino, -NH-Acyl, -NHSO₂-(C₁-C₄-alkyl), C₆-C₁₂-Aryloxy, Heteroaryloxy, NH-SO₂-Aryl oder NH-Aryl, worin Aryl bzw. Heteroaryl in den letztgenannten 4 Resten unsubstituiert oder durch einen oder mehrere Reste aus der Gruppe Halogen, Nitro, (C₁-C₄)-Alkyl, (C₁-C₄)-Alkoxy, (C₁-C₄)-Haloalkyl und (C₁-C₄)-Haloalkoxy substituiert ist,
T O oder S, NR⁷, NOR⁷ oder NO-Acyl,
Q O oder S,
q eine ganze Zahl von 0 bis 4,
i eine Laufziffer, welche bei q ungleich 0 alle ganzen Zahlen von 1 bis q annimmt, wobei q die oben angegebene Bedeutung hat,
Xᵢ unabhängig voneinander O, S, NR⁷, N-(Aᵢ-Xᵢ-)_{q}-R,
Aᵢ unabhängig voneinander unsubstituiertes oder substituiertes C₁-C₆-Alkylen, C₂-C₆-Alkenylen, C₂-C₆-Alkinylen, C₃-C₆-Cycloalkylen, C₃-C₆-Cycloalkenylen, Heterocyclylen, Arylen oder Heteroarylen und
R⁷ unabhängig voneinander H, C₁-C₄-Alkyl, C₂-C₄-Alkenyl, C₂-C₄-Alkinyl, C₃-C₆-Cycloalkyl, C₃-C₆-Cycloalkenyl, Heterocyclyl, Aryl oder Heteroaryl
bedeuten, als Safener zum Schützen von Kulturpflanzen gegen phytotoxische Nebenwirkungen von Herbiziden.

2. Verwendung nach Anspruch 1, **dadurch gekennzeichnet, daß**
Ar einen unsubstituierten oder substituierten Phenyl- bzw. Naphthylrest der Formel 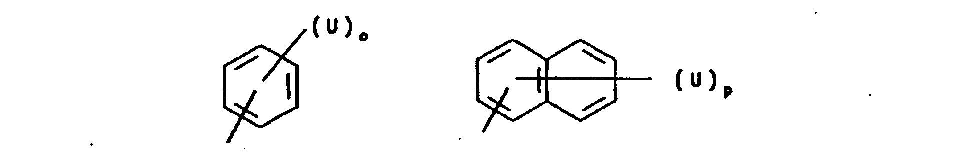
worin
(U) für gleiche oder verschiedene Reste stehen, welche unabhängig voneinander Wasserstoff, Halogen, Cyano, Nitro, Amino oder C₁-C₈-Haloalkyl, C₁-C₈-Haloalkoxy, C₁-C₈-Alkyl, C₁-C₈-Alkoxy, Mono-(C₁-C₄-alkyl)-amino, Di-(C₁-C₄-alkyl)-amino, C₁-C₈-Alkylthio oder C₁-C₈-Alkylsulfonyl, wobei jeder der letztgenannten 8 Reste unsubstituiert oder durch einen oder mehrere gleiche oder verschiedene Substituenten aus der Gruppe enthaltend Halogen, C₁-C₈-Haloalkoxy, Nitro, Cyano, Hydroxy, C₁-C₈-Alkoxy, worin eine oder mehrere CH₂-Gruppen durch Sauerstoff ersetzt sein können, C₁-C₈-Alkylthio, C₁-C₆-Alkylsulfinyl, C₁-C₆-Alkylsulfonyl, C₂-C₈-Alkenylthio, C₂-C₈-Alkinylthio, C₂-C₈-Alkenyloxy, C₂-C₈-Alkinyloxy, C₃-C₇-Cycloalkyl, C₃-C₇-Cycloalkoxy, Mono- und Di-(C₁-C₄-alkyl)-amino und C₁-C₈-Alkoxycarbonyl substituiert ist, bedeuten, und
o eine ganze Zahl von 1 bis 5 ist und
p eine ganze Zahl von 1 bis 7 ist, oder
Ar einen monocyclischen oder bicyclischen Heteroarylrest aus der Gruppe Furyl, Thienyl, Pyrrolyl, Pyrazolyl, Thiazolyl, Oxazolyl, Pyridinyl, Pyrimidinyl, Pyrazinyl, Pyridazinyl und Chinolinyl, der jeweils unsubstituiert oder durch einen oder mehrere der genannten Reste U substituiert ist,
R Wasserstoff, C₁-C₁₈-Alkyl, C₃-C₁₂-Cycloalkyl, C₂-C₈-Alkenyl oder C₂-C₈-Alkinyl, Heterocyclyl, Phenyl oder Heteroaryl,
wobei jeder der vorstehenden C-haltigen Reste unabhängig voneinander unsubstituiert oder durch einen oder mehrere Reste aus der Gruppe enthaltend Halogen, Cyano, Thio, Nitro, Hydroxy, C₁-C₈-Alkyl, letzteres nur für den Fall cyclischer Reste, C₁-C₈-Haloalkyl, C₁-C₈-Alkoxy, C₂-C₈-Alkenyloxy, C₂-C₈-Alkinyloxy, C₁-C₈-Haloalkoxy, C₁-C₈-Alkylthio, C₂-C₈-Alkenylthio, C₂-C₈-Alkinylthio, C₃-C₇-Cycloalkyl, C₃-C₇-Cycloalkoxy, Reste der Formeln -NR*R** und -CO-NR*R** und -O-CO-NR*R**, wobei R* und R** in den letztgenannten 3 Resten unabhängig voneinander Wasserstoff, C₁-C₈-Alkyl, C₂-C₈-Alkenyl, C₂-C₈-Alkinyl, Benzyl, Phenyl oder substituiertes Phenyl sind oder gemeinsam mit dem N-Atom einen 3 bis 8- gliedrigen Heterocyclus, der noch bis zu 2 weitere Heteroatome aus der Gruppe N, O und S enthalten und durch C₁-C₄-Alkyl substituiert sein kann, bedeuten, sowie (C₁-C₈-Alkoxy)-carbonyl, C₁-C₈-Alkoxy)-thiocarbonyl, (C₂-C₈-Alkenyloxy)-carbonyl, (C₁-C₈-Alkylthio)-carbonyl, (C₂-C₈-Alkenylthio)- carbonyl, (C₂-C₈-Alkinylthio)-carbonyl, (C₂-C₈-Alkinyloxy)-carbonyl, Formyl, (C₁-C₈-Alkyl)-carbonyl, (C₂-C₈-Alkenyl)-carbonyl, (C₂-C₈-Alkinyl)-carbonyl, C₁-C₄-Alkylimino, C₁-C₄-Alkoxyimino, (C₁-C₈-Alkyl)-carbonylamino, (C₂-C₈-Alkenyl)-carbonylamino, (C₂-C₈-Alkinyl)-carbonylamino, (C₁-C₈-Alkoxy)-carbonylamino, (C₂-C₈-Alkenyloxy)-carbonylamino, (C₂-C₈-Alkinyloxy)-carbonylamino, (C₁-C₈-Alkyl)-amino-carbonylamino, (C₁-C₆-Alkyl)-carbonyloxy, das unsubstituiert oder durch Halogen, NO₂, C₁-C₄-Alkoxy oder gegebenenfalls substituiertes Phenyl substituiert ist, (C₂-C₆-Alkenyl)-carbonyloxy, (C₂-C₆-Alkinyl)-carbonyloxy, (C₁-C₈-Alkoxy)-carbonyloxy, (C₂-C₈-Alkenyloxy)-carbonyloxy, (C₂-C₈-Alkinyloxy)-carbonyloxy, C₁-C₈-Alkylsulfonyl, Phenyl, Phenyl-C₁-C₆-alkoxy, Phenyl-(C₁-C₆-alkoxy)-carbonyl, Phenoxy, Phenoxy-C₁-C₆-alkoxy, Phenoxy-(C₁-C₆-alkoxy)-carbonyl, Phenoxycarbonyl, Phenylcarbonyloxy, Phenylcarbonylamino, Phenyl-(C₁-C₆-alkyl)-carbonylamino und Phenyl-(C₁-C₆-alkyl)-carbonyloxy, wobei die letztgenannten 11 Reste im Phenylring unsubstituiert oder durch einen oder mehrere Reste aus der Gruppe Halogen, C₁-C₄-Alkyl, C₁-C₄-Alkoxy, C₁-C₄-Haloalkyl, C₁-C₄-Haloalkoxy und Nitro substituiert sind, und Reste der Formeln -SiR'₃, -O-SiR'₃, (R')₃Si-C₁-C₆-alkoxy, -CO-O-NR'₂, -O-N = CR'₂, -N = CR'₂, -O-NR'₂, -CH(OR')₂ und -O-(CH₂)ₘ-CH(OR')₂, worin die R' in den genannten Formeln unabhängig voneinander Wasserstoff, C₁-C₄-Alkyl oder Phenyl, das unsubstituiert oder ein- oder mehrfach durch Reste aus der Gruppe Halogen, C₁-C₄-Alkyl, C₁-C₄-Alkoxy, C₁-C₄-Haloalkyl, C₁-C₄-Haloalkoxy und Nitro substituiert ist, oder paarweise eine C₂-C₆-Alkylenkette und m = 0 bis 6 bedeuten, und einen substituierten Alkoxyrest der Formel R"O-CHR'''CH(OR")-C₁-C₆-alkoxy, worin die R" unabhängig voneinander C₁-C₄-Alkyl oder zusammen eine C₁-C₆-Alkylengruppe und R''' Wasserstoff oder C₁-C₄-Alkyl bedeuten, substituiert ist, bedeuten.

3. Verwendung nach Anspruch 1 oder 2, **dadurch gekennzeichnet, daß**
R Wasserstoff, C₁-C₈-Alkyl, C₅-C₆-Cycloalkyl, C₂-C₈-Alkenyl oder C₂-C₈-Alkinyl, wobei jeder der letztgenannten 4 Reste unabhängig voneinander unsubstituiert oder durch einen oder mehrere Reste aus der Gruppe enthaltend Halogen, Cyano, Nitro, C₁-C₄-Alkoxy, C₂-C₄-Alkenyloxy, C₂-C₄-Alkinyloxy, C₅-C₆-Cycloalkyl, C₅-C₆-Cycloalkoxy, Mono- und Di-(C₁-C₄-alkyl)-amino, Reste der Formeln -SiR'₃, -O-N=CR'₂, -N=CR'₂, worin die R' in den genannten Formeln unabhängig voneinander Wasserstoff, C₁-C₂-Alkyl oder Phenyl oder paarweise eine C₂-C₅-Alkylenkette bedeuten, substituiert ist,
R^{T} -CO-R oder -NR^{f}R^{g} oder -N = CR^{h}Rⁱ, worin
R^{f}, R^{g} unabhängig voneinander H, C₁-C₂-Alkyl, Benzyl oder Phenyl oder gemeinsam mit dem N-Atom Pyrrolidin-1-yl, Piperidin-1-yl, Morpholin-4-yl, Piperazin-1-yl oder Imidazol-1-yl, bzw.
R^{h}, Rⁱ unabhängig voneinander H, C₁-C₂-Alkyl, Benzyl oder Phenyl bedeuten,
R⁴ und R⁵ gleich oder verschieden sind und unabhängig voneinander Wasserstoff, C₁-C₄-Alkyl, C₂-C₄-Alkenyl, C₂-C₄-Alkinyl, C₅-C₆-Cycloalkyl oder C₅-C₆-Cycloalkenyl,
R⁶ Wasserstoff, C₁-C₄-Alkyl oder Phenyl, Benzyl, Hydroxy, NH-CO-NH₂, -NH- Aryl oder C₁-C₄-Alkoxy,
T O, S oder NR⁷,
Q O oder S,
q eine ganze Zahl von O bis 4,
i eine Laufziffer, welche bei q ungleich 0 alle ganzen Zahlen von 1 bis q annimmt,
Xᵢ unabhängig voneinander O, S, NR⁷, N-(Aᵢ-Xᵢ-)_{q}-R,
Aᵢ unabhängig voneinander unsubstituiertes oder substituiertes C₁-C₄-Alkylen, C₂-C₄-Alkenylen, C₅-C₆-Cycloalkylen oder
R⁷ unabhängig voneinander H, C₁-C₄-Alkyl, C₂-C₄-Alkenyl, C₂-C₄-Alkinyl, C₅-C₆-Cycloalkyl bedeuten.

4. Verwendung nach einem der Ansprüche 1 bis 3, **dadurch gekennzeichnet, daß**
R¹ und R² unabhängig voneinander Reste der Formel 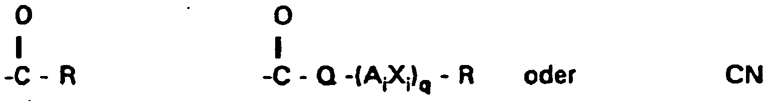
bedeuten.

5. Verbindungen der Formel I oder deren Salzen wie sie in einem der Ansprüche 1 bis 4 definiert sind, ausgenommen
a) Verbindungen der Formel I, worin
Ar Phenyl, das durch die Reste U¹, U² und U³ substituiert ist, wobei U¹ ein Rest aus der Gruppe Halogen, C₁ -C₄ -Alkyl, C₁-C₄ -Alkoxy, CF₃ und C₁-C₄ -Alkylsulfonyl ist und U² und U³ gleich oder verschieden sind und jeweils aus der Gruppe bestehend aus Wasserstoff, Halogen, C₁ -C₄ -Alkyl, C₁ -C₄ -Alkoxy, CF₃ und C₁ -C₄ -Alkylsulfonyl ausgewählt sind,
X ein Sauerstoffatom,
R¹ eine Gruppe der Formel -COOR,
R² eine Gruppe der Formel -COOR,
R³ C₁ -C₄ -Alkyl und
R gleiche oder verschiedene Reste aus der Gruppe Wasserstoff und C₁-C₄ -Alkyl
bedeuten, oder
b) Verbindungen der Formel 1, worin
Ar Phenyl, 1,3-Dichlorphenyl, 1,3,5-Trichlorphenyl, 3-Methoxyphenyl, Naphthyl, Cumarinyl, 4-Methyl-cumarinyl oder 7-Flavonyl,
X ein Sauerstoffatom,
R¹ eine Gruppe der Formel -COOR,
R² eine Gruppe der Formel -COOR,
R³ Wasserstoff und
R gleiche oder verschiedene Reste aus der Gruppe Wasserstoff, Aryl, Alkyl und Aralkyl bedeuten, oder
c) 2-(Chinolin-8-yl-mercapto)-malonsäurediethylester,
2-(Chinolin-8-yl-mercapto)-acetessigsäureethylester oder
4-Chlor-phenoxymalonsäurediethylester.

6. Verfahren zur Herstellung von Verbindungen der Formel I nach Anspruch 5, **dadurch gekennzeichnet, daß** man
a) eine Verbindung der Formel Ar-(X)ₙ-H, in der Ar und X die bei Formel I genannte Bedeutung haben und n = 1 ist, mit einer Verbindung der Formel II, 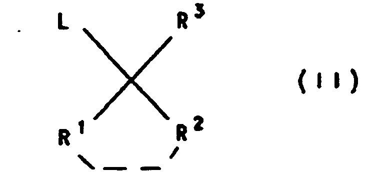 worin
L eine Abgangsgruppe bedeutet und
R¹, R² und R³ wie bei der genannten Formel I definiert sind,
umsetzt oder
b) eine Verbindung der Formel Ar-W mit einer Verbindung der Formel III, 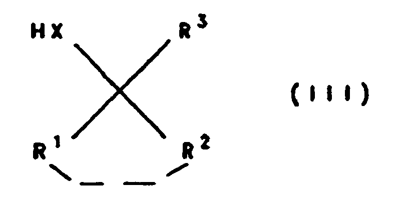 wobei
W eine Abgangsgruppe bedeutet und
Ar, X, R¹, R² und R³ wie bei der genannten Formel I definiert sind, umsetzt, oder
c) eine Verbindung der Formel Ar-X-W mit einer Verbindung der Formel IV, 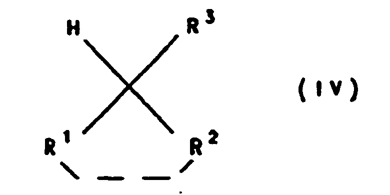 wobei
W eine Abgangsgruppe bedeutet, und
Ar, X, R¹, R² und R³ wie bei Formel I definiert sind,
umsetzt oder
d) ein Aryl- oder Heteroaryloxycarbonsäurederivat der Formel V 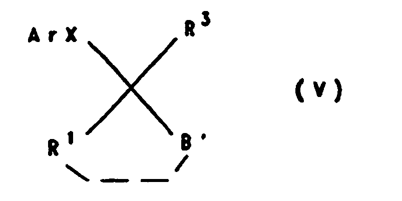 worin
Ar, X, R¹ und R³ wie bei Formel I definiert sind und B' eine Gruppe der Formeln 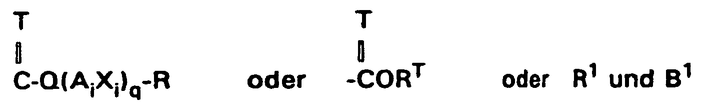 miteinander verbunden sind und gemeinsam eine Gruppe der Formel -CO-Q¹-D-Q²-CO- bedeuten, wobei T, Q, Aᵢ, Xᵢ, q, R, R^{T}, Q¹, Q² und D analog den gleichnamigen Resten in Formel I definiert sind, mit Alkoholen oder Mercaptanen umestert.

7. Mittel zum Schützen von Kulturpflanzen gegen phytotoxische Nebenwirkungen von Herbiziden **dadurch gekennzeichnet, daß** es als wirksamen Safener einen oder mehrere Verbindungen der Formel I oder deren Salze nach Anspruch 5 und übliche Formulierungshilfsmittel enthält.

8. Selektives herbizides Mittel, **dadurch gekennzeichnet, daß** es ein oder mehrere Herbizide und einen oder mehrere Safener der Verbindungen der Formel I oder deren Salzen nach einem der Ansprüche 1 bis 5 enthält.

9. Selektives herbizides Mittel nach Anspruch 8, **dadurch gekennzeichnet, daß** es ein oder mehrere Herbizide aus der Gruppe enthaltend Carbamate, Thiocarbamate, Halogenacetanilide, substituierte Phenoxy-, Naphthoxy- und Phenoxy-phenoxycarbonsäurederivate sowie Heteroaryloxyphenoxyalkancarbonsäurederivate, wie Chinolyloxy-, Chinoxalyloxy-, Pyridyloxy-, Benzoxalyloxy- und Benzthiazolyloxy-phenoxyalkancarbonsäureester, Cyclohexandionabkömmlinge, Imidazolinone, Pyrimidyloxy-pyridincarbonsäure-Derivate, Pyrimidyloxy-benzoesäure-derivate, Sulfonylharnstoffe sowie Triazolopyrimidin-sulfonamid-derivate sowie S-(N-Aryl-alkyl-carbamoylmethyl)-dithiophosphorsäureester enthält.

10. Mittel nach Anspruch 9, **dadurch gekennzeichnet, daß** das Gewichtsverhältnis Safener:Herbizid 1:10 bis 10:1 beträgt.

11. Mittel nach einem der Ansprüche 7, 8, 9 oder 10, **dadurch gekennzeichnet, daß** es 0,1 bis 99 Gewichtsprozent Wirkstoff der Formel I oder dessen Salz bzw. des Safener/Herbizid-Wirkstoffgemischs und 1 bis 99,9 Gew.-% eines festen oder flüssigen Zusatzstoffes und 0 bis 25 Gew.-% eines Tensides enthält.

12. Verfahren zum Schutz von Kulturpflanzen vor phytotoxischen Nebenwirkungen von Herbiziden, **dadurch gekennzeichnet, daß** eine wirksame Menge einer Verbindung der Formel I, wie sie in einem der Ansprüche 1 bis 5 definiert ist, vor, nach oder gleichzeitig mit dem Herbizid auf die Pflanzen, Pflanzenteile, Pflanzensamen oder die Anbaufläche appliziert wird.

13. Verfahren nach Anspruch 11, **dadurch gekennzeichnet, daß** die Kulturpflanzen Getreidepflanzen, Reispflanzen oder Maispflanzen sind.

14. Verfahren nach einem der Ansprüche 12 bis 13, **dadurch gekennzeichnet, daß** die Verbindungen der Formel I oder deren Salz in einer Aufwandmenge von 0,001 bis 5 kg/ha Aktivsubstanz und einem Gewichtsverhältnis Safener:Herbizid von 1:10 bis 10:1 appliziert werden.

## Claims

1. The use of compounds of the formula I or salts thereof 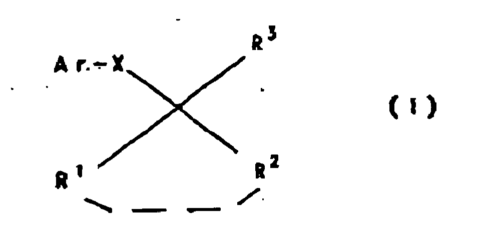 in which
R¹ and R², independently of one another, are radicals of the formula 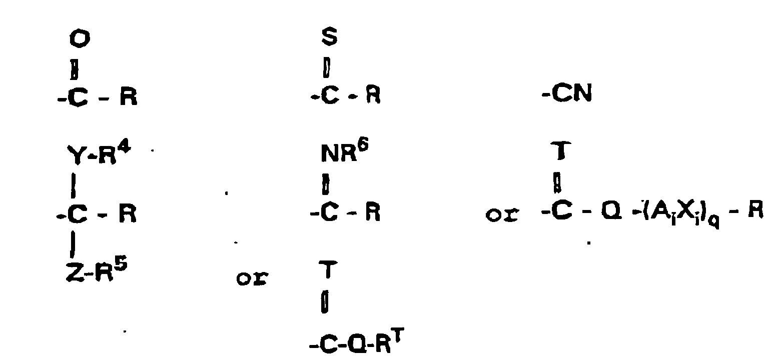 in which R, R^{T}, R⁴, R⁵, R⁶, Y, T, Z, Q, Aᵢ, Xᵢ and q are as defined below, or
R¹ and R² are bonded to one another and together are a group of the formula
-CO-Q¹-D-Q²-CO-
in which
Q¹ and Q², independently of one another, are as defined for Q and
D is a divalent group of the formula CR'R" or C=O, where R' and R", independently of one another, are hydrogen or C₁-C₄-alkyl,
R³ is hydrogen, halogen, C₁-C₁₈-alkyl, C₂-C₈-alkenyl, C₂-C₈-alkynyl, C₁-C₁₈-alkoxy, C₂-C₈-alkenyloxy, C₂-C₈-alkynyloxy, C₁-C₁₈-alkylthio, C₂-C₈-alkenylthio, C₂-C₈-alkynylthio, where each of the 9 last-mentioned radicals is in each case unsubstituted or substituted by one or more radicals from the group consisting of halogen, nitro and cyano, or is C₃-C₁₂-cycloalkyl which is unsubstituted or substituted by one or more radicals from the group consisting of C₁-C₄-alkyl, halogen, nitro and cyano, or is SiR^{a}R^{b}R^{c}, in which R^{a}, R^{b} and R^{c}, independently of one another, are C₁-C₄-alkyl, C₂-C₄-alkenyl, C₂-C₄-alkynyl or substituted or unsubstituted phenyl, or is a radical of the formula Ar'X'-, in which Ar' and X' are defined analogously to Ar and X,
X is O, S, NH-NH or NR^{d}, where R^{d} is defined analogously to R⁴, or is -CH₂O-, -CH₂S-, -CH(Ar)O- Or -CH(Ar)S-,
Ar is an aromatic radical,
R is hydrogen or an aliphatic, aromatic, heteroaromatic, araliphatic or hetero-araliphatic radical having 1 to 30 carbon atoms and, if desired, containing one or more furlctional groups,
R^{T} is a radical of the formula -CO-R, -CS-R, -NR^{f}R^{g}, -N=CR^{h}Rⁱ or SiR^{a}R^{b}R^{c}, where R is as defined above, and R^{f}, R^{g}, R^{h} and Rⁱ, independently of one another are hydrogen, C₁-C₄-alkyl, C₂-C₄-alkenyl, C₂-C₄-alkynyl, benzyl, phenyl or substituted phenyl, or R^{f} and R^{g} together with the nitrogen atom are a 5- or 6-membered heterocyclic ring which may contain up to 2 further heteroatoms from the group consisting of N, O and S, and which may be substituted by C₁-C₄-alkyl, and
R^{a}, R^{b} and R^{c}, independently of one another, are C₁-C₄-alkyl, C₂-C₄-alkenyl, C₂-C₄-alkynyl, phenyl or substituted phenyl,
Y and Z, independently of one another, are oxygen, sulfur in its various oxidation states or -NR^{e}, where R^{e} is defined analogously to R⁴,
R⁴ and R⁵ are identical or different and, independently of one another, are hydrogen, C₁-C₆-alkyl, C₂-C₆-alkenyl, C₂-C₆-alkynyl, (C₁-C₄-alkyl)-carbonyl, where each of the 4 last-mentioned radicals is unsubstituted or substituted by one or more substituents from the group consisting of halogen, C₁-C₈-haloalkoxy, nitro, cyano, hydroxyl, C₁-C₈-alkoxy and C₁-C₈-alkoxy, in which one or more, preferably up to three, CH₂ groups which are not bonded directly to one another are replaced by oxygen, and C₁-C₈-alkylthio, C₁-C₆-alkylsulfonyl, C₂-C₈-alkenylthio, C₂-C₈-alkynylthio, C₂-C₈-alkenyloxy, C₂-C₈-alkynyloxy, C₃-C₇-cycloalkyl, C₃-C₇-cycloalkoxy and amino, mono- and di-(C₁-C₄-alkyl)amino, or are formyl, SiR^{a}R^{b}R^{c}, in which R^{a}, R^{b} and R^{c}, independently of one another, are C₁-C₄- alkyl, C₂-C₄-alkenyl, C₂-C₄-alkynyl or substituted or unsubstituted phenyl, or are C₃-C₈-cycloalkyl, C₃-C₈-cycloalkenyl, heterocyclyl having 3 to 7 ring atoms, aryl, heteroaryl or azylcarbonyl, where each of the 6 last-mentioned radicals is unsubstituted or substituted by one or more radicals from the group consisting of C₁-C₈-alkyl, halogen, C₁-C₈-haloalkoxy, nitro, cyano, hydroxyl, C₁-C₈-alkoxy and C₁-C₈-alkoxy, in which one or more, preferably up to three, CH₂ groups which are not bonded directly to one another are replaced by oxygen, and C₁-C₈-alkylthio, C₁-C₆-alkylsulfonyl, C₂-C₈-alkenylthio, C₂-C₈-alkynylthio, C₂-C₈-alkenyloxy, C₂-C₈-alkynyloxy, C₃-C₇-cycloalkyl, C₃-C₇-cycloalkoxy, and amino, mono- and di-(C-₁-C₄-alkyl)amino, or
R⁴ and R⁵ together are a C₂-C₄-alkylene chain or a C₂-C₄-alkenylene chain which is unsubstituted or substituted by 1 or 2 radicals from the group consisting of methyl, ethyl, methoxy, ethoxy and halogen,
R⁶ is hydrogen, C₁-C₄-alkyl, C₂-C₄-alkenyl, C₂-C₄-alkynyl, C₆-C₁₂-aryl, heteroaryl, benzyl, C₁-C₄-alkoxy, acyloxy, hydroxyl,-NH-CO-NH₂, -NH-CS-NH₂, mono- and di-(C₁-C₄-alkyl)amino, -NH-acyl, -NHSO₂-(C₁-C₄-alkyl), C₆-C₁₂-aryloxy, heteroaryloxy, NH-SO₂-aryl, or NH-aryl, in which aryl or heteroaryl in the 4 last-mentioned radicals is unsubstituted or substituted by one or more radicals from the group consisting of halogen, nitro, (C₁-C₄)-alkyl, (C₁-C₄)-alkoxy, (C₁-C₄)-haloalkyl and (C₁-C₄)-haloalkoxy,
T is O, S, NR⁷, NOR⁷ or NO-acyl,
Q is O or S,
q is a integer from 0 to 4,
i is a serial number which, if q is not equal to 0, adopts all integers from 1 to q, where q is as defined above,
Xᵢ independently of one another, are O, S, NR⁷ or N-(Aᵢ-Xᵢ-)_{q}-R,
Aᵢ independently of one another, are unsubstituted or substituted C₁-C₆-alkylene, C₂-C₆-alkenylene, C₂-C₆-alkynylene, C₃-C₆-cycloalkylene, C₃-C₆-cycloalkenylene, heterocyclylene, arylene or heteroarylene, and
R⁷ independently of one another, are H, C₁-C₄-alkyl, C₂-C₄-alkenyl, C₂-C₄-alkynyl, C₃-C₆-cycloalkyl, C₃-C₆-cycloalkenyl, heterocyclyl, aryl or heteroaryl,
as safeners for protecting crop plants against the phytotoxic side effects of herbicides.

2. The use as claimed in claim 1, wherein
Ar is an unsubstituted or substituted phenyl or naphthyl radical of the formula 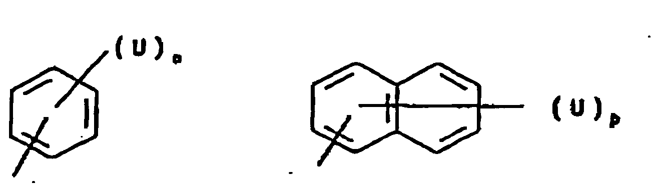 in which
(U) are identical or different radicals which, independently of one another, are hydrogen, halogen, cyano, nitro, amino or C₁-C₈-haloalkyl, C₁-C₈-haloalkoxy, C₁-C₈-alkyl, C₁-C₈-alkoxy, mono-(C₁-C₄-alkyl)amino, di-(C₁-C₄-alkyl)amino, C₁-C₈-alkylthio or C₁-C₈-alkylsulfonyl, where each of the 8 last-mentioned radicals is unsubstituted or substi- tuted by one or more identical or different substituents from the group consisting of halogen, C₁-C₈-haloalkoxy, nitro, cyano, hydroxyl, C₁-C₈-alkoxy, in which one or more CH₂ groups may be replaced by oxygen, C₁-C₈-alkylthio, C₁-C₆-alkylsulfinyl, C₁-C₆-alkylsulfonyl, C₂-C₈-alkenylthio, C₂-C₈-alkynylthio, C₂-C₈-alkenyloxy, C₂-C₈-alkynyloxy, C₃-C₇-cycloalkyl , C₃-C₇-cycloalkoxy, mono- and di- (C₁-C₄-alkyl) amino and C₁-C₈-alkoxycarbonyl, and
o is an integer from 1 to 5 and
p is an integer from 1 to 7 or
Ar is a monocyclic or bicyclic heteroaryl radical from the group consisting of furyl, thienyl, pyrrolyl, pyrazolyl, thiazolyl, oxazolyl, pyridinyl, pyrimidinyl, pyrazinyl, pyridazinyl and quinolinyl, each of which is unsubstituted or substituted by one or more of said radicals U,
R is hydrogen, C₁-C₁₈-alkyl, C₃-C₁₂-cycloalkyl, C₂-C₈-alkenyl, C₂-C₈-alkynyl, heterocyclyl, phenyl or heteroaryl,
where each of the above C-containing radicals, independently of one another, is unsubstituted or substituted by one or more radicals from the group consisting of halogen, cyano, thio, nitro, hydroxyl, C₁-C₈-alkyl, the latter only in the case of cyclic radicals, C₁-C₈-halo-alkyl, C₁-C₈-alkoxy, C₂-C₈-alkenyloxy, C₂-C₈₋alkynyloxy, C₁-C₈-haloalkoxy, C₁-C₈-alkylthio, C₂-C₈-alkenylthio, C₂-C₈-alkynylthio, C₃-C₇₋cycloalkyl, C₃-C₇-cycloalkoxy, radicals of the formulae -NR*R** and -CO-NR*R** and -O-CO-NR*R**, where R* and R** in the three last-mentioned radicals are, independently of one another, hydrogen, C₁-C₈-alkyl, C₂-C₈₋alkenyl, C₂-C₈-alkynyl, benzyl, phenyl or substituted phenyl, or together with the nitrogen atom are a 3- to 8-membered heterocyclic ring which may contain up to 2 further heteroatoms from the group consisting of N, O and S, and may be substituted by C₁-C₄₋alkyl, and (C₁-C₈-alkoxy) carbonyl, (C₁-C₈₋alkoxy)thiocarbonyl, (C₂-C₈-alkenyloxy)carbonyl, (C₁-C₈-alkylthio)carbonyl, (C₂-C₈₋alkenylthio)carbonyl, (C₂-C₈-alkynylthio)-carbonyl, (C₂-C₈-alkynyloxy)carbonyl, formyl, (C₁-C₈-alkyl)carbonyl, (C₂-C₈-alkenyl)carbonyl, (C₂-C₈-alkynyl)carbonyl, C₁-C₄-alkylimino, C₁-C₄-alkoxyimino, (C₁-C₈-alkyl)carbonylamino, (C₂-C₈-alkenyl)carbonylamino, (C₂-C₈-alkynyl)carbonylamino, (C₁-C₈-alkoxy)carbonylamino, (C₂-C₈-alkenyloxy)carbonylamino, (C₂-C₈-alkynyloxy)carbonylamino, (C₁-C₈-alkyl)aminocarbonylamino, (C₁-C₆-alkyl)carbonyloxy, which is unsubstituted or substituted by halogen, NO₂, C₁-C₄-alkoxy or substituted or unsubstituted phenyl, (C₂-C₆-alkenyl) carborlyloxy, (C₂-C₆₋alkynyl) carbonyloxy, (C₁-C₈-alkoxy)carbonyloxy, (C₂-C₈-alkenyloxy) -carbonyloxy, (C₂-C₈-alkynyloxy)carbonyloxy, C₁-C₈-alkylsulfonyl, phenyl, phenyl-C₁-C₆-alkoxy, phenyl-(C₁-C₆-alkoxy)carbonyl, phenoxy, phenoxy-C₁-C₆-alkoxy, phenoxy-(C₁-C₆-alkoxy) -carbonyl, phenoxycarbonyl, phenylcarbonyloxy, phenylcarbonylamino, phenyl-(C₁-C₆-alkyl)-carbonylamino and phenyl-(C₁-C₆-alkyl)-carbonyloxy, where the 11 last-mentioned radicals are unsubstituted or substituted on the phenyl ring by one or more radicals from the group consisting of halogen, C₁-C₄-alkyl, C₁-C₄-alkoxy, C₁-C₄-haloalkyl, C₁-C₄-haloalkoxy and nitro, and radicals of the formulae -SiR'₃, -O-SiR'₃, (R')₃Si-C₁-C₆-alkoxy, -CO-O-NR'₂, -O-N=CR' ₂. -N=CR'₂, -O-NR'₂, -CH(OR')₂. and -O-(CH₂)ₘ-CH(OR')₂, in which the R' in said formulae are, independently of one another, hydrogen, C₁-C₄-alkyl or phenyl, which is unsubstituted or monosubstituted or polysubstituted by radicals from the group consisting of halogen, C₁-C₄-alkyl, C₁-C₄₋alkoxy, C₁-C₄-haloalkyl, C₁-C₄-haloalkoxy and nitro, or in pairs are a C₂-C₆-alkylene chain and m = 0 to 6, or a substituted alkoxy radical of the formula R"O-CHR'''CH(OR")-C₁-C₆₋alkoxy, in which the R", independently of one another, are C₁-C₄-alkyl or together are a C₁-C₆-alkylene group, and R"' is hydrogen or C₁-C₄-alkyl.

3. The use as claimed in claim 1 or 2, wherein
R is hydrogen, C₁-C₈-alkyl, C₅-C₆-cycloalkyl, C₂-C₈-alkenyl or C₂-C₆-alkynyl, where each of the 4 last-mentioned radicals, independently of one another, are unsubstituted or substituted by one or more radicals from the group consisting of halogen, cyano, nitro, C₁-C₄-alkoxy, C₂-C₄-alkenyloxy, C₂-C₄-alkynyloxy, C₅-C₆-cycloalkyl, C₅-C₆-cycloalkoxy, mono- and di-(C₁-C₄-alkyl)amino, radicals of the formulae -SiR'₃, -O-N=CR'₂, -N=CR'₂, in which the R' in said formulae are, independently of one another, hydrogen, C₁-C₂-alkyl or phenyl or in pairs are a C₂-C₅-alkylene chain,
R^{T} is -CO-R, -NR^{f}R^{g} or -N=CR^{h}Rⁱ, in which
R^{f} and R^{g} independently of one another, are H, C₁-C₂-alkyl, benzyl or phenyl or together with the nitrogen atom are pyrrolidin-1-yl, piperidin-1-yl, morpholin-4-yl, piperazin-1-yl or imidazol-1-yl, and
R^{h} and Rⁱ, independently of one another, are H, C₁-C₂-alkyl, benzyl or phenyl,
R⁴ and R⁵ are identical or different and, independently of one another, are hydrogen, C₁-C₄-alkyl, C₂-C₄-alkenyl, C₂-C₄-alkynyl, C₅-C₆-cycloalkyl or C₅-C₆-cycloalkenyl,
R⁶ is hydrogen, C₁-C₆-alkyl, phenyl, benzyl, hydroxyl, NH-CO-NH₂, -NH-aryl or C₁-C₄-alkoxy,
T is O, S or NR⁷,
Q is O or S,
q is an integer from 0 to 4,
i is a serial number which, if q is not equal to 0, adopts all integers from 1 to q,
Xᵢ independently of one another, are O, S, NR⁷ or N-(Aᵢ-Xᵢ-)_{q}-R,
Aᵢ independently of one another, are unsubstituted or substituted C₁-C₄-alkylene, C₂-C₄-alkenylene or C₅-C₆-cycloalkylene,
R⁷ independently of one another, are H, C₁-C₄-alkyl, C₂-C₄-alkenyl, C₂-C₄-alkynyl or C₅-C₆-cycloalkyl.

4. The use as claimed in any one of claims 1 to 3, wherein
R¹ and R², independently of one another, are radicals of the formula

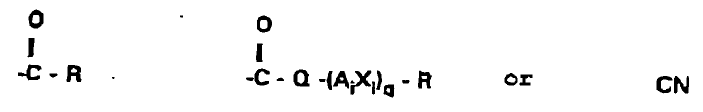

5. A compound of the formula I, or a salt thereof, as defined in any one of claims 1 to 4, other than
a) compounds of the formula I wherein
Ar is phenyl substituted by the radicals U¹, U² and U³, U¹ being a radical selected from the group consisting of halogen, C₁-C₄-alkyl, C₁-C₄-alkoxy, CF₃ and C₁-C₄-alkylsulfonyl and U² and U³ being the same or different and each being selected from the group consisting of hydrogen, halogen, C₁-C₄-alkyl, C₁-C₄-alkoxy, CF₃ and C₁-C₄-alkylsulfonyl,
X is an oxygen atom,
R¹ is a group of the formula -COOR,
R² is a group of the formula -COOR,
R³ is C₁-C₄-alkyl, and
R represents identical or different radicals from the group consisting of hydrogen and C₁-C₄-alkyl,
or
b) compounds of the formula I wherein
Ar is phenyl, 1,3-dichlorophenyl, 1,3,5-tri-chlorophenyl, 3-methoxyphenyl, naphthyl, coumarinyl, 4-methylcoumarinyl or 7-flavonyl,
X is an oxygen atom,
R¹ is a group of the formula -COOR,
R² is a group of the formula -COOR,
R³ is hydrogen, and
R represents identical or different radicals from the group consisting of hydrogen, aryl, alkyl and aralkyl,
or
c) diethyl 2-(quinolin-8-yl-mercapto)malonate, ethyl 2-(quinolin-8-yl-mercapto)acetoacetate, or
diethyl 4-chlorophenoxymalonate.

6. A process for the preparation of a compound of the formula I as claimed in claim 5, which comprises
a) reacting a compound of the formula Ar-(X)ₙ-H, in which Ar and X are as defined under formula I and n = 1, with a compound of the formula II 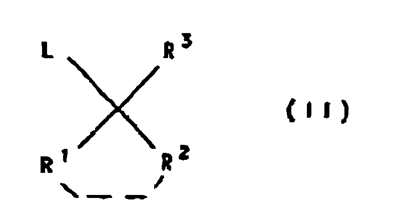 in which
L is a leaving group and
R¹, R² and R³ are as defined under said formula I,
or
b) reacting a compound of the formula Ar-W with a compound of the formula III 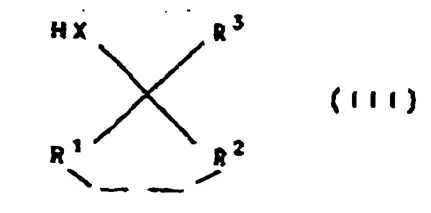 where
W is a leaving group and
Ar, X, R¹, R² and R³ are as defined under said formula I, or
c) reacting a compound of the formula Ar-X-W with a compound of the formula IV 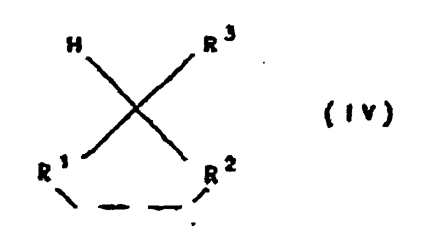 where
W is a leaving group and
Ar, X, R¹, R² and R³ are as defined under formula I,
or
d) transesterifying an aryl- or heteroaryloxycarboxylic acid derivative of the formula V 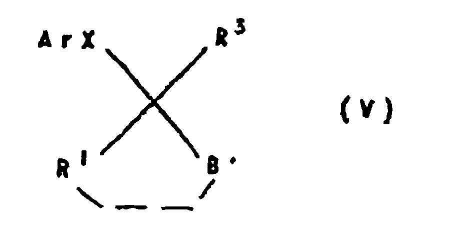 in which
Ar, X, R¹ and R³ are as defined under formula I, and
B' is a group of the formulae 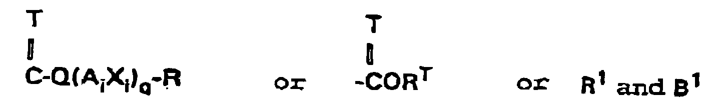 are bonded to one another and together are a group of the formula -CO-Q¹-D-Q²-CO-, where T, Q, Aᵢ, Xᵢ, q, R, R^{T}, Q¹, Q² and D are defined analogously to the radicals of the same names in formula I, with alcohols or mercaptans.

7. A composition for protecting crop plants against the phytotoxic side effects of herbicides which contains one or more compounds of the formula I, or salts thereof, as claimed in claim 5 and conventional formulation auxiliaries as effective safeners.

8. A selective herbicidal composition which contains one or more herbicides and one or more safeners of compounds of the formula I, or salts thereof, as claimed in any one of claims 1 to 5.

9. A selective herbicidal composition as claimed in claim 8, which contains one or more herbicides from the group consisting of carbamates, thiocarbamates, haloacetanilides, substituted phenoxy-, naphthoxy- and phenoxyphenoxycarboxylic acid derivatives and heteroaryloxyphenoxyalkanecarboxylic acid derivatives, such as quinolyloxy-, quinoxalyloxy-, pyridyloxy-, benzoxalyloxy- and benzothiazolyloxyphenoxyalkanecarboxylic esters, cyclohexanedione derivatives, imidazolinones, pyrimidyloxypyridine-carboxylic acid derivatives, pyrimidyloxybenzoic acid derivatives, sulfonylureas and triazolopyrimidinesulfonamide derivatives and S-(N-arylalkylcarbamoylmethyl)dithiophosphoric esters.

10. A composition as claimed in claim 9, wherein the safener:herbicide weight ratio is from 1:10 to 10:1.

11. A composition as claimed in any one of claims 7, 8, 9 or 10, which contains from 0.1 to 99 percent by weight of an active compound of the formula I or a salt thereof or of the safener/herbicide active compound mixture and from 1 to 99.9% by weight of a solid or liquid additive and from 0 to 25% by weight of a surfactant.

12. A method of protecting crop plants against the phytotoxic side effects of herbicides, wherein an effective amount of a compound of the formula I as defined in any one of claims 1 to 5 is applied to the plants, parts of plants, plant seeds or cultivated area before, after or simultaneously with the herbicide.

13. The method as claimed in claim 11, wherein the crop plants are cereal plants, rice plants or corn plants.

14. A method as claimed in either claim 12 or 13, wherein the compound of the formula I or a salt thereof is applied at a rate of from 0.001 to 5 kg/ha of active substance and in a safener:herbicide weight ratio of from 1:10 to 10:1.

## Revendications

1. Utilisation de composés de formule I ou leurs sels ou leurs sels 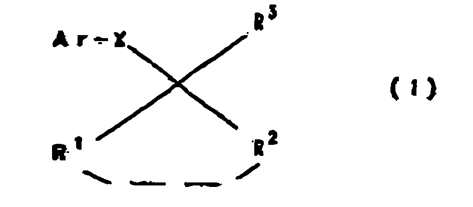 où
R¹ et R² indépendamment l'un de l'autre représentent des restes de formule 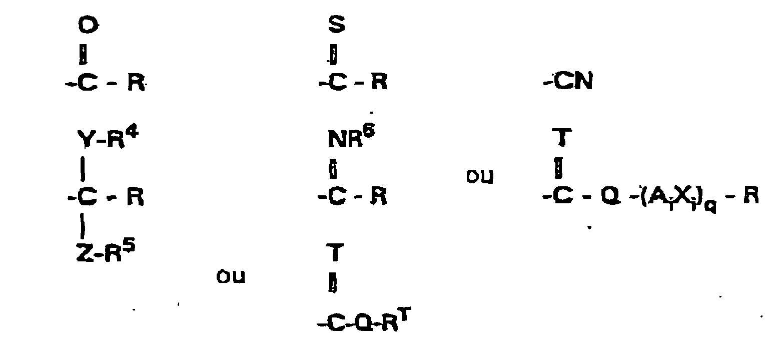
où R, R^{T}, R⁴, R⁵, R⁶, Y, T, Z, Q, Aᵢ, Xᵢ, q sont définis comme ci-après, ou
R¹ et R² sont liés l'un à l'autre et conjointement forment un groupe de formule
-CO-Q¹-D-Q²-CO-
où
Q¹, Q² indépendamment l'un de l'autre sont définis comme Q et
D représente un groupe bivalent de formule CR'R" ou C=O, R' et R" indépendamment l'un de l'autre représentent un atome d'hydrogène ou un groupe alkyle en C₁-C₄,
R³ représente un atome d'hydrogène, un atome d'halogène, un groupe alkyle en C₁-C₁₈, alcényle en C₂-C₈, alcynyle en C₂-C₈, alkoxy en C₁-C₁₈, alcényloxy en C₂-C₈, alcynyloxy en C₂-C₈, (alkyl en C₁-C₁₈)thio, (alcényl en C₂-C₈)thio, (alcynyl en C₂-C₈)thio, chacun des derniers 9 restes étant à chaque fois non substitué ou substitué par un ou plusieurs restes pris dans le groupe comprenant halogène, nitro et cyano, ou cycloalkyle en C₃-C₁₂, qui est non substitué ou substitué par un ou plusieurs restes pris dans le groupe comprenant alkyle en C₁-C₄, halogène, nitro et cyano, ou un groupe SiR^{a}R^{b}R^{c}, où R^{a}, R^{b} et R^{c} indépendamment les uns des autres représentent un groupe alkyle en C₁-C₄, alcényle en C₂-C₄, alcynyle en C₂-C₄ ou phényle non substitué ou substitué, ou un reste de formule Ar'X'-, où Ar' et X' sont définis de façon analogues à Ar ou à X,
X représente O, S, NH-NH ou NR^{d}, R^{d} est défini de façon analogue à R⁴, ou -CH₂O-, -CH₂S-, -CH(Ar)O- ou -CH(Ar)S-,
Ar représente un reste aromatique,
R représente un atome d'hydrogène ou un reste aliphatique, aromatique, hétéroaromatique, araliphatique ou hétéroaliphatique, présentant 1 à 30 atomes de carbone et éventuellement un ou plusieurs groupes fonctionnels,
R^{T} représente un reste de formule -CO-R, -CS-R, -NR^{f}R^{g}, -N=CR^{h}Rⁱ ou SiR^{a}R^{b}R^{c},
R possède la signification citée et R^{f}, R^{g}, R^{h} et Rⁱ représentent indépendamment les uns des autres un atome d'hydrogène, un groupe alkyle en C₁-C₄, alcényle en C₂-C₄, alcynyle en C₂-C₄, benzyle, phényle ou phényle substitué ou R^{f} et R^{g} conjointement avec l'atome de N forment un hétérocycle de 5 ou 6 chaînons, qui peut présenter encore jusqu'à 2 autres hétéroatomes pris dans le groupe comprenant N, O et S et peut être substitué par un substituant alkyle en C₁-C₄, et
R^{a}, R^{b} et R^{c} indépendamment les uns des autres représentent un groupe alkyle en C₁-C₄, alcényle en C₂-C₄, alcynyle en C₂-C₄, phényle ou phényle substitué,
Y et Z indépendamment l'un de l'autre représentent un atome d'oxygène, un atome de soufre dans ses différents degrés d'oxydation, ou -NR^{e}, où R^{e} est défini de façon analogue à R⁴,
R⁴ et R⁵ sont identiques ou différents et représentent indépendamment l'un de l'autre un atome d'hydrogène, un groupe alkyle en C₁-C₆, alcényle en C₂-C₆, alcynyle en C₂-C₆, (alkyl en C₁-C₄)carbonyle, les 4 derniers restes cités sont non substitués ou substitués par un ou plusieurs substituants pris dans le groupe comprenant un atome d'halogène, un groupe haloalkoxy en C₁-C₈, nitro, cyano, hydroxy, alkoxy en C₁-C₈ et alkoxy en C₁-C₈, où un ou plusieurs, de préférence jusqu'à trois groupes CH₂ non liés directement l'un à l'autre sont remplacés par des atomes d'oxygène, et (alkyl en C₁-C₈)thio, (alkyl en C₁-C₆)sulfonyle, (alcényl en C₂-C₈)thio, (alcynyl en C₂-C₈)thio, (alcényl en C₂-C₈)oxy, (alcynyl en C₂-C₈)oxy, cycloalkyle en C₃-C₇, cycloalkoxy en C₃-C₇, ainsi qu'amino, mono- et di-(alkyl en C₁-C₄)-amino, ou formyle, SiR^{a}R^{b}R^{c}, où R^{a}, R^{b} et R^{c} indépendamment les uns des autres représentent un groupe alkyle en C₁-C₄, alcényle en C₂-C₄, alcynyle en C₂-C₄ ou phényle non substitué ou substitué, ou cycloalkyle en C₃-C₈, cycloalcényle en C₃-C₈, hétérocyclyle présentant 3 à 7 chaînons, aryle, hétéroaryle ou arylcarbonyle, chacun des 6 derniers restes cités pouvant être non substitué ou substitué par un ou plusieurs restes pris dans le groupe comprenant alkyle en C₁-C₈, halogène, haloalkoxy en C₁-C₈, nitro, cyano, hydroxy, alkoxy en C₁-C₈ et alkoxy en C₁-C₈, où un ou plusieurs, de préférence jusqu'à trois, groupes CH₂ non liés directement les uns aux autres sont remplacés par des atomes d'oxygène, et (alkyl en C₁-C₈)thio, (alkyl en C₁-C₆) sulfonyle, (alcényl en C₂-C₈)thio, (alcynyl en C₂-C₈)thio, (alcényl en C₂-C₈)oxy, (alcynyl en C₂-C₈)oxy, cycloalkyle en C₃-C₇, cycloalkoxy en C₃-C₇, ainsi qu'amino, mono- et di-(alkyl en C₁-C₄) -amino, ou
R⁴ et R⁵ conjointement forment une chaîne alkylène en C₂-C₄ ou alcénylène en C₂-C₄, qui est non substituée ou substituée par 1 ou 2 restes pris dans le groupe comprenant méthyle, éthyle, méthoxy, éthoxy et halogène,
R⁶ représente un atome d'hydrogène, un groupe alkyle en C₁-C₄, alcényle en C₂-C₄, alcynyle en C₂-C₄, aryle en C₆-C₁₂, hétéroaryle, benzyle, alkoxy en C₁-C₄, acyloxy, hydroxy, -NH-CO-NH₂, -NH-CS-NH₂, mono- et di- (alkyl en C₁-C₄)-amino, -NH-acyle, -NHSO₂-(alkyle en C₁-C₄), (aryl en C₆-C₁₂)-oxy, hétéroaryloxy, NH-SO₂-aryle ou NH-aryle, où aryle ou hétéroaryle dans les 4 derniers restes cités sont non substitués ou substitués par un ou plusieurs restes pris dans le groupe comprenant halogène, nitro, alkyle en C₁-C₄, alkoxy en C₁-C₄, haloalkyle en C₁-C₄ et haloalkoxy en C₁-C₄,
T représente un atome de 0 ou de S, un groupe NR⁷, NOR⁷ ou NO-acyle,
Q représente un atome de 0 ou de S,
q est un entier de 0 à 4,
i est un numéro d'ordre, qui adopte pour q inégal à 0 tous les entiers de 1 à q, q possédant la signification donnée ci-dessus,
Xᵢ indépendamment les uns des autres représentent un atome de O, de S, un groupe NR⁷, N- (Aᵢ-Xᵢ-)_{q}-R,
Aᵢ indépendamment les uns des autres représentent un groupe alkylène en C₁-C₆, alcénylène en C₂-C₆, alcynylène en C₂-C₆, cycloalkylène en C₃-C₆, cycloalcénylène en C₃-C₆, hétérocyclylène, arylène ou hétéroarylène non substitué ou substitué, et
R⁷ indépendamment les uns des autres représentent un atome d'hydrogène, un groupe alkyle en C₁-C₄, alcényle en C₂-C₄, alcynyle en C₂-C₄, cycloalkyle en C₃-C₆, cycloalcényle en C₃-C₆, hétérocyclyle, aryle ou hétéroaryle,
en tant qu'antidote pour la protection de plantes de culture contre les effets secondaires phytotoxiques des herbicides.

2. Utilisation selon la revendication 1, **caractérisée en ce que**
Ar représente un reste phényle ou naphtyle non substitué ou substitué de formule 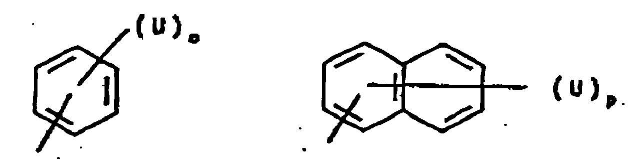
où
(U) représente des restes identiques ou différents, qui indépendamment les uns des autres représentent un atome d'hydrogène, un atome d'halogène, un groupe cyano, nitro, amino ou haloalkyle en C₁-C₈, haloalkoxy en C₁-C₈, alkyle en C₁-C₈, alkoxy en C₁-C₈, mono-(alkyl en C₁-C₄)-amino, di-(alkyl en C₁-C₄)-amino, (alkyl en C₁-C₈)-thio ou (alkyl en C₁-C₈)sulfonyle, chacun des 8 derniers cités est non substitué ou substitué par un ou plusieurs substituants identiques ou différents pris dans le groupe comprenant un atome d'halogène, un groupe haloalkoxy en C₁-C₈, nitro, cyano, hydroxy, alkoxy en C₁-C₈, où un ou plusieurs groupes CH₂ peuvent être remplacés par des atomes d'oxygène, (alkyl en C₁-C₈)thio, (alkyl en C₁-C₆)-sulfinyle, (alkyl en C₁-C₆)-sulfonyle, (alcényl en C₂-C₈)thio, (alcynyl en C₂-C₈)thio, (alcényl en C₂-C₈)oxy, (alcynyl en C₂-C₈)oxy, cycloalkyle en C₃-C₇, cycloalkoxy en C₃-C₇, mono- et di-(alkyl en C₁-C₄)amino et (alkoxy en C₁-C₈)-carbonyle, et
o est un entier de 1 à 5 et
p est un entier de 1 à 7, ou
Ar représente un reste hétéroaryle monocyclique ou bicyclique, pris dans le groupe comprenant furyle, thiényle, pyrrolyle. pyrazolyle, thiazolyle, oxazolyle, pyridinyle, pyrimidinyle, pyrazinyle, pyridazinyle et quinoléinyle, chacun pouvant être non substitué ou substitué par un ou plusieurs des restes U précités,
R représente un atome d'hydrogène, un groupe alkyle en C₁-C₁₈, cycloalkyle en C₃-C₁₂, alcényle en C₂-C₈, alcynyle en C₂-C₈, hétérocyclyle, phényle ou hétéroaryle,
chacun des restes carbonés précités indépendamment les uns des autres est non substitué ou substitué par un ou plusieurs restes pris dans le groupe comprenant halogène, cyano, thio, nitro, hydroxy, alkyle en C₁-C₈, ce dernier seulement dans le cas de restes cycliques, haloalkyle en C₁-C₈, alkoxy en C₁-C₈, (alcényl en C₂-C₈)oxy, (alcynyl en C₂-C₈)oxy,
haloalkoxy en C₁-C₈, (alkyl en C₁-C₈)thio, (alcényl en C₂-C₈)thio, (alcynyl en C₂-C₈)thio, cycloalkyle C₃-C₇, cycloalkoxy C₃-C₇, des restes de formules -NR*R** et -CO-NR*R** et -O-CO-NR*R**, R* et R** dans les 3 derniers restes cités indépendamment l'un de l'autre représentent un atome d'hydrogène, un groupe alkyle en C₁-C₁₆, alcényle en C₂-C₈, alcynyle en C₂-C₈, benzyle, phényle ou phényle substitué, ou conjointement avec l'atome d'azote forment un hétérocycle de 3 à 8 chaînons, qui peut présenter encore jusqu'à 2 autres hétéroatomes pris dans le groupe comprenant N, 0 et S, et peut être substitué par un substituant alkyle en C₁-C₄, ainsi que (alkoxy en C₁-C₈)carbonyle, (alkoxy en C₁-C₈)-thiocarbonyle, (alcényl en C₂-C₈)-oxycarbonyle, (alkyl en C₁-C₈)-thiocarbonyle, (alcényl en C₂-C₈)-thiocarbonyle, (alcynyl en C₂-C₈)-thiocarbonyle, (alcynyl en C₂-C₈)-oxycarbonyle, formyle, (alkyl en C₁-C₈)-carbonyle, (alcényl en C₂-C₈)carbonyle, (alcynyl en C₂-C₈)carbonyle, (alkyl en C₁-C₄)-imino, (alkoxy en C₁-C₄) imino, (alkyl en C₁-C₈) -carbonylamino, (alcényl en C₂-C₈)-carbonylamino, (alcynyl en C₂-C₈)-carbonylamino, (alkoxy en C₁-C₈)-carbonylamino, (alcényl en C₂-C₈)-oxycarbonylamino, (alcynyl en C₂-C₈)-oxycarbonylamino, (alkyl en C₁-C₈)-aminocarbonylamino, (alkyl en C₁-C₆)-carbonyloxy, qui est non substitué ou substitué par un substituant halogène, NO₂, alkoxy en C₁-C₄ ou phényle éventuellement substitué, (alcényl en C₂-C₆) -carbonyloxy, (alcynyl en C₂-C₆)-carbonyloxy, (alkoxy en C₁-C₈)carbonyloxy, (alcényloxy en C₂-C₈)carbonyloxy, (alcynyloxy en C₂-C₈) carbonyloxy, (alkyl en C₁-C₈)-sulfonyle, phényle, phénylalkoxy en C₁-C₆, phényl(alkoxy en C₁-C₆) carbonyle, phénoxy, phénoxyalkoxy en C₁-C₆, phénoxy(alkoxy en C₁-C₆) carbonyle, phénoxycarbonyle, phénylcarbonyloxy, phénylcarbonylamino, phényl(alkyl en C₁-C₆)-carbonylamino et phényl(alkyl en C₁-C₆)-carbonyloxy, les 11 derniers restes cités sont non substitués ou substitués dans le cycle phényle par un ou plusieurs restes pris dans le groupe comprenant halogène, alkyle en C₁-C₄, alkoxy en C₁-C₄, haloalkyle en C₁-C₄, haloalkoxy en C₁-C₄ et nitro, et les restes de formules -SiR'₃, -O-SiR'₃, (R')₃Si-(alkoxy en C₁-C₆), -CO-O-NR'₂, -O-N=CR'₂, -N=CR'₂, -O-NR'₂, -CH(OR')₂ et -O-(CH₂)ₘ-CH(OR')₂, où R' dans les formules citées, indépendamment les uns des autres, représentent un atome d'hydrogène, un groupe alkyle en C₁-C₄ ou phényle qui est non substitué ou substitué une ou plusieurs fois par des restes pris dans le groupe comprenant halogène, alkyle en C₁-C₄, alkoxy en C₁-C₄, haloalkyle en C₁-C₄, haloalkoxy en C₁-C₄ et nitro, ou par paires représentent une chaîne alkylène en C₂-C₆ et m = 0 à 6, et un reste alkoxy substitué de formule R"O-CHR'''CH(OR")- alkoxy en C₁-C₆, dans laquelle R" indépendamment les uns des autres représentent un groupe alkyle en C₁-C₄ ou conjointement un groupe alkylène en C₁-C₆ et R"' représente un atome d'hydrogène ou un groupe alkyle en C₁-C₄.

3. Utilisation selon la revendication 1 ou 2, **caractérisée en ce que**
R représente un atome d'hydrogène, un groupe alkyle en C₁-C₈, cycloalkyle en C₅-C₆, alcényle en C₂-C₈ ou alcynyle en C₂-C₈, chacun des 4 derniers restes cités indépendamment les uns des autres sont non substitués ou substitués par un ou plusieurs restes pris dans le groupe comprenant halogène, cyano, nitro, alkoxy en C₁-C₄, (alcényl en C₂-C₄)oxy, (alcynyl en C₂-C₄)-oxy, cycloalkyle en C₅-C₆, cycloalkoxy en C₅-C₆, mono- et di-(alkyl en C₁-C₄)amino, des restes de formules -SiR'₃, -O-N=CR'₂, -N=CR'₂, où les R' dans les formules précitées représentent indépendamment les uns des autres un atome d'hydrogène, un groupe alkyle en C₁-C₂ ou phényle, ou par paires une chaîne alkylène en C₂-C₅,
R^{T} représente un groupe -CO-R ou NR^{f}R^{g} ou -N=CR^{h}Rⁱ,
où
R^{f}, R^{g} indépendamment l'un de l'autre représentent un atome d'hydrogène, un groupe alkyle en C₁-C₂, benzyle ou phényle, ou conjointement avec l'atome de N forment un cycle pyrrolidin-1-yle, pipéridin-1-yle, morpholin-4-yle, pipérazin-1-yle ou imidazol-1-yle, ou
R^{b}, Rⁱ indépendamment l'un de l'autre représentent un atome d'hydrogène, un groupe alkyle en C₁-C₂, benzyle ou phényle,
R⁴ et R⁵ identiques ou différents, indépendamment l'un de l'autre, représentent un atome d'hydrogène, un groupe alkyle en C₁-C₄, alcényle en C₂-C₄, alcynyle en C₂-C₄, cycloalkyle en C₅-C₆ ou cycloalcényle en C₅-C₆,
R⁶ représente un atome d'hydrogène, un groupe alkyle en C₁-C₄ ou phényle, benzyle, hydroxy, NH-CO-NH₂, -NH-aryle ou alkoxy en C₁-C₄,
T représente un atome de O, de S ou un groupe NR⁷,
Q représente un atome de O ou de S,
q est un entier de 0 à 4,
i est un numéro d'ordre, qui adopte pour q inégal à 0 tous les entiers de 1 à q,
Xᵢ indépendamment les uns des autres représentent un atome de O, de S, un groupe NR⁷, N- (Aᵢ-Xᵢ-)_{q}-R,
Aᵢ indépendamment les uns des autres représentent un groupe alkylène en C₁-C₄, alcénylène en C₂-C₄, cycloalkylène en C₅-C₆ non substitué ou substitué ou
R⁷ indépendamment l'un de l'autre représentent un atome d'hydrogène, un groupe alkyle en C₁-C₄, alcényle en C₂-C₄, alcynyle en C₂-C₄, cycloalkyle en C₅-C₆.

4. Utilisation selon l'une des revendications 1 à 3, **caractérisée en ce que**
R₁ et R₂ indépendamment l'un de l'autre représentent des restes de formule

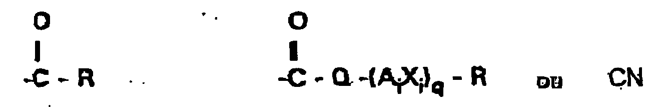

5. Composés de formule I ou leurs sels tels que définis dans une des revendications 1 à 4, à l'exception
a) de composés de formule I, où
Ar représente un groupe phényle, qui est substitué par des restes U¹, U² et U³, U¹ étant un reste pris dans le groupe comprenant halogène, alkyle en C₁-C₄, alkoxy en C₁-C₄, CF₃ et (alkyl en C₁-C₄) - sulfonyle et U² et U³ sont identiques ou différents et sont pris chacun dans le groupe comprenant un atome d'hydrogène, un atome d'halogène, un groupe alkyle en C₁-C₄, alkoxy en C₁-C₄, CF₃ et (alkyl en C₁-C₄)-sulfonyle,
X représente un atome d'oxygène,
R¹ représente un groupe de formule -COOR,
R² représente un groupe de formule -COOR,
R³ représente un groupe alkyle en C₁-C₄ et
R représente des restes identiques ou différents pris dans le groupe comprenant un atome d'hydrogène et un groupe alkyle en C₁-C₄,
ou
b) de composés de formule I, où
Ar représente un groupe phényle, 1,3-dichlorophényle, 1,3,5-trichlorophényle, 3-méthoxyphényle, naphtyle, coumarinyle, 4-méthyl-coumarinyle ou 7-flavonyle,
X représente un atome d'oxygène,
R¹ représente un groupe de formule -COOR,
R² représente un groupe de formule -COOR,
R³ représente un atome d'hydrogène et
R représente des restes identiques ou différents pris dans le groupe comprenant un atome d'hydrogène, un groupe aryle, alkyle et aralkyle, ou
c) 2-(quinoléin-8-yl-mercapto)maléate de diéthyle,
2-(quinoléin-8-yl-mercapto)acétate d'éthyle ou
4-chloro-phénoxymalonate de diéthyle.

6. Procédé de préparation de composés de formule I selon la revendication 5, **caractérisé en ce que**
a) on fait réagir un composé de formule Ar-(X)ₙ-H, dans laquelle Ar et X possèdent les significations données à la formule I et n = 1, sur un composé de formule II, 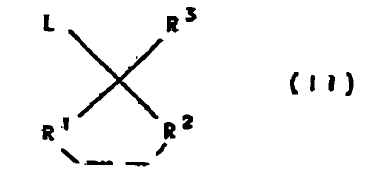 où
L représente un groupe partant et
R¹, R² et R³ sont définis comme à la formule I, ou
b) on fait réagir un composé de formule Ar-W sur un composé de formule III 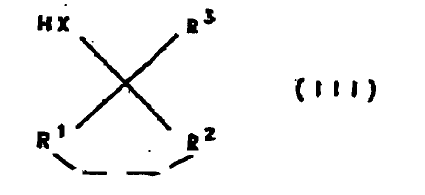 où
W est un groupe partant et
Ar, X, R¹, R² et R³ sont définis comme à la formule I précitée, ou
c) on fait réagir un composé de formule Ar-X-W sur un composé de formule IV, 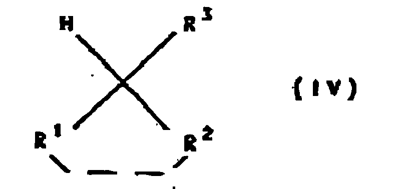 où
W est un groupe partant et
Ar, X, R¹, R² et R³ sont définis comme à la formule I,
ou
d) on transestérifie un dérivé d'un acide aryl- ou hétéroaryloxycarboxylique de formule V 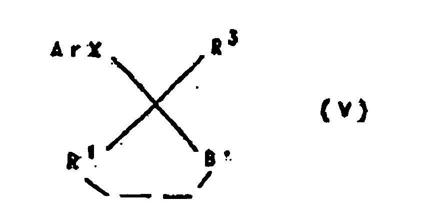 où
Ar, X, R¹ et R³ sont définis comme à la formule I et B' est un groupe de formules 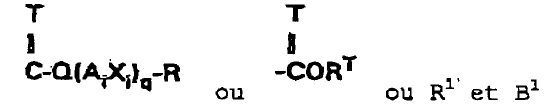 sont liés l'un à l'autre et conjointement
représentent un groupe de formule -CO-Q¹-D-Q²-CO-, où T, Q, Aᵢ, Xᵢ, R, R^{T}, Q¹, Q² et D sont définis de façon analogue aux restes du même nom à la formule I, par des alcools ou mercaptans.

7. Agent pour la protection de plantes de culture contre les effets secondaires phytotoxiques des herbicides, **caractérisé en ce qu'**il renferme en tant qu'antidote efficace un ou plusieurs composés de formule I ou leurs sels selon la revendication 5 et les agents de formulation usuels.

8. Agent herbicide sélectif, **caractérisé en ce qu'**il renferme un ou plusieurs herbicides et. un ou plusieurs antidotes des composés de formule I ou leurs sels selon l'une des revendications 1 à 5.

9. Agent herbicide sélectif selon la revendication 8, **caractérisé en ce qu'**il renferme un ou plusieurs herbicides pris parmi les carbamates, les thiocarbamates, les halogénoacétanilides, les dérivés substitués des acides phénoxy-, naphtoxy- et phénoxy-phénoxycarboxylique ainsi que les dérivés de l'acide hétéroaryloxy-phénoxyalcanecarboxylique, comme les esters de l'acide quinolyloxy-, quinoxalyloxy-, pyridyloxy-, benzoxalyloxy- et benzthiazolyloxy-phénoxyalcanecarboxyliques, les dérivés de la cyclohexandione, les imidazolinones, les dérivés de l'acide pyrimidyloxy-pyridine-carboxylique, les dérivés de l'acide pyrimidyloxybenzoique, les sulfonylurées ainsi que les dérivés du triazolopyrimidine-sulfonamide ainsi que les esters de l'acide S-(N-aryl-alkyl-carbamoylméthyl)-dithiophosphorique.

10. Agent selon la revendication 9,
**caractérisé en ce que** le rapport en poids antidote:herbicide est de 1:10 à 10:1.

11. Agent selon l'une quelconque des revendications 7, 8, 9 ou 10, **caractérisé en ce qu'**il renferme de 0,1 à 99 % en masse de matière active de formule I, ou de son sel, ou du mélange de matières actives antidote/herbicide et de 1 à 99,9 % en masse en une matière d'addition liquide ou solide et de 0 à 25 % en masse d'un agent de surface.

12. Procédé pour la protection de plantes de culture contre les effets secondaires phytotoxiques des herbicides, **caractérisé en ce qu'**on applique une quantité efficace d'un composé de formule I, tel que défini selon l'une quelconque des revendications 1 à 5, avant, après ou en même temps que l'herbicide, aux plantes, aux parties de plantes, à la semence de plantes ou à la surface cultivable.

13. Procédé selon la revendication 11,
**caractérisé en ce que** les plantes de culture sont des plantes de céréales, des plantes de riz ou des plantes de maïs.

14. Procédé selon l'une des revendications 12 ou 13, **caractérisé en ce qu'**on applique les composés de formule I ou leur sel en une concentration d'emploi de 0,001 à 5 kg/ha de substance active et dans un rapport en poids antidote:herbicide de 1:10 à 10:1.
